# EUROPEAN PATENT APPLICATION

(11) **EP 4 692 064 A1**
(43) Date of publication of application: **11.02.2026**
(21) Application number: 24784163.8
(22) Date of filing: 28.03.2024
(51) Int. Cl.: C07D 221/22, A61K 31/439

(54) **BULLEYACONITINE A DERIVATIVE HAVING ANALGESIC ACTIVITY AND PREPARATION METHOD THEREFOR AND USE THEREOF**

(30) Priority: 03.04.2023 CN 202310346715
(71) Applicant: Southwest Jiaotong University, Sichuan 610031 (CN)
(72) Inventor: ZHOU, Xianli, Chengdu, Sichuan 610031 (CN); CHEN, Lin, Chengdu, Sichuan 610031 (CN); HUANG, Shuai, Chengdu, Sichuan 610031 (CN); WU, Jingchuan, Chengdu, Sichuan 610031 (CN)
(74) Representative: Vitina, Maruta
(86) International application number: PCT/CN2024/084396
(87) International publication number: WO 2024/208078

(57) **Abstract**

A bulleyaconitine A derivative with analgesic activity, which is a compound represented by 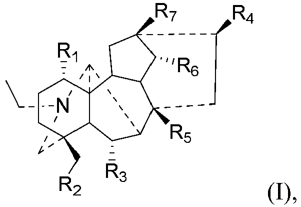 or a stereoisomer, a deuterated compound, a solvate, a prodrug, a metabolite, a crystalline form, or a pharmaceutically acceptable salt thereof. A preparation method and an application thereof are also provided. The bulleyaconitine A derivative provided herein has excellent analgesic and anti-inflammatory activities, low toxicity, high therapeutic index, and good water solubility, exhibiting a great potential to be a non-addictive anti-inflammatory and analgesic drug with low toxicity and high efficacy.

## Description

### TECHNICAL FIELD

This application relates to pharmaceutical chemistry, and more particularly to a bulleyaconitine A derivative with analgesic activity, and a preparation method and an application thereof.

### BACKGROUND

Pain has become one of diseases that threaten the human health. Chronic pain, also known as "non-lethal cancer", severely impacts both physical and mental well-being as well as quality of life, and has emerged as a major global health challenge.

Bulleyaconitine A, also known as crassicauline A, is a C19-diterpenoid diester-type alkaloid distributed in most Aconitum species, primarily found in Aconitum bulleyanum Diels. As early as 1986, researchers had demonstrated analgesic effects of bulleyaconitine A through a writhing test, a hot plate test, a tail-flick test and a formalin test, and develop the bulleyaconitine A into a clinical drug. Currently available dosage form includes tablets, capsules, soft capsules, and injections. Bulleyaconitine A has significant analgesic, anti-inflammatory, and immunosuppressive effects, and is widely applicable in treating various chronic pain, such as rheumatoid arthritis, osteoarthritis, neuropathic pain, herpes zoster pain, periarthritis of the shoulder, lumbar muscle strain, sprain, cancer pain, and postoperative pain. Bulleyaconitine A can also alleviate inflammatory responses including redness, swelling, and heat caused by rheumatic and rheumatoid arthritis. In addition, due to its non-addictive properties, lack of drug resistance, and absence of gastrointestinal reactions, toxicity and side effects of bulleyaconitine A are significantly lower than those of opioids and nonsteroidal anti-inflammatory drugs, rendering it highly suitable for long-term analgesic management. In 2013, bulleyaconitine A was included in Expert Consensus on the Diagnosis and Treatment of Neuropathic Pain, playing a critical role in managing chronic pain of diverse origins.

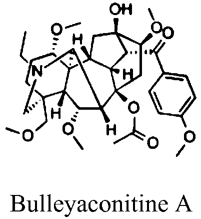

Bulleyaconitine A, as a first non-addictive and non-opioid analgesic derived from traditional Chinese medicine, has significant advantages of high analgesic potency, long duration of action, absence of dependence, and non-addictive properties. However, its safety profile is relatively narrow, with notable acute toxicity. During clinical use, it may cause side effects in patients, including transient and mild palpitations, nausea, numbness of the lips and tongue, and cardiac arrhythmias. Animal toxicological studies indicate that the diester alkaloids contained in Aconitum primarily involves stimulation of the nervous system (especially vagus and sensory nerves), with direct effects on the myocardium, potentially leading to death due to respiratory or myocardial paralysis. Therefore, modifying bulleyaconitine A to develop novel derivatives with higher bioactivity and lower toxicity is of significant importance for the clinical application of analgesic drugs.

### SUMMARY

An object of this application is to provide a bulleyaconitine A derivative with low toxicity and high analgesic activity, and a preparation method and an application thereof.

This application provides a compound of formula (I), or a stereoisomer, a deuterated compound, a solvate, a prodrug, a metabolite, a crystalline form, or a pharmaceutically acceptable salt thereof: 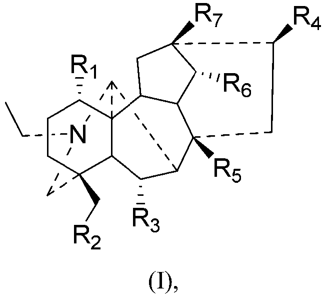
wherein R₁, R₂, R₃, R₄ and R₅ are each independently selected from the group consisting of hydrogen, hydroxyl, halogen, C₁₋₁₈ alkoxy, C₁₋₁₈ alkyl, COORₐ and OCORₐ; wherein Rₐ is selected from the group consisting of hydrogen, C₁₋₁₈ alkyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl;
R₆ and R₇ are each independently selected from the group consisting of hydrogen, hydroxyl, C₁₋₁₈ alkoxy, C₁₋₁₈ alkyl, OCOR₁, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl; wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z}; and
each R_{z} is independently selected from the group consisting of hydrogen, substituted and unsubstituted C₁₋₆ alkyl, C₂₋₆ alkenyl, C₂₋₆ alkynyl, substituted and unsubstituted C₁₋₆ alkoxy, 3 to 8-membered saturated cycloalkyl, NR₃R₄, COOR₅, SO₂R₆, halogen, cyano group, nitro, hydroxyl, carboxyl and phenyl, wherein substituted C₁₋₆ alkyl and substituted C₁₋₆ alkoxy independently have a substituent selected from the group consisting of halogen, cyano group, nitro, hydroxy and carboxy.

In an embodiment, the compound is represented by formula (II): 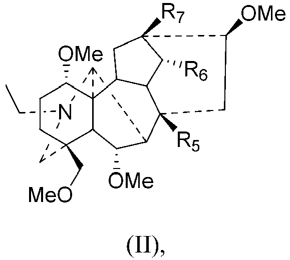
wherein R₅ is selected from the group consisting of hydrogen, hydroxyl, halogen, C₁₋₁₈ alkoxy, C₁₋₁₈ alkyl, COORₐ and OCORₐ, wherein Rₐ is selected from the group consisting of hydrogen, C₁₋₁₈ alkyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl;
R₆ and R₇ are each independently selected from the group consisting of hydrogen, hydroxyl, C₁₋₁₈ alkoxy, C₁₋₁₈ alkyl, OCOR_{b}, OCOCH₂R_{b} and OSO₂Rₑ;
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl, wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z};
each R_{z} is independently selected from the group consisting of hydrogen, substituted and unsubstituted C₁₋₆ alkyl, C₂₋₆ alkenyl, C₂₋₆ alkynyl, substituted and unsubstituted C₁₋₆ alkoxy, 3 to 8-membered saturated cycloalkyl, NR₃R₄, COOR₅, SO₂R₆, halogen, cyano group, nitro, hydroxyl, carboxyl and phenyl, wherein substituted C₁₋₆ alkyl and substituted C₁₋₆ alkoxy independently have a substituent selected from the group consisting of halogen, cyano group, nitro, hydroxyl and carboxyl, and R₃ and R₄ are each independently selected from the group consisting of hydrogen and C₁₋₆ alkyl.

In an embodiment, the compound is represented by formula (III): 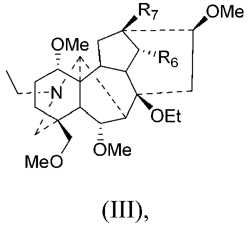
wherein R₆ and R₇ are each independently selected from the group consisting of hydrogen, hydroxyl, C₁₋₆ alkoxy, C₁₋₆ alkyl, OCOR_{b}, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl, wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z};
each R_{z} is independently selected from the group consisting of hydrogen, halogenated and unhalogenated C₁₋₆ alkyl, C₂₋₆ alkenyl, C₂₋₆ alkynyl, halogenated and unhalogenated C₁₋₆ alkoxy, 3 to 8-membered saturated cycloalkyl, NR₃R₄, COOR₅, SO₂R₆, halogen, cyano group, nitro, hydroxyl, carboxyl and phenyl; and
R₃ and R₄ are each independently selected from the group consisting of hydrogen and C₁₋₆ alkyl; and R₅ is C₁₋₆ alkyl.

In an embodiment, the compound is represented by formula (IV): 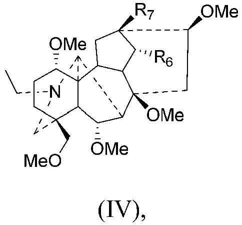
wherein R₆ and R₇ are each independently selected from the group consisting of hydrogen, hydroxyl, C₁₋₆ alkoxy, C₁₋₆ alkyl, OCOR_{b}, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl, wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z};
each R_{z} is independently selected from the group consisting of hydrogen, halogenated and unhalogenated C₁₋₆ alkyl, C₂₋₆ alkenyl, C₂₋₆ alkynyl, halogenated and unhalogenated C₁₋₆ alkoxy, 3 to 8-membered saturated cycloalkyl, NR₃R₄, COOR₅, SO₂R₆, halogen, cyano group, nitro, hydroxyl, carboxyl and phenyl; and
R₃ and R₄ are each independently selected from the group consisting of hydrogen and C₁₋₆ alkyl; and R₅ is C₁₋₆ alkyl.

In an embodiment, the compound is represented by formula (V): 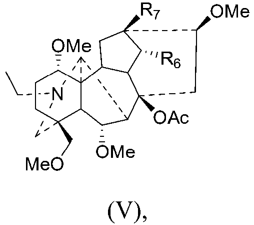
wherein R₆ and R₇ are each independently selected from the group consisting of hydrogen, hydroxy, C₁₋₆ alkoxy, C₁₋₆ alkyl, OCOR_{b}, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl; wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z};
each R_{z} is independently selected from the group consisting of hydrogen, halogenated and unhalogenated C₁₋₆ alkyl, C₂₋₆ alkenyl, C₂₋₆ alkynyl, halogenated and unhalogenated C₁₋₆ alkoxy, 3 to 8-membered saturated cycloalkyl, NR₃R₄, COOR₅, SO₂R₆, halogen, cyano group, nitro, hydroxyl, carboxyl and phenyl; and
R₃ and R₄ are each independently selected from the group consisting of hydrogen and C₁₋₆ alkyl; and R₅ is C₁₋₆ alkyl.

In an embodiment, the compound is selected from the group consisting of:

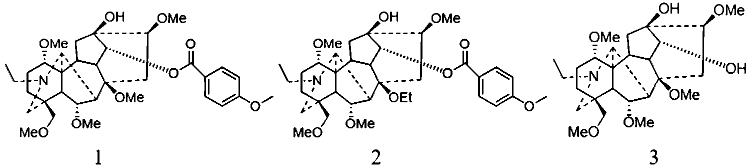

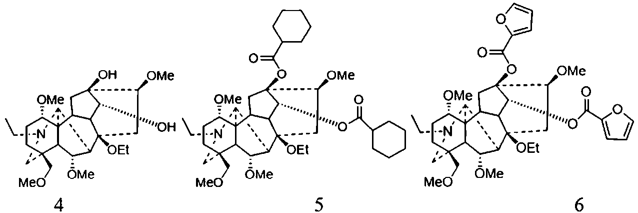

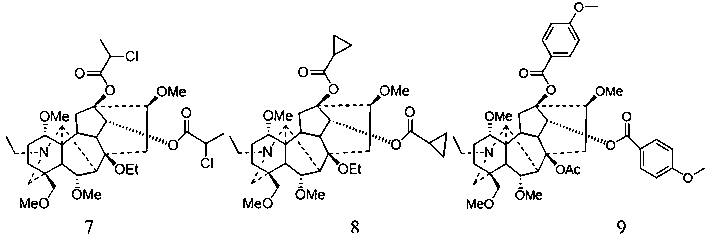

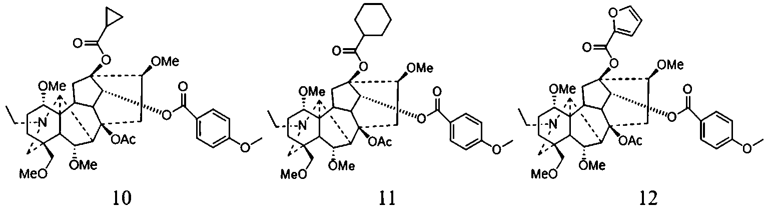

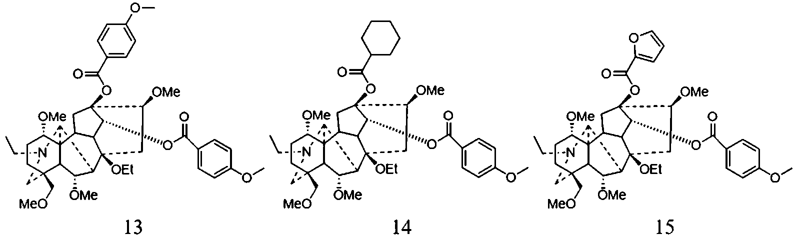

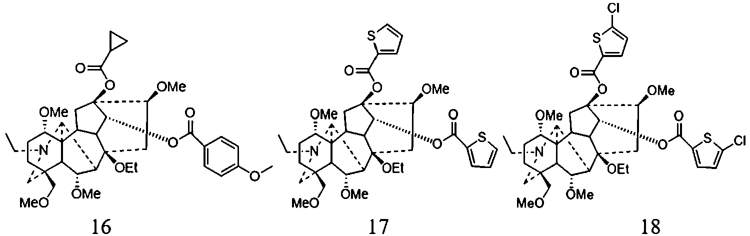

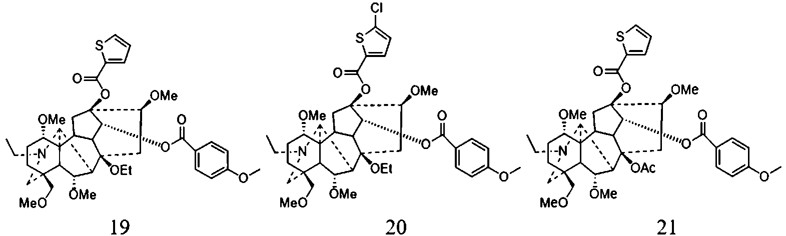

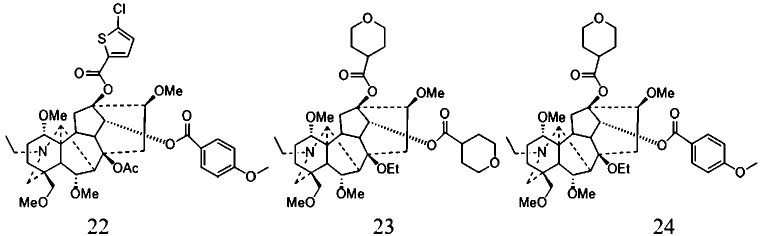

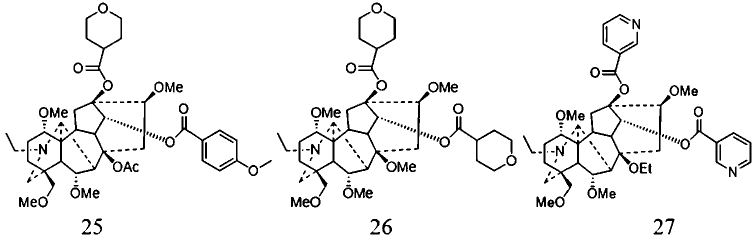

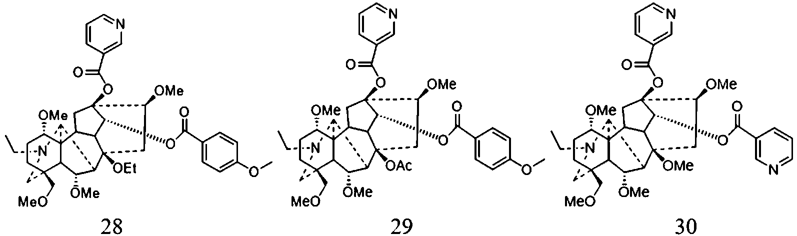

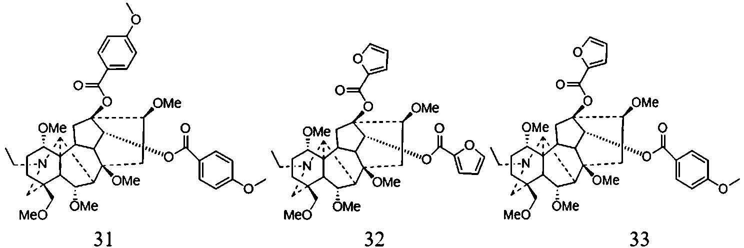

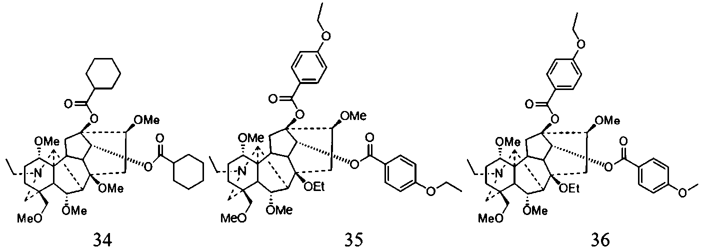

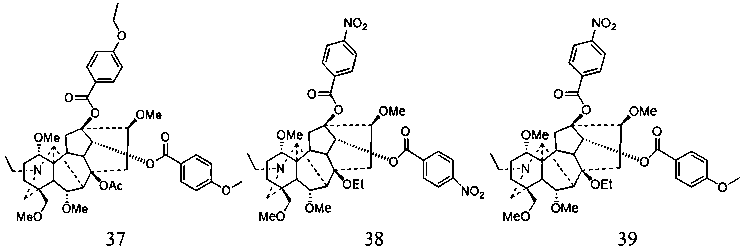

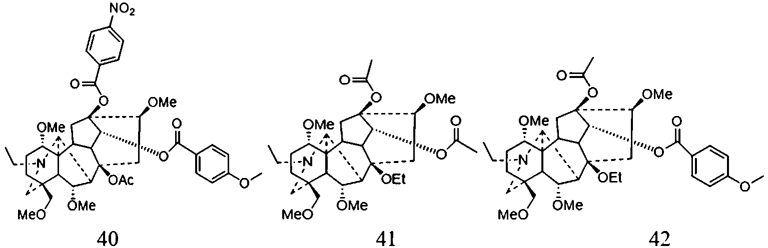

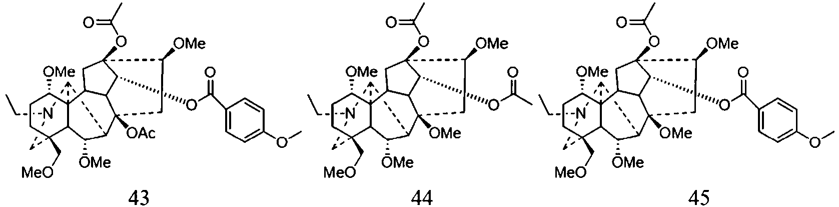

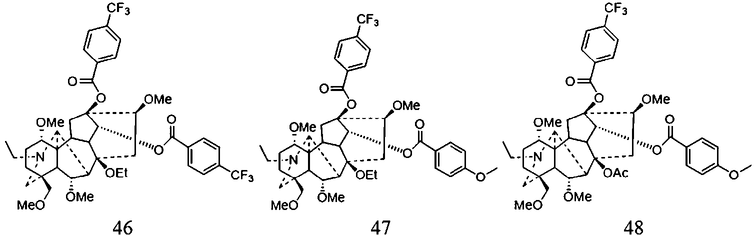

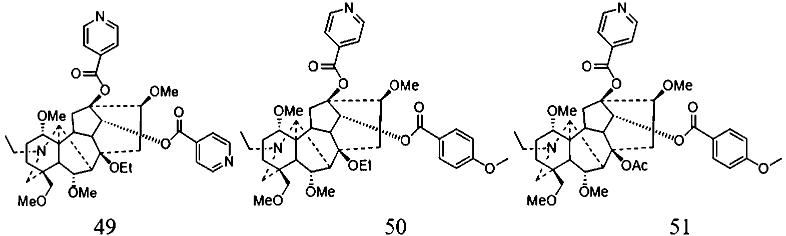

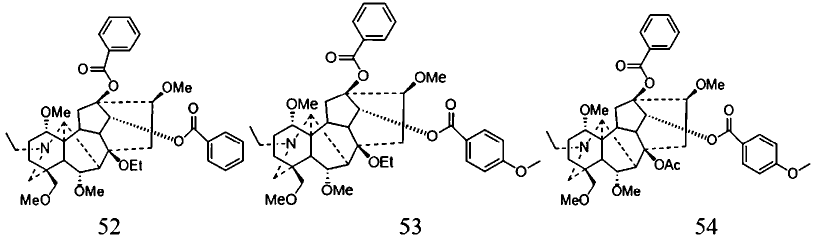

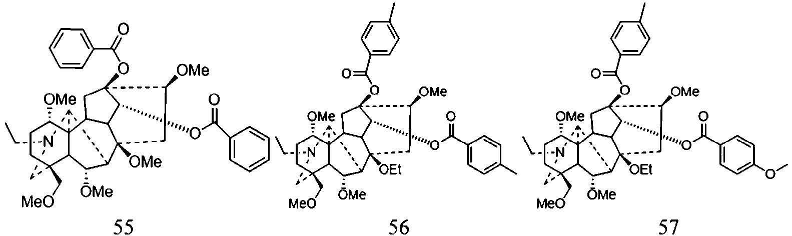

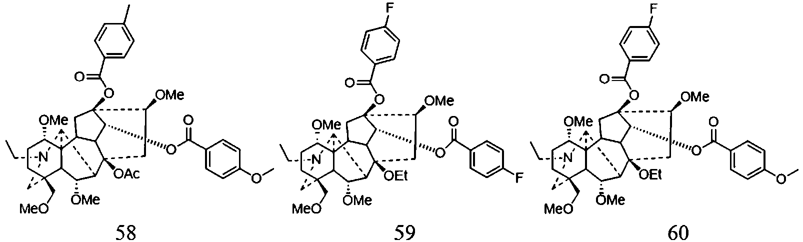

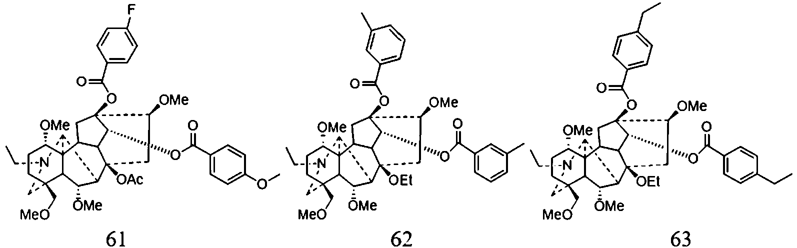

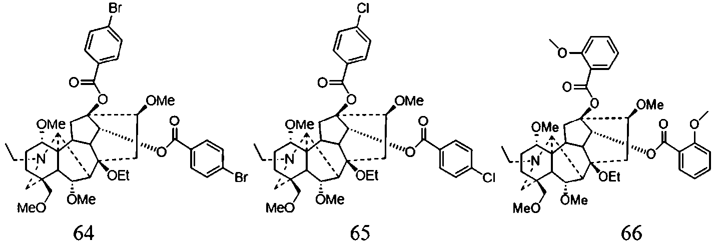

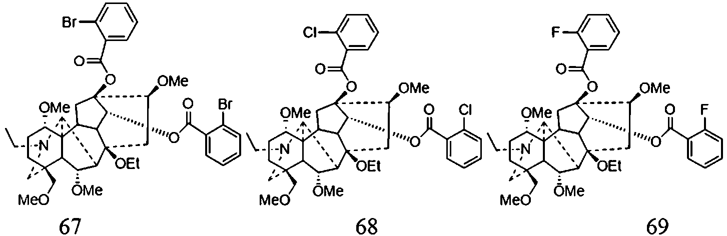

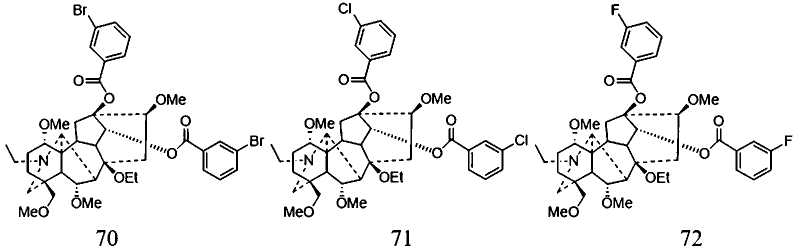

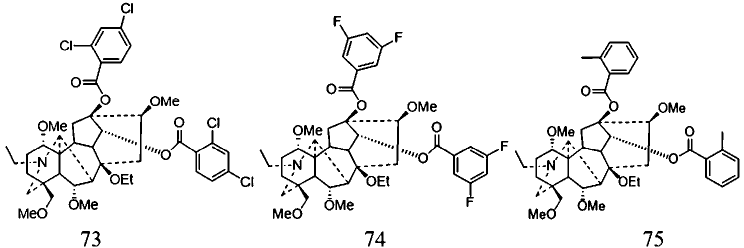

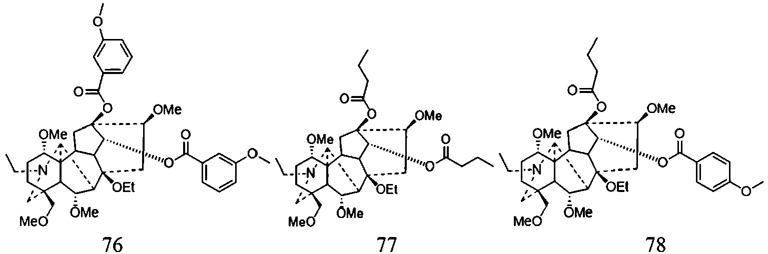

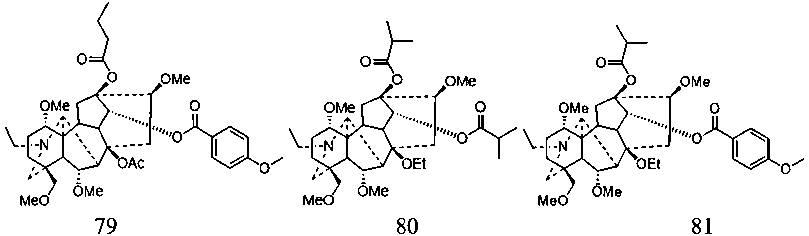

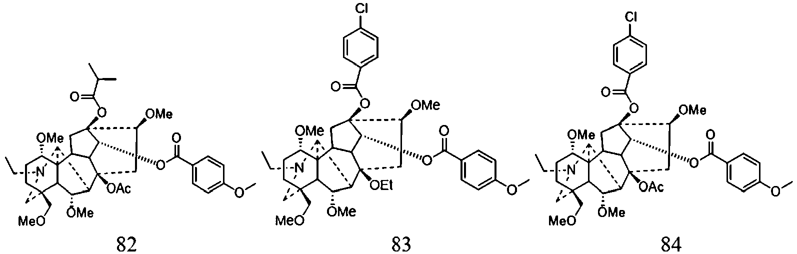

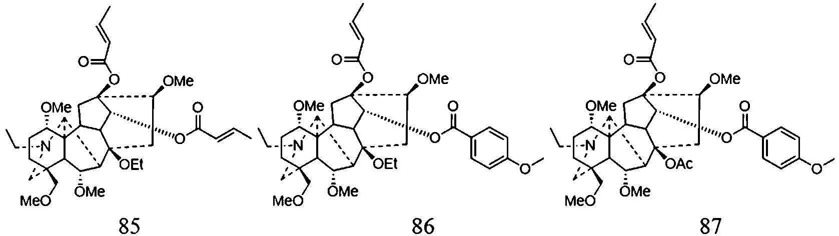

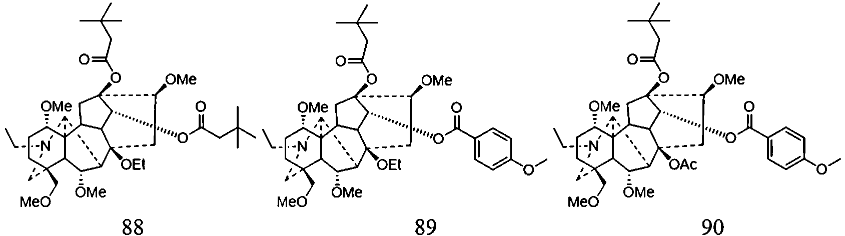

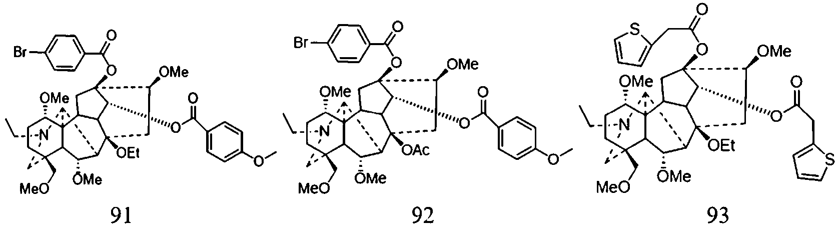

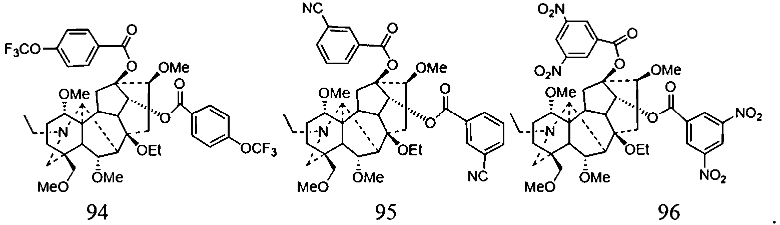

This application further provides a pharmaceutical composition for analgesia and/or anti-inflammation, comprising:
an active ingredient; and
a pharmaceutically acceptable excipient;
wherein the active ingredient is any one of the above compounds, or a stereoisomer, a deuterated compound, a solvate, a prodrug, a metabolite, a crystalline form, or a pharmaceutically acceptable salt thereof.

This application further provides an application of the above compound, or a stereoisomer, a deuterated compound, a solvate, a prodrug, a metabolite, a crystalline form, or a pharmaceutically acceptable salt thereof in the preparation of a drug for analgesia and/or anti-inflammation.

In an embodiment, the drug for analgesia and/or anti-inflammation has low toxicity.

In an embodiment, a median lethal dose of the drug is higher than that of bulleyaconitine A.

In an embodiment, a therapeutic index of the drug is higher than that of bulleyaconitine A.

This application uses bikhaconine as a semi-synthetic raw material, and obtains various C19-diterpenoid alkaloid derivatives through diverse synthetic reactions. The preparation method for these C19-diterpenoid alkaloid derivatives from bikhaconine is simple, employs mild reaction conditions, and is suitable for large-scale production. The prepared diterpenoid alkaloid derivatives have good analgesic activity, good anti-inflammatory activity, low toxicity and high therapeutic index. Besides, as analgesic drugs, diterpenoid alkaloids have advantages of non-addictive properties and non-gastrointestinal irritation, which is expected to become a low-toxic and highly effective non-addictive analgesic drug with broad application prospects.

The compounds and derivatives provided in this application can be named according to IUPAC (International Union of Pure and Applied Chemistry) or CAS (Chemical Abstracts Service) nomenclature systems.

Unless otherwise specified, the initial definitions of groups or terms provided herein are applicable to the groups or terms throughout the specification. The terms not specifically defined herein can be understood by those skilled in the art based on the description of the present disclosure.

In the compound of formula (I), the minimum and maximum content of carbon atoms in a hydrocarbon group are indicated by prefixes. For example, the prefix C_{a-b} alkyl represents any alkyl group containing "a" to "b" carbon atoms. For example, C₁₋₆ alkyl refers to a straight or branched chain alkyl containing 1 to 6 carbon atoms.

The term "substitution" used herein refers to the replacement of one or more hydrogen atoms in a molecule with other different atoms or molecules, including one or more substitutions on the same or different atoms of the molecule.

The term "cycloalkyl" refers to a saturated or unsaturated cyclic hydrocarbon substituent. For example, "3 to 8-membered saturated cycloalkyl" refers to a saturated cycloalkyl with 3 to 8 ring carbon atoms.

The term "heterocycloalkyl" refers to a saturated or unsaturated cyclic hydrocarbon substituent. Such cyclic hydrocarbon has at least one ring heteroatom (including but not limited to O, S, or N). For example, "3 to 8-membered saturated heterocycloalkyl" refers to a saturated heterocycloalkyl with 3 to 8 ring atoms.

The term "aryl" refers to an all-carbon monocyclic group with conjugated π-electron system, such as phenyl.

The term "heteroaryl" refers to a heteroaromatic group containing one to more heteroatoms. The heteroatoms referred herein include oxygen, sulfur and nitrogen, such as, furyl, thienyl, pyridyl, pyrazolyl, pyrrolyl, N-alkylpyrrolyl, pyrimidinyl, pyrazinyl, imidazolyl and tetrazolyl.

The term "deuterated compound" refers to a compound on which one or more hydrogens are replaced with deuterium.

The term "bridged cycloalkyl" refers to a polycyclic cycloalkyl group in which two rings are linked by a single bond, e.g.,

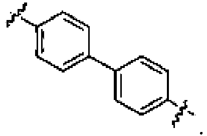

The term "bridged heterocycloalkyl" refers to a polycyclic group in which two rings are connected by a single bond, and at least one of the two rings is a heterocyclic ring, e.g.,

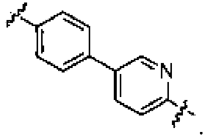

The term "fused cycloalkyl" refers to a polycyclic cycloalkyl group in which two rings share two adjacent carbon atoms, e.g.

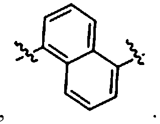

The term "hetero-fused cycloalkyl" refers to a polycyclic group in which two rings share two adjacent carbon atoms, and at least one of the two rings is a heterocyclic ring, e.g.,

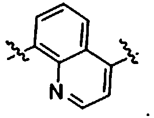

The term "halogen" refers to fluorine, chlorine, bromine or iodine.

The term "pharmaceutically acceptable" means that a carrier, a diluent, an excipient and/or the formed salt is usually chemically or physically compatible with other ingredients constituting a certain pharmaceutical dosage form, and is physiologically compatible with the receptor.

The term "salt" refers to an acid or a basic salt formed by combining a compound or its stereoisomer with an inorganic or organic acid or a base, and may also refers to a zwitterionic salt (internal salt) or a quaternary ammonium salt such as an alkyl ammonium salt. These salts may be obtained directly in the final isolation and purification of the compound, and may also be obtained by mixing the compound or its stereoisomer with a certain amount of acid or base appropriately (for example, equivalent). These salts may be collected through collecting a precipitate in a solution through filtration, or recovered after evaporation of a solvent. These salts may also be obtained by freeze-drying after reacting in an aqueous medium.

The pharmaceutically acceptable salt described herein may be a hydrochloride, a sulfate, a citrate, a benzenesulfonate, a hydrobromide, a hydrofluoride, a phosphate, an acetate, a propionate, an oxalate, a malate, a succinate, a fumarate, a maleate, a tartrate or a trifluoroacetate.

Obviously, various modifications, replacements and alterations can be made by those skilled in the art without departing from the spirit of the present disclosure.

The present disclosure will be further described below with reference to the embodiments. It should be noted that the embodiments provided herein are not intended to limit the present disclosure, and technical solutions obtained based on this disclosure should fall within the scope of the present disclosure.

### DETAILED DESCRIPTION OF EMBODIMENTS

The raw materials and equipment used herein are all known products and commercially available.

The target compound of the disclosure is synthesized according to the following routes.

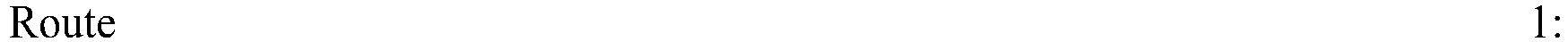

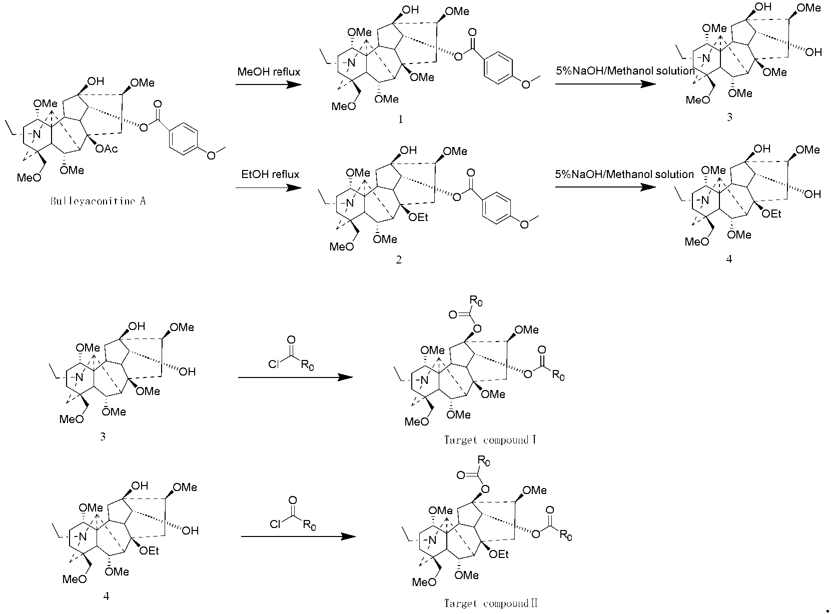

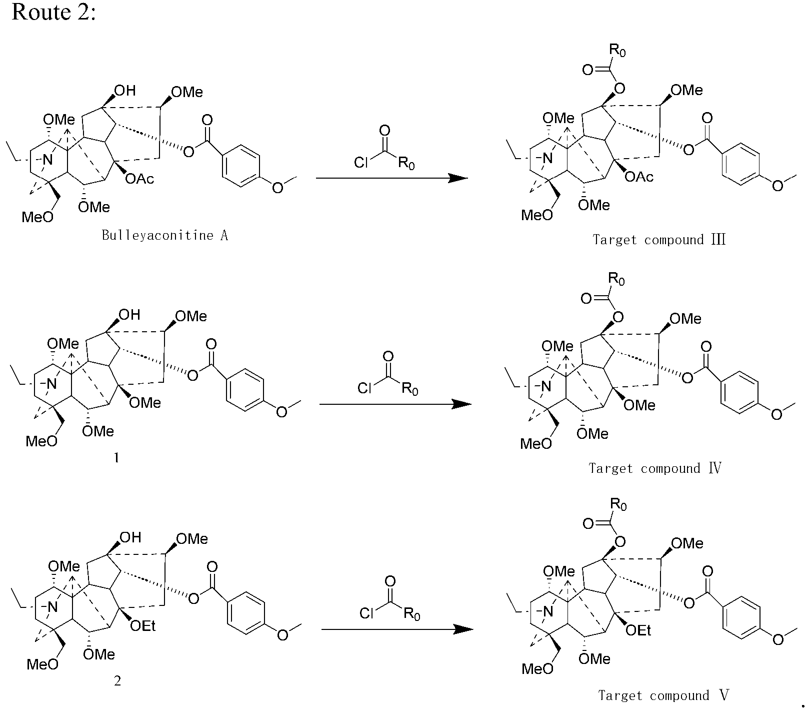

Example 1 Preparation of compound 1

1 g of bulleyaconitine A was added into a 500 mL round-bottom flask, dissolved with 250 mL of methanol, and heated under reflux in an oil bath, where the reaction was monitored through thin-layer chromatography. After reacted for 24 h, the reaction mixture was subjected to rotary evaporation, and purified by column chromatography to obtain 641 mg of a light yellow solid as a target compound 1 (C₃₄H₄₉NO₉, 67.1% yield), which was structurally shown as follows:

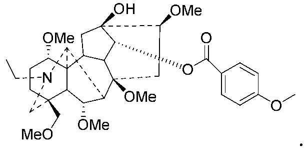

The target compound 1 was characterized as follows.

HRESIMS *m*/*z :* [(M + H)⁺, 616.36]

¹H NMR (400 MHz, CDCl₃) δ 8.00 (d, *J* = 8.8 Hz, 2H), 6.90 (d, *J* = 8.8 Hz, 2H), 4.86 (d, *J* = 5.2 Hz, 1H), 3.97 (d, *J* = 6.6 Hz, 1H), 3.84 (s, 3H), 3.80 (s, 1H), 3.60 (d, *J* = 8.2 Hz, 1H), 3.50 (s, 3H), 3.38 (dd, *J* = 8.8, 6.2 Hz, 1H), 3.28 (s, 3H), 3.25 (s, 3H), 3.24 (s, 3H), 3.14 (d, *J* = 8.2 Hz, 1H), 3.02 (dd, *J* = 9.8, 6.2 Hz, 1H), 2.97 (s, 3H), 2.79 (s, 1H), 2.71 (d, *J* = 10.6 Hz, 1H), 2.59 (t, *J* = 6.2 Hz, 1H), 2.53 (s, 1H), 2.52 - 2.48 (m, 2H), 2.44 (d, *J* = 12.4 Hz, 1H), 2.36 (s, 1H), 2.31 (d, *J* = 6.0 Hz, 1H), 2.27 - 2.20 (m, 1H), 2.05 (dd, *J* = 8.2*,* 5.4 Hz, 3H), 1.91 (q, *J* = 6.2, 5.6 Hz, 2H), 1.66 - 1.58 (m, 2H), 1.08 (t, *J =* 7.2 Hz, 3H).

¹³C NMR (100 MHz, CDCl₃) δ 166.57, 163.19, 131.88, 131.88, 123.31, 113.55, 113.55, 85.46, 84.02, 82.93, 80.34, 79.19, 78.45, 75.56, 61.58, 59.17, 58.89, 58.73, 56.43, 55.47, 53.91, 50.68, 49.14, 48.72, 48.54, 48.54, 46.56, 41.61, 39.16, 36.59, 36.52, 35.01, 26.46, 13.63.

### Example 2 Preparation of compound 2

1 g of bulleyaconitine A was added into a 500 mL round-bottom flask, dissolved with 250 mL ethanol, and heated under reflux in an oil bath, where the reaction was monitored through thin-layer chromatography. After reacted for 72 h, the reaction mixture was subjected to rotary evaporation, and purified by column chromatography to obtain 550 mg of a light yellow solid as a target compound 2 (C₃₅H₅₁NO₉, 56.2% yield), which was structurally shown as follows:

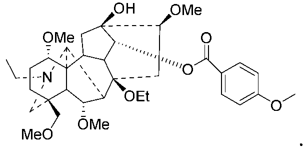

The target compound 2 was characterized as follows.

HRESIMS m/z : [(M + H)⁺, 630.37]

¹H NMR (400 MHz, CDCl₃) δ 8.01 (d, *J* = 8.8 Hz, 2H), 6.89 (d, *J* = 8.8 Hz, 2H), 4.82 (d, *J* = 5.2 Hz, 1H), 3.99 (d, *J =* 6.6 Hz, 1H), 3.84 (s, 3H), 3.81 (s, 1H), 3.60 (d, *J* = 8.2 Hz, 1H), 3.51 (s, 3H), 3.38 (t, *J =* 7.6 Hz, 1H), 3.32 (t, *J =* 7.6 Hz, 1H), 3.28 (s, 3H), 3.24 (d, *J* = 2.2 Hz, 6H), 3.11 (dd, *J =* 13.2, 7.8 Hz, 2H), 3.02 (dd, *J =* 9.8, 6.2 Hz, 1H), 2.77 (s, 1H), 2.70 (s, 1H), 2.68 - 2.63 (m, 1H), 2.49 (s, 3H), 2.34 (s, 1H), 2.31 - 2.26 (m, 2H), 2.11 - 2.01 (m, 3H), 1.94 - 1.87 (m, 1H), 1.63 (dd, *J =* 10.0, 4.2 Hz, 2H), 1.41 (s, 1H), 1.32 - 1.26 (m, 1H), 1.07 (t, *J* = 7.2 Hz, 3H), 0.58 (t, *J =* 6.8 Hz, 3H).

¹³C NMR (100 MHz, CDCl₃) δ 166.45, 163.19, 131.85, 131.85, 123.47, 113.51, 113.51, 85.53, 84.16, 83.04, 80.24, 79.25, 78.16, 75.50, 61.52, 59.14, 58.85, 58.82, 56.46, 55.94, 55.47, 53.91, 50.71, 49.27, 49.11, 48.48, 46.52, 41.49, 39.15, 37.38, 36.60, 35.02, 26.46, 15.41, 13.58.

### Example 3 Preparation of compound 3

500 mg of compound 1 was added into a 500 mL round-bottom flask, added with 120 mL of a methanol solution containing 5 wt.% NaOH, and reacted at 50 °C, where the reaction was monitored through thin-layer chromatography. The reaction was confirmed to be completed after 30 min, and then the reaction mixture was subjected to rotary evaporation, and purified by column chromatography to obtain 373 mg of a light yellow solid as a target compound 3 (C₂₆H₄₃NO₇, 95.3% yield), which was structurally shown as follows:

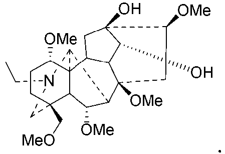

The target compound 3 was characterized as follows.

HRESIMS *m*/*z :* [(M + H)⁺, 481.31]

¹H NMR (400 MHz, Chloroform-d) δ 5.29 (s, 1H), 4.05 (d, *J =* 6.7 Hz, 1H), 3.83 (d, *J* = 4.9 Hz, 1H), 3.66 (d, *J =* 8.4 Hz, 1H), 3.43 (s, 3H), 3.36 - 3.33 (m, 1H), 3.32 (s, 3H), 3.30 (s, 3H), 3.25 (s, 3H), 3.22 (s, 3H), 3.11 (d, *J =* 8.3 Hz, 1H), 3.08 - 2.91 (m, 3H), 2.59 - 2.39 (m, 6H), 2.37 - 2.27 (m, 3H), 2.26 - 2.21 (m, 1H), 2.09 (d, *J =* 6.6 Hz, 1H), 1.96 - 1.85 (m, 3H), 1.61 (dd, *J =* 9.6, 3.9 Hz, 2H), 1.08 (t, *J* = 7.1 Hz, 3H).

¹³C NMR (101 MHz, CDCl₃) δ 85.70, 84.15, 82.95, 80.38, 78.72, 78.63, 76.58, 62.37, 59.24, 59.02, 58.65, 56.46, 53.68, 50.56, 49.77, 49.32, 49.21, 48.91, 48.39, 41.80, 39.34, 36.44, 35.48, 34.13, 26.27, 13.74.

### Example 4 Preparation of compound 4

500 mg of compound 2 was added into a 500 mL round-bottom flask, added with 120 mL of a methanol solution containing 5 wt.% NaOH, and reacted at 50 °C, where the reaction was monitored through thin-layer chromatography. The reaction was confirmed to be completed after 30 min, and then the reaction mixture was subjected to rotary evaporation, and purified by column chromatography to obtain 368 mg of a light yellow solid as a target compound 4 (C₂₇H₄₅NO₇, 93.6% yield), which was structurally shown as follows:

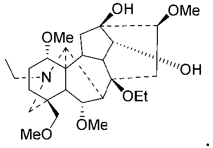

The target compound 4 was characterized as follows.

HRESIMS m/z : [(M + H)⁺, 496.34]

¹H NMR (400 MHz, CDCl₃) δ 4.06 (d, J = 6.6 Hz, 1H), 3.84 (t, J = 5.2 Hz, 1H), 3.65 (d, J = 8.2 Hz, 1H), 3.54 - 3.48 (m, 1H), 3.48 - 3.43 (m, 1H), 3.42 (s, 3H), 3.32 (dd, J = 6.6, 1.2 Hz, 1H), 3.30 (s, 3H), 3.29 (s, 3H), 3.21 (s, 3H), 3.11 (d, J = 5.8 Hz, 1H), 3.06 (d, J = 8.2 Hz, 1H), 2.97 (dd, J = 10.2, 6.4 Hz, 1H), 2.88 (s, 1H), 2.53 (dd, J = 11.8, 4.8, Hz, 2H), 2.50 - 2.45 (m, 2H), 2.45 - 2.42 (m, 1H), 2.39 (s, 1H), 2.29 (d, J = 3.8 Hz, 2H), 2.27 (s, 1H), 2.08 (d, J = 6.6 Hz, 1H), 1.94 - 1.88 (m, 2H), 1.88 - 1.83 (m, 1H), 1.63 (dd, J = 4.8, 1.8 Hz, 1H), 1.59 (dd, J = 6.0, 3.4 Hz, 1H), 1.39 (s, 1H), 1.27 (s, 1H), 1.16 (t, J = 7.0 Hz, 3H), 1.06 (t, J = 7.0 Hz, 3H).

¹³C NMR (100 MHz, CDCl₃) δ 85.61, 84.05, 83.05, 80.30, 78.83, 78.62, 76.77, 62.23, 59.17, 59.06, 58.73, 56.56, 56.43, 53.61, 50.49, 49.64, 49.25, 49.09, 49.00, 41.57, 39.29, 36.63, 35.61, 35.49, 26.32, 16.12, 13.68.

### Example 5 Preparation of compound 5

100 mg of compound 4 was added into a 5 mL round-bottom flask and dissolved with 2 mL of dry pyridine. Under an argon atmosphere, the reaction mixture was added with 2 equivalents of cyclohexanecarbonyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 122 mg of a light yellow foamy solid as a target compound 5 (C₄₁H₆₅NO₉, 85.0% yield), which was structurally shown as follows:

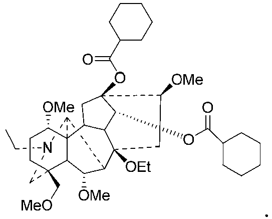

The target compound 5 was characterized as follows.

HRESIMS m/z : [(M + H)⁺, 716.47]

¹H NMR (400 MHz, CDCl₃) δ 4.93 (d, *J =* 5.2 Hz, 1H), 3.98 (dd, *J =* 6.6, 1.6 Hz, 1H), 3.85 (t, *J =* 7.8 Hz, 1H), 3.63 (d, *J =* 8.2 Hz, 1H), 3.52 - 3.44 (m, 1H), 3.40 - 3.32 (m, 1H), 3.29 (s, 6H), 3.25 (s, 3H), 3.20 (s, 3H), 3.05 (d, *J* = 8.2 Hz, 1H), 2.98 (dd, *J* = 9.2, 6.2 Hz, 1H), 2.78 (s, 1H), 2.52 (dd, *J =* 10.6, 1.6 Hz, 1H), 2.48 - 2.44 (m, 1H), 2.43 (d, *J* = 4.8 Hz, 1H), 2.38 (d, *J =* 6.2 Hz, 3H), 2.35 - 2.30 (m, 1H), 2.28 (d, *J =* 3.6 Hz, 1H), 2.25 (t, *J* = 3.6 Hz, 1H), 2.22 (d, *J* = 3.0 Hz, 1H), 2.20 (d, *J =* 2.2 Hz, 1H), 2.05 (d, *J =* 6.4 Hz, 1H), 1.99 - 1.91 (m, 3H), 1.86 (s, 2H), 1.83 (d, *J =* 3.2 Hz, 2H), 1.70 (t, *J =* 8.2, 3.8 Hz, 5H), 1.61 (t, *J* = 5.6 Hz, 3H), 1.50 - 1.44 (m, 2H), 1.42 (s, 1H), 1.41 - 1.38 (m, 1H), 1.32 (s, 1H), 1.28 (s, 2H), 1.25 - 1.22 (m, 3H), 1.21 (s, 1H), 1.10 (t, *J* = 7.4 Hz, 3H), 1.06 (t, *J* = 6.8 Hz, 3H).

¹³C NMR (100 MHz, CDCl₃) δ 175.85, 175.26, 84.86, 83.55, 81.96, 80.30, 80.19, 78.30, 76.67, 60.38, 59.16, 58.81, 57.88, 56.10, 56.07, 54.38, 50.91, 48.75, 48.57, 47.63, 45.19, 43.24, 42.34, 39.02, 37.44, 36.25, 34.75, 31.57, 30.32, 29.16, 29.08, 28.93, 28.82, 26.37, 26.08, 26.05, 25.62, 25.53, 25.49, 16.14, 13.54.

### Example 6 Preparation of compound 6

100 mg of compound 4 was added into a 5 mL round-bottom flask and dissolved with 2 mL of dry pyridine. Under an argon atmosphere, the reaction mixture was added with 2 equivalents of 2-furoyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 137 mg of a light yellow foamy solid as a target compound 6 (C₃₇H₄₉NO₁₁, 90.0% yield), which was structurally shown as follows:

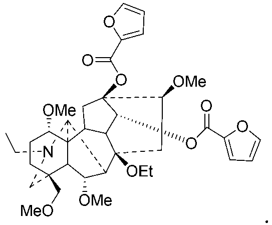

The target compound 6 was characterized as follows.

HRESIMS m/z : [(M + H)+, 684.34]

¹H NMR (400 MHz, CDCl₃) δ 7.57 (dd, *J =* 1.8, 0.8 Hz, 1H), 7.52 (dd, *J =* 1.8, 0.8 Hz, 1H), 7.19 (dd, *J =* 3.4, 0.8 Hz, 1H), 7.10 (dd, *J =* 3.4, 0.8 Hz, 1H), 6.48 (dd, *J =* 3.4, 1.6 Hz, 1H), 6.43 (dd, *J* = 3.4*,* 1.6 Hz, 1H), 5.20 (d, *J* = 5.2 Hz, 1H), 4.15 - 4.06 (m, 1H), 4.01 (d, *J* = 5.8 Hz, 1H), 3.63 (d, *J* = 8.2 Hz, 1H), 3.50 (d, *J* = 9.6 Hz, 1H), 3.44 - 3.35 (m, 2H), 3.35 (s, 3H), 3.29 (s, 4H), 3.27 (s, 5H), 3.23 (s, 4H), 3.08 (d, *J* = 8.2 Hz, 1H), 3.01 (dd, *J* = 9.8, 6.2 Hz, 1H), 2.86 (s, 1H), 2.65 (t, *J =* 6.2 Hz, 1H), 2.55 - 2.43 (m, 4H), 2.40 (s, 2H), 2.37 (d, *J* = 6.6 Hz, 2H), 2.31 (dd, *J =* 14.2, 8.8 Hz, 2H), 2.11 - 2.02 (m, 3H), 1.90 (dd, *J =* 12.2, 6.0 Hz, 1H), 1.74 (s, 1H), 1.68 - 1.58 (m, 2H), 1.25 (s, 1H), 1.10 *(t, J=* 7.2 Hz, 3H), 0.69 (t, *J* = 6.8 Hz, 3H).

¹³C NMR (100 MHz, CDCl₃) δ 158.79, 157.92, 146.28, 146.17, 145.27, 145.03, 118.23, 118.11, 111.80, 111.80, 85.12, 83.61, 83.34, 80.76, 80.27, 78.19, 77.79, 61.24, 59.16, 58.86, 58.22, 56.25, 56.04, 53.89, 50.83, 49.08, 49.01, 48.29, 45.05, 42.07, 39.14, 37.47, 36.20, 35.11, 26.50, 15.28, 13.64.

### Example 7 Preparation of compound 7

100 mg of compound 4 was added into a 5 mL round-bottom flask and dissolved with 2 mL of dry pyridine. Under an argon atmosphere, the reaction mixture was added with 2 equivalents of 2-chloropropanoyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 98 mg of a light yellow foamy solid as a target compound 7 (C₃₃H₅₁Cl₂NO₉, 71.9% yield), which was structurally shown as follows:

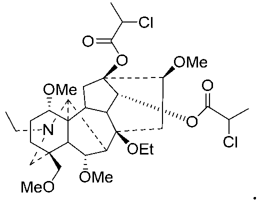

The target compound 7 was characterized as follows.

HRESIMS m/z : [(M + H)+, 676.31]

¹H NMR (400 MHz, CDCl₃) δ 4.46 - 4.37 (m, 2H), 4.30 (t, *J* = 6.6 Hz, 3H), 4.07 - 4.02 (m, 1H), 3.85 (d, *J* = 7.8 Hz, 1H), 3.62 (d, *J =* 8.2 Hz, 1H), 3.51 - 3.45 (m, 1H), 3.40 (t, *J* = 7.4 Hz, 1H), 3.32 (s, 3H), 3.30 (s, 6H), 3.27 (s, 3H), 3.18 (s, 1H), 2.99 (t, *J =* 6.6 Hz, 2H), 2.89 - 2.81 (m, 1H), 2.52 - 2.47 (m, 1H), 2.45 (d, *J* = 4.6 Hz, 1H), 2.33 - 2.26 (m, 2H), 2.15 - 2.11 (m, 1H), 2.03 (q, *J =* 7.2 Hz, 2H), 1.72 (d, *J =* 2.8 Hz, 2H), 1.70 (d, *J =* 7.4 Hz, 3H), 1.66 (dd, *J =* 7.0, 1.8 Hz, 2H), 1.55 (dd, *J* = 8.2, 3.6 Hz, 1H), 1.45 (d, *J* = 7.6 Hz, 1H), 1.42 (q, *J* = 1.4 Hz, 1H), 1.40 (d, *J =* 1.8 Hz, 1H), 1.13 (t, *J =* 6.2 Hz, 3H), 1.09 (d, *J =* 7.4 Hz, 3H), 0.95 (t, *J* = 7.4 Hz, 1H).

¹³C NMR (100 MHz, CDCl₃) δ 169.54, 167.87, 83.28, 82.77, 82.30, 81.19, 79.65, 78.91, 78.23, 60.42, 59.21, 59.01, 57.99, 56.62, 56.09, 52.82, 51.02, 49.18, 48.81, 44.19, 41.36, 38.95, 38.83, 38.60, 37.21, 36.76, 36.64, 34.86, 26.85, 22.81, 22.81, 15.91, 14.25.

### Example 8 Preparation of compound 8

100 mg of compound 4 was added into a 5 mL round-bottom flask and dissolved with 5 mL of dry pyridine. Under an argon atmosphere, the reaction mixture was added with 2 equivalents of cyclopropanecarbonyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 102 mg of a light yellow foamy solid as a target compound 8 (C₃₅H₅₃NO₉, 79.9% yield), which was structurally shown as follows:

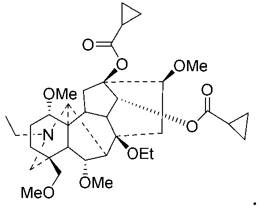

The target compound 8 was characterized as follows.

HRESIMS m/z : [(M + H)⁺, 632.38]

¹H NMR (400 MHz, CDCl₃) δ 4.87 (d, *J =* 5.2 Hz, 1H), 3.98 (dd, *J =* 6.6, 1.6 Hz, 1H), 3.87 (t, *J =* 8.2 Hz, 1H), 3.61 (d, *J* = 8.2 Hz, 1H), 3.47 (dd, *J =* 8.4, 7.0 Hz, 1H), 3.36 (dd, *J* = 8.4, 6.8 Hz, 1H), 3.30 (s, 3H), 3.28 (s, 3H), 3.28 (s, 3H), 3.19 (s, 3H), 3.05 (d, *J* = 8.2 Hz, 1H), 2.98 (dd, *J* = 9.2, 6.0 Hz, 1H), 2.78 (s, 1H), 2.55 - 2.42 (m, 5H), 2.36 (d, *J* = 1.6 Hz, 1H), 2.23 (dd, *J =* 7.8, 1.7 Hz, 3H), 2.04 (d, *J =* 6.6 Hz, 1H), 1.95 - 1.79 (m, 3H), 1.69 - 1.65 (m, 1H), 1.64 - 1.57 (m, 3H), 1.40 (d, *J* = 6.4 Hz, 1H), 1.13 (t, *J =* 6.8 Hz, 3H), 1.06 (t, *J* = 7.2 Hz, 3H), 0.88 - 0.84 (m, 2H), 0.84 - 0.82 (m, 2H), 0.82 - 0.80 (m, 2H), 0.80 - 0.75 (m, 2H).

¹³C NMR (100 MHz, CDCl₃) δ 174.87, 174.02, 84.63, 83.22, 82.11, 80.44, 80.04, 78.06, 77.20, 65.57, 60.43, 59.03, 58.72, 58.04, 56.06, 55.95, 54.22, 50.68, 48.69, 44.74, 41.86, 38.88, 37.46, 36.12, 26.10, 22.69, 19.18, 15.98, 13.37, 13.29, 13.17, 8.72, 8.66, 8.63, 8.52.

### Example 9 Preparation of compound 9

100 mg of bulleyaconitine A was added into a 25 mL round-bottom flask, and dissolved with 5 mL of dry pyridine. Under an argon atmosphere, the reaction mixture was added with 2 equivalents of 4-methoxybenzoyl chloride in an ice water bath, and reacted at a room temperature (about 20 °C), where the reaction was monitored through thin-layer chromatography. After reacted for about 12 h, the reaction mixture was subjected to rotary evaporation, and purified by column chromatography to obtain 91 mg of a light yellow foamy solid as a target compound 9 (C₄₃H₅₅NO₁₂, 75.5% yield), which was structurally shown as follows:

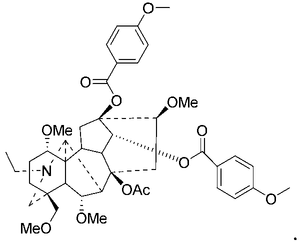

The target compound 9 was characterized as follows.

HRESIMS m/z : [(M + H)⁺, 778.39]

¹H NMR (400 MHz, CDCl₃) δ 8.07 (d, *J* = 8.8 Hz, 2H), 7.94 (d, *J =* 8.8 Hz, 2H), 6.91 (d, *J* = 8.8 Hz, 2H), 6.84 (d, *J* = 8.8 Hz, 2H), 5.29 - 5.25 (m, 1H), 4.14 (dd, *J* = 8.8, 5.6 Hz, 1H), 3.99 (d, *J* = 6.6 Hz, 1H), 3.84 (s, 3H), 3.81 (s, 3H), 3.62 (d, *J* = 8.4 Hz, 1H), 3.52 (d, *J* = 10.4 Hz, 1H), 3.32 (s, 3H), 3.28 (s, 3H), 3.25 (s, 3H), 3.16 (s, 3H), 3.03 (s, 3H), 2.88 (t, *J* = 6.2 Hz, 1H), 2.58 (dd, *J =* 12.2, 7.2 Hz, 1H), 2.55 - 2.51 (m, 1H), 2.49 (d, *J* = 5.8 Hz, 1H), 2.47 (s, 1H), 2.37 - 2.21 (m, 1H), 2.15 - 2.05 (m, 3H), 1.92 (t, *J* = 9.2 Hz, 1H), 1.65 - 1.57 (m, 2H), 1.41 (d, *J =* 5.4 Hz, 1H), 1.35 (s, 3H), 1.28 (s, 1H), 1.21 - 1.15 (m, 1H), 1.10 (t, *J* = 7.2 Hz, 3H).

¹³C NMR (100 MHz, CDCl₃) δ 169.97, 166.37, 165.50, 163.60, 163.28, 132.10, 132.10, 131.94, 131.94, 123.38, 122.74, 113.88, 113.88, 113.54, 113.54, 85.69, 84.81, 83.40, 82.56, 80.53, 80.53, 77.28, 61.53, 59.22, 58.40, 57.94, 56.12, 55.56, 55.49, 53.99, 50.43, 49.32, 49.09, 44.05, 41.96, 39.82, 39.22, 35.79, 35.02, 29.81, 26.38, 21.85, 13.59.

### Example 10 Preparation of compound 10

100 mg of bulleyaconitine A was added into a 25 mL round-bottom flask, and dissolved with 5 mL of dry pyridine. Under an argon atmosphere, the reaction mixture was added with 2 equivalents of cyclopropylcarbonyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 80 mg of a light yellow foamy solid as a target compound 10 (C₃₉H₅₃NO₁₁, 73.1% yield), which was structurally shown as follows:

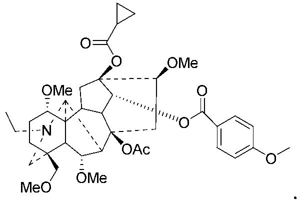

The target compound 10 was characterized as follows.

HRESIMS m/z : [(M + H)+, 712.38]

¹H NMR (400 MHz, CDCl₃) δ 8.07 (d, *J* = 8.8 Hz, 2H), 6.93 (d, *J =* 8.8 Hz, 2H), 5.12 (d, *J* = 5.2 Hz, 1H), 4.02 - 3.96 (m, 2H), 3.87 (s, 3H), 3.62 (d, *J* = 8.4 Hz, 1H), 3.37 (s, 3H), 3.29 (s, 3H), 3.24 (s, 3H), 3.16 (s, 3H), 3.02 (d, *J =* 11.8 Hz, 3H), 2.98 (s, 1H), 2.85 (t, *J* = 6.2 Hz, 1H), 2.59 - 2.53 (m, 1H), 2.52 - 2.49 (m, 1H), 2.46 (d, *J* = 6.0 Hz, 2H), 2.35 - 2.25 (m, 1H), 2.12 - 2.06 (m, 2H), 2.03 - 1.97 (m, 1H), 1.95 (s, 1H), 1.65 - 1.62 (m, 1H), 1.61 - 1.57 (m, 1H), 1.42 (s, 1H), 1.32 (s, 3H), 1.30 (s, 1H), 1.27 (s, 2H), 1.09 (t, *J =* 7.2 Hz, 3H), 0.96 (dd, *J* = 4.4, 2.8 Hz, 2H).

¹³C NMR (100 MHz, CDCl₃) δ 173.94, 169.84, 166.16, 163.46, 131.97, 131.97, 122.64, 113.74, 113.74, 85.53, 84.62, 83.24, 82.02, 80.38, 80.29, 77.02, 61.34, 59.10, 58.30, 57.80, 55.89, 55.44, 53.86, 50.25, 49.14, 48.93, 48.90, 43.82, 41.71, 39.64, 39.07, 35.58, 34.85, 26.24, 21.68, 13.42, 13.23, 8.53, 8.47.

### Example 11 Preparation of compound 11

100 mg of bulleyaconitine A was added into a 25 mL round-bottom flask, and dissolved with 5 mL of dry pyridine. Under an argon atmosphere, the reaction mixture was added with 2 equivalents of cyclohexanecarbonyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 76 mg of a light yellow foamy as a target compound 11 (C₄₂H₅₉NO₁₁, 65.1% yield), which was structurally shown as follows:

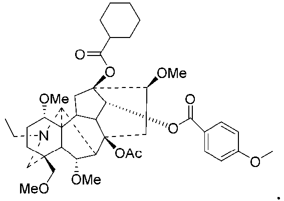

The target compound 11 was characterized as follows.

HRESIMS m/z : [(M + H)+, 754.43]

¹H NMR (400 MHz, CDCl₃) δ 8.04 (d, *J* = 8.8 Hz, 2H), 6.92 (d, *J* = 8.8 Hz, 2H), 5.06 (d, *J =* 5.2 Hz, 1H), 3.97 (dd, *J =* 9.8, 6.2 Hz, 2H), 3.85 (s, 3H), 3.61 (d, *J* = 8.4 Hz, 1H), 3.39 (dd, *J* = 15.6, 5.8 Hz, 1H), 3.33 (s, 3H), 3.27 (s, 3H), 3.23 (s, 3H), 3.14 (s, 3H), 3.03 - 2.93 (m, 4H), 2.83 (dd, *J* = 7.4, 5.4 Hz, 1H), 2.58 - 2.48 (m, 2H), 2.46 - 2.39 (m, 3H), 2.27 (ddt, *J =* 11.2, 7.2, 3.6 Hz, 2H), 2.11 - 2.02 (m, 2H), 1.95 - 1.86 (m, 3H), 1.84 (d, *J =* 2.4 Hz, 1H), 1.70 (dd, *J* = 9.6, 3.6 Hz, 2H), 1.63 - 1.55 (m, 3H), 1.46 - 1.35 (m, 3H), 1.31 (s, 3H), 1.28 (d, *J* = 2.8 Hz, 1H), 1.21 (s, 1H), 1.16 (s, 1H), 1.08 (t, *J* = 7.2 Hz, 3H).

¹³C NMR (100 MHz, CDCl₃) δ 175.39, 169.96, 166.26, 163.56, 132.05, 132.05, 122.86, 113.87, 113.87, 85.74, 84.69, 83.39, 81.74, 80.53, 80.15, 77.18, 61.40, 59.23, 58.22, 57.92, 55.99, 55.57, 54.03, 50.38, 49.28, 49.02, 48.99, 43.86, 43.20, 41.95, 39.72, 39.19, 35.68, 34.97, 29.22, 29.07, 26.39, 25.94, 25.54, 25.47, 21.82, 13.56.

### Example 12 Preparation of compound 12

100 mg of bulleyaconitine A was added into a 25 mL round-bottom flask, and dissolved with 5 mL of dry pyridine. Under an argon atmosphere, the reaction mixture was added with 2 equivalents of 2-furoyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 72 mg of a light yellow solid as a target compound 12 (C₄₀H₅₁NO₁₂, 63.5% yield), which was structurally shown as follows:

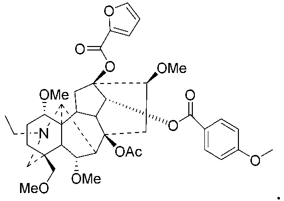

The target compound 12 was characterized as follows.

HRESIMS m/z : [(M + H)+, 738.36]

¹H NMR (400 MHz, CDCl₃) δ 8.05 (d, *J =* 8.8 Hz, 2H), 7.52 (dd, *J =* 1.6, 0.8 Hz, 1H), 7.08 (dd, *J* = 3.4, 0.8 Hz, 1H), 6.91 (d, *J* = 8.8 Hz, 2H), 6.43 (dd, *J* = 3.4, 1.6 Hz, 1H), 5.23 (d, *J =* 5.2 Hz, 1H), 4.12 (dd, *J =* 9.0, 5.8 Hz, 1H), 3.98 (d, *J =* 6.6 Hz, 1H), 3.84 (s, 3H), 3.62 (d, *J =* 8.4 Hz, 1H), 3.55 - 3.49 (m, 1H), 3.36 (s, 3H), 3.27 (s, 3H), 3.24 (s, 3H), 3.15 (s, 3H), 3.07 - 2.97 (m, 4H), 2.92 - 2.86 (m, 1H), 2.59 (dd, *J =* 12.2, 7.2 Hz, 1H), 2.53 (d, *J* = 5.8 Hz, 1H), 2.49 (d, *J* = 5.8 Hz, 2H), 2.47 - 2.43 (m, 1H), 2.33 - 2.25 (m, 1H), 2.14 - 2.08 (m, 2H), 2.07 (s, 1H), 1.97 - 1.89 (m, 1H), 1.65 - 1.61 (m, 1H), 1.61 - 1.54 (m, 1H), 1.33 (s, 3H), 1.20 - 1.14 (m, 1H), 1.10 (t, *J =* 7.2 Hz, 3H).

¹³C NMR (100 MHz, CDCl₃) δ 169.95, 166.29, 163.62, 157.88, 146.24, 144.95, 132.09, 132.09, 122.66, 118.08, 113.89, 113.89, 111.80, 85.56, 84.83, 83.38, 83.24, 80.55, 80.51, 77.12, 61.94, 59.23, 58.44, 57.94, 56.16, 55.57, 53.75, 50.39, 49.40, 49.27, 49.09, 43.99, 41.85, 39.78, 39.24, 35.65, 35.09, 26.43, 21.81, 13.62.

### Example 13 Preparation of compound 13

100 mg of compound 4 was added into a 25 mL round-bottom flask and dissolved with 5 mL of dry pyridine. Under an argon atmosphere, the reaction mixture was added with 2 equivalents of 4-methoxybenzoyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 118 mg of a light yellow foamy solid as a target compound 13 (C₄₃H₅₇NO₁₁, 76.4% yield), which was structurally shown as follows:

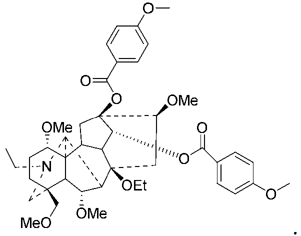

The target compound 13 was characterized as follows.

HRESIMS m/z : [(M + H)+, 764.41]

¹H NMR (400 MHz, CDCl₃) δ 8.09 (d, *J* = 8.8 Hz, 2H), 7.95 (d, *J* = 9.0 Hz, 2H), 6.91 (d, *J* = 8.8 Hz, 2H), 6.85 (d, *J* = 9.0 Hz, 2H), 5.24 (d, *J* = 5.2 Hz, 1H), 4.17 - 4.11 (m, 1H), 4.04 (d, *J* = 7.4 Hz, 1H), 3.85 (s, 3H), 3.81 (s, 3H), 3.63 (d, *J* = 8.2 Hz, 1H), 3.49 - 3.44 (m, 1H), 3.41 - 3.35 (m, 1H), 3.32 (s, 3H), 3.30 (s, 3H), 3.27 (s, 3H), 3.24 (s, 3H), 3.23 - 3.18 (m, 1H), 3.10 (d, *J =* 8.2 Hz, 1H), 3.03 (dd, *J =* 9.6, 6.2 Hz, 1H), 2.91 (s, 1H), 2.66 (t, *J=* 5.4 Hz, 1H), 2.53 (t, *J =* 9.6 Hz, 2H), 2.46 (d, *J =* 11.2 Hz, 2H), 2.41 - 2.33 (m, 3H), 2.09 (d, *J =* 8.4 Hz, 3H), 1.89 (dd, *J =* 11.8, 5.8 Hz, 1H), 1.64 (q, *J =* 5.4 Hz, 2H), 1.22 - 1.15 (m, 1H), 1.11 (t, *J =* 7.2 Hz, 3H), 0.64 (t, *J =* 6.8 Hz, 3H).

¹³C NMR (100 MHz, CDCl₃) δ 166.51, 165.61, 163.27, 163.21, 132.14, 132.14, 131.96, 131.96, 123.61, 123.48, 113.55, 113.55, 113.53, 113.53, 85.18, 83.35, 83.16, 80.78, 80.34, 78.18, 77.68, 60.90, 59.19, 58.86, 58.24, 56.25, 56.02, 55.51, 55.51, 54.20, 50.90, 49.08, 48.94, 48.23, 45.28, 42.37, 39.17, 37.69, 36.36, 35.03, 26.45, 15.49, 13.65.

### Example 14 Preparation of compound 14

100 mg of compound 2 was added into a 25 mL round-bottom flask and dissolved with 5 mL of dry pyridine. Under an argon atmosphere, the reaction mixture was added with 2 equivalents of cyclohexanecarbonyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 101 mg of a light yellow foamy solid as a target compound 14 (C₄₂H₆₁NO₁₀, 85.8% yield), which was structurally shown as follows:

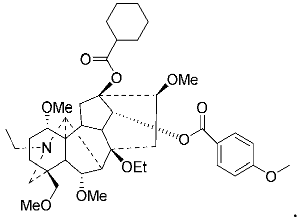

The target compound 14 was characterized as follows.

HRESIMS m/z : [(M + H)+, 740.45]

¹H NMR (400 MHz, CDCl₃) δ 8.05 (d, *J* = 8.8 Hz, 2H), 6.91 (d, *J* = 8.8 Hz, 2H), 5.03 (d, *J* = 5.2 Hz, 1H), 3.98 (dd, *J =* 12.2, 6.2 Hz, 2H), 3.85 (s, 3H), 3.61 (d, *J =* 8.2 Hz, 1H), 3.36 (s, 1H), 3.33 (s, 3H), 3.31 (s, 1H), 3.29 (s, 3H), 3.25 (s, 3H), 3.22 (s, 3H), 3.17 - 3.10 (m, 1H), 3.08 (d, *J =* 8.2 Hz, 1H), 3.00 (dd, *J* = 9.4*,* 6.2 Hz, 1H), 2.83 (s, 1H), 2.64 - 2.59 (m, 1H), 2.51 (t, *J =* 8.8 Hz, 2H), 2.48 - 2.41 (m, 2H), 2.35 - 2.33 (m, 1H), 2.31 - 2.26 (m, 3H), 2.06 (d, *J =* 6.4 Hz, 1H), 2.04 - 1.98 (m, 1H), 1.90 - 1.86 (m, 2H), 1.86 - 1.83 (m, 2H), 1.69 *(dd, J =* 9.6, 3.6 Hz, 2H), 1.62 *(dd, J =* 6.6, 4.8 Hz, 2H), 1.59 - 1.54 (m, 1H), 1.41 (d, *J* = 4.6 Hz, 1H), 1.40 - 1.37 (m, 1H), 1.33 (s, 1H), 1.28 (d, *J =* 2.6 Hz, 2H), 1.25 (s, 1H), 1.20 (d, *J* = 7.8 Hz, 2H), 1.07 (t, *J = 7.2* Hz, 3H), 0.57 (t, *J =* 6.8 Hz, 3H).

¹³C NMR (100 MHz, CDCl₃) δ 175.49, 166.38, 163.20, 132.05, 132.05, 123.57, 113.52, 113.52, 85.04, 83.32, 82.30, 80.44, 80.33, 78.15, 77.59, 60.73, 59.17, 58.82, 58.06, 56.09, 55.94, 55.50, 54.25, 50.83, 48.96, 48.84, 48.13, 45.03, 43.21, 42.32, 39.11, 37.56, 36.22, 34.93, 29.24, 29.06, 26.43, 25.97, 25.56, 25.47, 15.42, 13.59.

### Example 15 Preparation of compound 15

100 mg of compound 2 was added into a 25 mL round-bottom flask and dissolved with 5 mL of dry pyridine. Under an argon atmosphere, the reaction mixture was added with 2 equivalents of 2-furoyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 102 mg of a light yellow foamy solid as a target compound 15 (C₄₀H₅₃NO₁₁, 89% yield), which was structurally shown as follows:

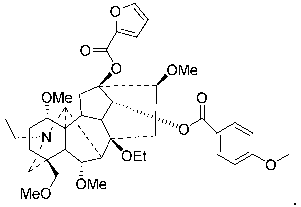

The target compound 15 was characterized as follows.

HRESIMS m/z : [(M + H)+, 724.38]

¹H NMR (400 MHz, CDCl₃) δ 8.06 (d, *J* = 8.8 Hz, 2H), 7.51 (dd, *J* = 1.8, 0.8 Hz, 1H), 7.08 (dd, *J* = 3.5*,* 0.8 Hz, 1H), 6.90 (d, *J =* 8.8 Hz, 2H), 6.42 (dd, *J* = 3.4, 1.6 Hz, 1H), 5.20 (d, *J= 5.2* Hz, 1H), 4.15 - 4.09 (m, 1H), 4.04 - 4.00 (m, 1H), 3.85 (s, 3H), 3.63 (d, *J* = 8.2 Hz, 1H), 3.48 - 3.44 (m, 1H), 3.36 (s, 3H), 3.29 (s, 3H), 3.26 (s, 3H), 3.23 (s, 3H), 3.18 (d, *J* = 14.8 Hz, 1H), 3.09 (d, *J* = 8.2 Hz, 1H), 3.02 (dd, *J =* 9.8, 6.2 Hz, 1H), 2.88 (s, 1H), 2.67 (dt, *J =* 6.2, 3.0 Hz, 1H), 2.52 (dd, *J* = 13.2, 7.2 Hz, 3H), 2.38 (d, *J* = 4.0 Hz, 2H), 2.37 - 2.32 (m, 2H), 2.08 (d, *J =* 5.2 Hz, 3H), 1.95 - 1.86 (m, 1H), 1.64 (q, *J =* 4.8 Hz, 2H), 1.26 (d, *J =* 11.2 Hz, 2H), 1.10 (t, *J =* 7.2 Hz, 3H), 0.61 (t, *J =* 6.8 Hz, 3H).

¹³C NMR (100 MHz, CDCl₃) δ 166.42, 163.27, 158.00, 146.14, 145.12, 132.11, 132.11, 123.36, 117.98, 113.55, 113.55, 111.78, 85.20, 83.82, 83.28, 80.79, 80.29, 78.08, 77.36, 61.34, 59.17, 58.86, 58.27, 56.28, 56.01, 55.50, 53.92, 50.85, 49.10, 48.31, 45.18, 42.22, 39.17, 37.60, 36.20, 35.11, 29.82, 26.47, 15.41, 13.64.

### Example 16 Preparation of compound 16

100 mg of compound 2 was added into a 25 mL round-bottom flask and dissolved with 5 mL of dry pyridine. Under an argon atmosphere, the reaction mixture was added with 2 equivalents of cyclopropylcarbonyl chloride in an ice water bath, and reacted at room temperature (about 20 °C), where the reaction was monitored through thin-layer chromatography. After reacted for about 12 h, the reaction mixture was subjected to rotary evaporation, and purified by column chromatography to obtain 103 mg of a light yellow foamy solid as a target compound 16 (C₃₉H₅₅NO₁₀, 93.5% yield), which was structurally shown as follows:

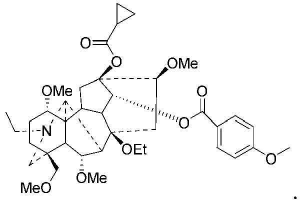

The target compound 16 was characterized as follows.

HRESIMS m/z : [(M + H)+, 698.40]

¹H NMR (400 MHz, CDCl₃) δ 8.05 (d, *J* = 8.8 Hz, 2H), 6.91 (d, *J* = 8.8 Hz, 2H), 5.06 (d, *J* = 5.2 Hz, 1H), 4.03 - 3.93 (m, 2H), 3.85 (s, 3H), 3.61 (d, *J* = 8.2 Hz, 1H), 3.36 (s, 3H), 3.32 (dd, *J* = 6.4*,* 1.6 Hz, 1H), 3.28 (s, 3H), 3.24 (s, 3H), 3.21 (s, 3H), 3.18 - 3.12 (m, 1H), 3.08 (d, *J =* 8.2 Hz, 1H), 3.00 (dd, *J* = 9.6*,* 6.2 Hz, 1H), 2.82 (s, 1H), 2.61 (t, *J* = 6.2 Hz, 1H), 2.54 - 2.47 (m, 2H), 2.48 - 2.40 (m, 2H), 2.36 - 2.31 (m, 2H), 2.30 (d, *J=* 3.8 Hz, 1H), 2.27 (d, *J* = 5.4 Hz, 1H), 2.08 - 2.04 (m, 1H), 2.01 (d, *J* = 5.6 Hz, 1H), 1.99 - 1.91 (m, 1H), 1.87 (dd, *J =* 12.0, 5.8 Hz, 1H), 1.62 (dd, *J =* 6.8, 4.8 Hz, 2H), 1.57 (dt, *J* = 8.0, 4.6 Hz, 1H), 1.25 (s, 1H), 1.07 (t, *J* = 7.2 Hz, 3H), 0.96 - 0.91 (m, 2H), 0.77 (dd, *J* = 8.2, 2.8 Hz, 2H), 0.57 (t, *J* = 6.8 Hz, 3H).

¹³C NMR (100 MHz, CDCl₃) δ 174.13, 166.40, 163.23, 132.10, 132.10, 123.48, 113.51, 113.51, 85.10, 83.29, 82.70, 80.69, 80.31, 78.10, 77.53, 60.82, 59.16, 58.82, 58.26, 56.12, 55.95, 55.50, 54.18, 50.82, 48.98, 48.88, 48.17, 45.12, 42.21, 39.12, 37.59, 36.25, 34.97, 26.43, 15.38, 13.60, 13.43, 8.56, 8.52.

### Example 17 Preparation of compound 17

100 mg of compound 4 was added into a 5 mL round-bottom flask and dissolved with 2 mL of dry pyridine. Under an argon atmosphere, the reaction mixture was added with 2 equivalents of thenoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 65.4 mg of a light yellow solid as a target compound 17 (C₃₇H₄₉NO₉S₂, 45.7% yield), which was structurally shown as follows:

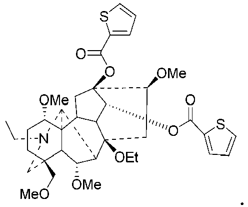

The target compound 17 was characterized as follows.

HRESIMS m/z : [(M + H)⁺, 716.28]

¹H NMR (400 MHz, Chloroform-d) δ 7.84 (dd, *J* = 3.6, 1.2 Hz, 1H), 7.74 (dd, *J* = 3.6, 1.2 Hz, 1H), 7.53 (dd, *J* = 5.0, 1.2 Hz, 1H), 7.48 (dd, *J* = 5.0, 1.2 Hz, 1H), 7.08 (dd, *J* = 5.0, 3.6 Hz, 1H), 7.03 (dd, *J =* 5.0, 3.6 Hz, 1H), 5.23 (d, *J =* 5.2 Hz, 1H), 4.09 (dd, *J* = 8.8*,* 6.4 Hz, 1H), 4.02 (dd, *J =* 6.6, 1.4 Hz, 1H), 3.65 (d, *J* = 8.2 Hz, 1H), 3.41 (dd, *J =* 13.2, 4.8 Hz, 2H), 3.33 (s, 3H), 3.30 (s, 3H), 3.28 (s, 3H), 3.24 (s, 3H), 3.08 (d, *J =* 8.2 Hz, 1H), 3.02 (dd, *J =* 9.6, 6.2 Hz, 1H), 2.87 (s, 1H), 2.64 (t, *J* = 6.2 Hz, 1H), 2.56 - 2.50 (m, 2H), 2.46 (d, *J =* 11.8 Hz, 2H), 2.43 - 2.39 (m, 2H), 2.31 (dd, *J* = 14.2, 8.8 Hz, 2H), 2.10 (d, *J* = 6.0 Hz, 3H), 1.90 (dd, *J* = 12.2, 6.0 Hz, 1H), 1.74 (s, 2H), 1.65 *(dd, J =* 13.0, 4.6 Hz, 2H), 1.10 (t, *J* = 7.2 Hz, 3H), 0.69 (t, *J =* 6.8 Hz, 3H).

¹³C NMR (100 MHz, CDCl₃) δ 162.24, 161.46, 134.70, 134.62, 133.69, 133.55, 132.40, 132.18, 127.70, 127.70, 85.12, 83.77, 83.30, 80.55, 80.27, 78.18, 77.84, 60.89, 59.17, 58.86, 58.05, 56.27, 56.10, 54.10, 50.84, 48.94, 48.89, 48.09, 45.22, 42.20, 39.11, 37.50, 36.12, 35.02, 26.43, 15.30, 13.61.

### Example 18 Preparation of compound 18

100 mg of compound 4 was added into a 5 mL round-bottom flask and dissolved with 2 mL of dry pyridine. Under an argon atmosphere, the reaction mixture was added with 2 equivalents of 5-chlorothiophene-2-carbonyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 87.4 mg of a light yellow solid as a target compound 18 (C₃₇H₄₇Cl₂NO₉S₂, 55.8% yield), which was structurally shown as follows:

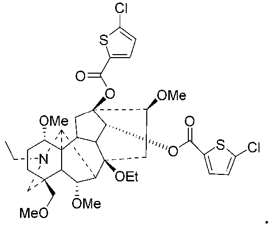

The target compound 18 was characterized as follows.

HRESIMS m/z: [(M + H)⁺, 784.20]

¹H NMR (400 MHz, Chloroform-d) δ 7.63 (d, *J=* 4.0 Hz, 1H), 7.52 (d, *J =* 4.0 Hz, 1H), 6.92 (d, *J* = 4.0 Hz, 1H), 6.87 (d, *J =* 4.0 Hz, 1H), 5.17 (d, *J =* 5.2 Hz, 1H), 4.08 - 3.97 (m, 2H), 3.65 (d, *J =* 8.2 Hz, 1H), 3.49 - 3.37 (m, 2H), 3.31 (s, 3H), 3.30 (s, 3H), 3.28 (s, 3H), 3.23 (s, 3H), 3.06 (d, *J* = 8.2 Hz, 1H), 3.01 (dd, *J* = 9.8, 6.2 Hz, 1H), 2.84 (s, 1H), 2.60 (t, *J* = 5.8 Hz, 1H), 2.50 (s, 2H), 2.48 (s, 2H), 2.41 (s, 1H), 2.32 (d, *J =* 6.8 Hz, 2H), 2.08 (dd, *J* = 11.6, 6.8 Hz, 3H), 1.90 (dd, *J =* 11.8, 5.8 Hz, 1H), 1.76 (s, 2H), 1.66 (d, *J =* 4.2 Hz, 1H), 1.61 (d, *J* = 5.8 Hz, 1H), 1.09 (t, *J* = 7.2 Hz, 3H), 0.78 (t, *J* = 6.8 Hz, 3H).

¹³C NMR (100 MHz, CDCl₃) δ 161.10, 160.37, 137.38, 137.11, 133.24, 133.11, 132.69, 132.58, 127.28, 127.24, 85.14, 84.05, 83.23, 80.28, 80.23, 78.12, 77.90, 61.03, 59.17, 58.91, 58.04, 56.25, 56.18, 53.98, 50.82, 49.00, 48.96, 48.13, 45.20, 42.13, 39.12, 37.52, 35.98, 35.10, 26.42, 15.51, 13.62.

### Example 19 Preparation of compound 19

100 mg of compound 2 was added into a 5 mL round-bottom flask and dissolved with 2 mL of dry pyridine. Under an argon atmosphere, the reaction mixture was added with 2 equivalents of thenoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 80.4 mg of a light yellow solid as a target compound 19 (C₄₀H₅₃NO₁₀S, 68.5% yield), which was structurally shown as follows:

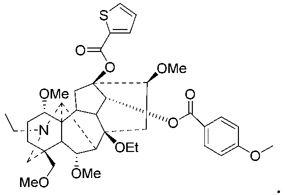

The target compound 19 was characterized as follows.

HRESIMS m/z : [(M + H)⁺, 740.33]

¹H NMR (400 MHz, Chloroform-d) δ 8.08 (d, *J =* 8.8 Hz, 2H), 7.73 (dd, *J* = 3.8, 1.2 Hz, 1H), 7.47 (dd, *J* = 4.8, 1.2 Hz, 1H), 7.02 (dd, *J =* 5.0, 3.6 Hz, 1H), 6.91 (d, *J =* 8.8 Hz, 2H), 5.22 (d, *J* = 5.2 Hz, 1H), 4.14 - 4.09 (m, 1H), 4.03 (d, *J* = 6.4 Hz, 1H), 3.85 (s, 3H), 3.63 (d, *J =* 8.2 Hz, 1H), 3.44 (d, *J =* 9.8 Hz, 1H), 3.38 (d, *J* = 8.6 Hz, 1H), 3.35 (s, 3H), 3.30 (s, 3H), 3.27 (s, 3H), 3.24 (s, 3H), 3.21 - 3.16 (m, 1H), 3.09 (d, *J =* 8.2 Hz, 1H), 3.03 (dd, *J* = 9.8, 6.2 Hz, 1H), 2.88 (s, 1H), 2.66 (t, *J =* 6.4 Hz, 1H), 2.53 (t, *J =* 8.4 Hz, 2H), 2.49 - 2.43 (m, 2H), 2.40 - 2.33 (m, 3H), 2.11 (d, *J =* 8.8 Hz, 2H), 1.90 (dd, *J =* 11.6, 5.8 Hz, 1H), 1.70 (s, 1H), 1.64 (dd, *J=* 11.6, 5.8 Hz, 2H), 1.28 (s, 1H), 1.10 (t, *J* = 7.2 Hz, 3H), 0.63 (t, *J* = 6.8 Hz, 3H).

¹³C NMR (100 MHz, CDCl₃) δ 166.45, 163.27, 161.52, 134.84, 133.47, 132.11, 132.11, 127.66, 123.42, 113.56, 113.56, 113.56, 85.16, 83.88, 83.32, 80.82, 80.32, 78.13, 77.36, 60.96, 59.18, 58.86, 58.29, 56.25, 56.03, 55.50, 54.13, 50.86, 49.05, 48.95, 48.24, 45.20, 42.31, 39.16, 37.65, 36.17, 35.04, 26.46, 15.46, 13.63.

### Example 20 Preparation of compound 20

100 mg of compound 2 was added into a 5 mL round-bottom flask and dissolved with 2 mL of dry pyridine. Under an argon atmosphere, the reaction mixture was added with 2 equivalents of 5-chlorothiophene-2-carbonyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 82.8 mg of a light yellow solid as a target compound 20 (C₄₀H₅₂ClNO₁₀S, 67.4% yield), which was structurally shown as follows:

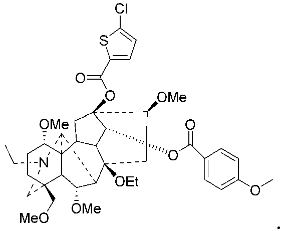

The target compound 20 was characterized as follows.

HRESIMS m/z: [(M + H)⁺, 774.30]

¹H NMR (400 MHz, Chloroform-d) δ 8.07 (d, *J =* 8.8 Hz, 2H), 7.51 (d, *J =* 4.0 Hz, 1H), 6.91 (d, *J =* 8.8 Hz, 2H), 6.85 (d, *J* = 4.0 Hz, 1H), 5.19 (d, *J =* 5.2 Hz, 1H), 4.09 - 4.00 (m, 2H), 3.86 (s, 3H), 3.64 (d, *J =* 8.2 Hz, 1H), 3.45 - 3.40 (m, 1H), 3.40 - 3.35 (m, 1H), 3.34 (s, 3H), 3.30 (s, 3H), 3.27 (s, 3H), 3.24 (s, 3H), 3.22 - 3.16 (m, 1H), 3.09 (d, *J* = 8.2 Hz, 1H), 3.03 (dd, *J =* 9.8, 6.2 Hz, 1H), 2.86 (s, 1H), 2.65 (t, *J =* 5.6 Hz, 1H), 2.57 - 2.49 (m, 2H), 2.49 - 2.42 (m, 2H), 2.35 (dd, *J =* 14.8, 8.2 Hz, 3H), 2.09 (d, *J =* 6.6 Hz, 3H), 1.91 (dd, *J =* 12.2, 6.2 Hz, 1H), 1.63 (s, 3H), 1.28 (s, 1H), 1.10 (t, *J* = 7.2 Hz, 3H), 0.64 (t, *J =* 6.8 Hz, 3H).

¹³C NMR (100 MHz, CDCl₃) δ 166.37, 163.32, 160.48, 136.99, 133.01, 132.96, 132.09, 132.09, 127.20, 123.35, 113.59, 113.59, 85.18, 84.21, 83.30, 80.66, 80.31, 78.10, 77.36, 61.03, 59.18, 58.87, 58.22, 56.24, 56.06, 55.52, 54.08, 50.86, 49.07, 48.98, 48.27, 45.21, 42.27, 39.17, 37.62, 36.13, 35.08, 26.47, 15.48, 13.65.

### Example 21 Preparation of compound 21

100 mg of bulleyaconitine A was added into a 5 mL round-bottom flask, and dissolved with 2 mL of dry pyridine. Under an argon atmosphere, the reaction mixture was added with 2 equivalents of thenoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 63.4 mg of a light yellow solid as a target compound 21 (C₄₀H₅₁NO₁₁S, 54.3% yield), which was structurally shown as follows:

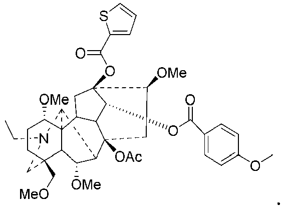

The target compound 21 was characterized as follows.

HRESIMS m/z: [(M + H)⁺, 754.31]

¹H NMR (400 MHz, Chloroform-d) δ 8.06 (d, *J =* 8.8 Hz, 2H), 7.72 (dd, *J =* 3.6, 1.2 Hz, 1H), 7.47 (dd, *J =* 5.0, 1.2 Hz, 1H), 7.02 (dd, *J =* 5.0, 3.6 Hz, 1H), 6.90 (d, *J =* 8.8 Hz, 2H), 5.24 (d, *J* = 5.2 Hz, 1H), 4.10 (dd, *J =* 8.8, 5.8 Hz, 1H), 3.98 (d, *J =* 6.6 40Hz, 1H), 3.84 (s, 3H), 3.62 (d, *J* = 8.4 Hz, 1H), 3.55 - 3.43 (m, 1H), 3.35 (s, 3H), 3.27 (s, 3H), 3.24 (s, 3H), 3.15 (s, 3H), 3.08 - 3.03 (m, 1H), 3.01 (d, *J* = 6.0 Hz, 3H), 2.89 (t, *J* = 6.4 Hz, 1H), 2.58 (dd, *J =* 12.2, 7.2 Hz, 1H), 2.51 (dd, *J* = 10.0, 5.8 Hz, 2H), 2.45 (dd, *J* = 14.8, 4.2 Hz, 2H), 2.35 - 2.23 (m, 1H), 2.12 (dd, *J* = 11.2, 5.8 Hz, 3H), 1.96 - 1.89 (m, 1H), 1.61 (dd,*J =* 12.8, 3.8 Hz, 2H), 1.34 (s, 3H), 1.27 (s, 1H), 1.09 (t, *J* = 7.2 Hz, 3H).

¹³C NMR (100 MHz, CDCl₃) δ 169.93, 166.29, 163.59, 161.36, 134.53, 133.53, 132.22, 132.06, 132.06, 127.66, 122.67, 113.87, 113.87, 85.57, 84.76, 83.36, 83.26, 80.55, 80.48, 77.09, 61.58, 59.20, 58.43, 57.92, 56.10, 55.54, 53.90, 50.38, 49.32, 49.10, 49.03, 43.98, 41.89, 39.77, 39.20, 35.60, 35.01, 26.37, 21.81, 13.56.

### Example 22 Preparation of compound 22

100 mg of bulleyaconitine A was added into a 5 mL round-bottom flask, and dissolved with 2 mL of dry pyridine. Under an argon atmosphere, the reaction mixture was added with 2 equivalents of 5-chlorothiophene-2-carbonyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 92.5 mg of a light yellow solid as a target compound 22 (C₄₀H₅₀ClNO₁₁S, 75.8% yield), which was structurally shown as follows:

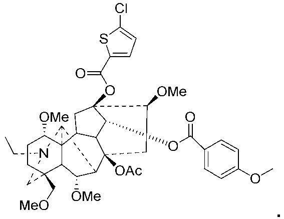

The target compound 22 was characterized as follows.

HRESIMS m/z: [(M + H)⁺, 788.27]

¹H NMR (400 MHz, Chloroform-d) δ 8.04 (d, *J=* 8.8 Hz, 2H), 7.51 (d, *J =* 4.0 Hz, 1H), 6.91 (d, *J =* 8.8 Hz, 2H), 6.85 (d, *J =* 4.0 Hz, 1H), 5.21 (d, *J* = 5.2 Hz, 1H), 4.05 (dd, *J* = 8.8*,* 5.8 Hz, 1H), 3.98 (d, *J* = 6.6 Hz, 1H), 3.84 (s, 3H), 3.62 (d, *J* = 8.4 Hz, 1H), 3.52 - 3.45 (m, 1H), 3.33 (s, 3H), 3.27 (s, 3H), 3.24 (s, 3H), 3.15 (s, 3H), 3.04 (d, *J=* 8.6 Hz, 1H), 3.00 (d, *J* = 12.4 Hz, 3H), 2.90 - 2.85 (m, 1H), 2.57 (dd, *J =* 12.2, 7.2 Hz, 1H), 2.53 - 2.40 (m, 4H), 2.35 - 2.23 (m, 1H), 2.17 - 2.05 (m, 3H), 1.98 - 1.88 (m, 1H), 1.80 (s, 1H), 1.69 - 1.54 (m, 2H), 1.35 (s, 3H), 1.09 (t, *J =* 7.2 Hz, 3H).

¹³C NMR (100 MHz, CDCl₃) δ 169.93, 166.21, 163.63, 160.31, 137.10, 133.08, 132.63, 132.04, 132.04, 127.20, 122.59, 113.90, 113.90, 85.51, 84.77, 83.57, 83.35, 80.47, 80.40, 76.95, 61.63, 59.21, 58.37, 57.94, 56.09, 55.55, 53.86, 50.37, 49.34, 49.12, 49.03, 43.96, 41.85, 39.73, 39.21, 35.54, 35.04, 26.39, 21.82, 13.57.

### Example 23 Preparation of compound 23

100 mg of compound 4 was added into a 5 mL round-bottom flask and dissolved with 2 mL of dry pyridine. Under an argon atmosphere, the reaction mixture was added with 2 equivalents of tetrahydro pyranoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 67.3 mg of a light yellow solid as a target compound 23 (C₃₉H₆₁NO₁₁, 46.8% yield), which was structurally shown as follows:

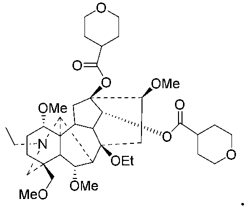

The target compound 23 was characterized as follows.

HRESIMS m/z : [(M + H)⁺, 720.42]

¹H NMR (400 MHz, Chloroform-d) δ 4.96 (d, *J* = 5.2 Hz, 1H), 3.97 (d, *J* = 5.8 Hz, 2H), 3.95 - 3.89 (m, 3H), 3.86 (t, *J =* 7.6 Hz, 1H), 3.64 (d, *J =* 8.2 Hz, 1H), 3.53 - 3.47 (m, 1H), 3.47 (d, *J =* 2.8 Hz, 1H), 3.45 - 3.40 (m, 3H), 3.40 - 3.32 (m, 2H), 3.29 (s, 3H), 3.29 (s, 3H), 3.23 (s, 3H), 3.20 (s, 3H), 3.03 (d, *J =* 8.2 Hz, 1H), 2.98 (dd, *J =* 9.4, 6.2 Hz, 1H), 2.77 (s, 1H), 2.54 (dd, *J* = 9.6, 5.2 Hz, 2H), 2.48 (dt, *J =* 11.2, 4.2 Hz, 3H), 2.43 (s, 1H), 2.40 (d, *J =* 4.6 Hz, 2H), 2.26 (dd, *J* = 13.8, 8.6 Hz, 2H), 2.16 (dd, *J =* 13.8, 6.8 Hz, 1H), 2.07 (d, *J* = 6.4 Hz, 1H), 1.97 (dd, *J =* 12.8, 5.8 Hz, 1H), 1.90 (s, 1H), 1.84 (dd, *J =* 8.6, 4.4 Hz, 3H), 1.78 (d, *J* = 4.2 Hz, 3H), 1.74 (d, *J* = 3.4 Hz, 3H), 1.68 - 1.57 (m, 2H), 1.11 (t, *J* = 6.8 Hz, 3H), 1.06 (t, *J* = 7.2 Hz, 3H).

¹³C NMR (100 MHz, CDCl₃) δ 174.13, 173.58, 84.92, 83.39, 82.48, 80.20, 79.82, 78.27, 67.32, 67.25, 67.20, 67.14, 60.55, 59.15, 58.88, 57.80, 56.14, 56.12, 54.19, 50.86, 48.82, 48.63, 47.66, 45.22, 42.23, 40.25, 39.98, 39.01, 37.51, 36.12, 34.89, 31.63, 28.85, 28.69, 28.64, 28.47, 26.36, 16.24, 13.55.

### Example 24 Preparation of compound 24

100 mg of compound 2 was added into a 5 mL round-bottom flask and dissolved with 2 mL of dry pyridine. Under an argon atmosphere, the reaction mixture was added with 2 equivalents of tetrahydro pyranoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 86.2 mg of a light yellow solid as a target compound 24 (C₄₁H₅₉NO₁₁, 73.2% yield), which was structurally shown as follows:

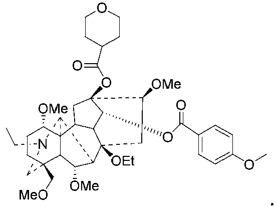

The target compound 24 was characterized as follows.

HRESIMS m/z: [(M + H)⁺, 742.40]

¹H NMR (400 MHz, Chloroform-d) δ 8.04 (d, *J =* 8.8 Hz, 2H), 6.91 (d, *J =* 8.8 Hz, 2H), 5.04 (d, *J =* 5.2 Hz, 1H), 3.98 (q, *J* = 7.0 Hz, 2H), 3.92 (t, *J =* 3.8 Hz, 1H), 3.90 - 3.87 (m, 1H), 3.86 (s, 3H), 3.62 (d, *J =* 8.2 Hz, 1H), 3.41 (dd, *J = 3.4,* 1.8 Hz, 1H), 3.38 (d, *J =* 3.6 Hz, 1H), 3.37 - 3.34 (m, 1H), 3.33 (s, 3H), 3.29 (s, 3H), 3.26 (s, 3H), 3.23 (s, 3H), 3.18 - 3.12 (m, 1H), 3.08 (d, *J =* 8.2 Hz, 1H), 3.01 (dd, *J =* 9.6, 6.2 Hz, 1H), 2.82 (s, 1H), 2.64 - 2.59 (m, 1H), 2.56 - 2.52 (m, 1H), 2.51 (s, 1H), 2.48 (d, *J =* 7.2 Hz, 1H), 2.45 (s, 1H), 2.43 (d, *J =* 3.0 Hz, 1H), 2.35 (s, 1H), 2.30 (dd, *J* = 7.8, 4.2 Hz, 2H), 2.07 (d, *J* = 6.6 Hz, 1H), 2.05 - 1.99 (m, 1H), 1.97 - 1.91 (m, 1H), 1.88 (t, *J =* 3.2 Hz, 1H), 1.83 - 1.76 (m, 3H), 1.74 (dt, *J* = 5.6, 2.8 Hz, 1H), 1.66 (s, 3H), 1.63 - 1.60 (m, 1H), 1.08 (t, *J =* 7.2 Hz, 3H), 0.60 (t, *J =* 6.8 Hz, 3H).

¹³C NMR (100 MHz, CDCl₃) δ 173.83, 166.31, 163.28, 132.01, 132.01, 123.44, 113.58, 113.58, 85.08, 83.31, 82.79, 80.31, 80.21, 78.15, 77.36, 67.23, 67.16, 60.82, 59.18, 58.85, 57.90, 56.11, 56.01, 55.52, 54.18, 50.85, 49.01, 48.89, 48.18, 45.04, 42.31, 40.00, 39.13, 37.53, 36.15, 34.99, 28.86, 28.80, 26.44, 15.47, 13.61.

### Example 25 Preparation of compound 25

100 mg of bulleyaconitine A was added into a 5 mL round-bottom flask and dissolved with 2 mL of dry pyridine. Under an argon atmosphere, the reaction mixture was added with 2 equivalents of tetrahydro pyranoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 65.9 mg of a light yellow solid as a target compound 25 (C₄₁H₅₇NO₁₂, 56.3% yield), which was structurally shown as follows:

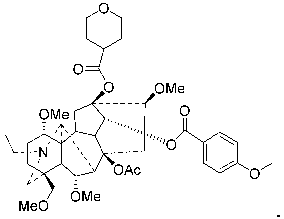

The target compound 25 was characterized as follows.

HRESIMS m/z: [(M + H)⁺, 756.38]

¹H NMR (400 MHz, Chloroform-d) δ 8.02 (d, *J =* 8.8 Hz, 2H), 6.91 (d, *J =* 8.8 Hz, 2H), 5.06 (d, *J =* 5.2 Hz, 1H), 3.99 - 3.93 (m, 2H), 3.93 - 3.87 (m, 2H), 3.85 (s, 3H), 3.61 (d, *J =* 8.4 Hz, 1H), 3.43 (dd, *J =* 7.8, 4.8 Hz, 1H), 3.40 (d, *J =* 2.2 Hz, 1H), 3.37 (d, *J =* 3.6 Hz, 1H), 3.32 (s, 3H), 3.27 (s, 3H), 3.23 (s, 3H), 3.14 (s, 3H), 3.12 (s, 1H), 2.98 (d, *J =* 6.2 Hz, 2H), 2.95 (s, 1H), 2.83 (dd, *J =* 7.2, 5.4 Hz, 1H), 2.58 - 2.53 (m, 1H), 2.53 - 2.50 (m, 1H), 2.50 (d, *J =* 4.8 Hz, 1H), 2.44 (t, *J* = 4.2 Hz, 2H), 2.42 - 2.39 (m, 1H), 2.34 - 2.22 (m, 1H), 2.13 - 2.03 (m, 2H), 1.92 (dd, *J =* 15.6, 12.2 Hz, 2H), 1.84 - 1.79 (m, 1H), 1.79 - 1.76 (m, 3H), 1.75 - 1.71 (m, 1H), 1.64 - 1.55 (m, 2H), 1.33 (s, 3H), 1.08 (t, *J =* 7.2 Hz, 3H).

¹³C NMR (100 MHz, CDCl₃) δ 173.70, 169.93, 166.16, 163.61, 131.98, 122.70, 113.90, 85.63, 84.69, 83.36, 82.18, 80.49, 79.92, 77.04, 67.18, 67.11, 61.45, 59.21, 58.06, 57.93, 55.98, 55.56, 53.96, 50.37, 49.29, 49.04, 48.99, 43.83, 41.91, 40.02, 39.65, 39.18, 35.59, 34.99, 31.55, 30.31, 28.83, 28.76, 26.38, 21.81, 13.55.

### Example 26 Preparation of compound 26

100 mg of compound 3 was added into a 5 mL round-bottom flask and dissolved with 2 mL of dry pyridine. Under an argon atmosphere, the reaction mixture was added with 2 equivalents of tetrahydro pyranoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 123.2 mg of a light yellow solid as a target compound 26 (C₃₈H₅₉NO₁₁, 83.2% yield), which was structurally shown as follows:

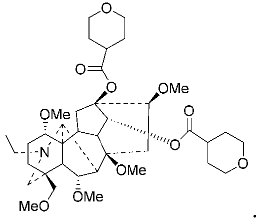

The target compound 26 was characterized as follows.

HRESIMS m/z : [(M + H)⁺, 706.40]

¹H NMR (400 MHz, Chloroform-d) δ 4.91 (d, *J* = 5.2 Hz, 1H), 3.97 (t, *J =* 3.8 Hz, 1H), 3.93 (d, *J =* 3.6 Hz, 3H), 3.90 (d, *J =* 3.8 Hz, 1H), 3.87 (d, *J* = 8.2 Hz, 1H), 3.63 (d, *J* = 8.2 Hz, 1H), 3.46 (dd, *J* = 4.8, 2.4 Hz, 1H), 3.45 - 3.42 (m, 2H), 3.42 - 3.37 (m, 2H), 3.30 (s, 3H), 3.29 (s, 3H), 3.25 (s, 3H), 3.20 (s, 3H), 3.15 (s, 3H), 3.08 (d, *J =* 8.2 Hz, 1H), 2.98 (dd, *J =* 9.4, 6.2 Hz, 1H), 2.78 (s, 1H), 2.55 (t, *J* = 4.6 Hz, 1H), 2.52 (s, 1H), 2.50 - 2.47 (m, 1H), 2.46 (d, *J* = 4.4 Hz, 1H), 2.44 (d, *J =* 2.4 Hz, 1H), 2.43 - 2.41 (m, 1H), 2.39 (t, *J =* 6.2 Hz, 2H), 2.29 - 2.24 (m, 1H), 2.21 (d, *J* = 8.6 Hz, 1H), 2.15 (d, *J =* 6.8 Hz, 1H), 2.04 (d, *J =* 6.4 Hz, 1H), 1.97 (ddd, *J =* 12.4, 7.2, 5.2 Hz, 1H), 1.88 (s, 1H), 1.84 (q, *J =* 2.8, 2.0 Hz, 3H), 1.81 (d, *J =* 3.6 Hz, 1H), 1.78 (d, *J =* 2.8 Hz, 1H), 1.76 (t, *J =* 5.2 Hz, 3H), 1.74 - 1.70 (m, 1H), 1.65 - 1.57 (m, 2H), 1.07 (t, *J =* 7.2 Hz, 3H).

¹³C NMR (100 MHz, CDCl₃) δ 174.22, 173.58, 84.89, 83.22, 82.43, 80.30, 79.86, 78.45, 77.03, 67.30, 67.27, 67.19, 67.15, 60.68, 59.17, 58.85, 57.82, 56.04, 54.18, 50.81, 48.87, 48.66, 48.14, 48.14, 44.95, 42.25, 40.30, 40.01, 39.07, 36.87, 36.15, 34.91, 28.83, 28.77, 28.70, 28.54, 26.39, 13.58.

### Example 27 Preparation of compound 27

100 mg of compound 4 was added into a 5 mL round-bottom flask and dissolved with 2 mL of dry pyridine. Under an argon atmosphere, the reaction mixture was added with 2 equivalents of nicotinoyl chloride hydrochloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 102.3 mg of a light yellow solid as a target compound 27 (C₃₉H₅₁N₃0₉, 72.5% yield), which was structurally shown as follows:

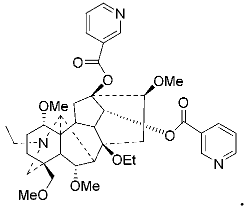

The target compound 27 was characterized as follows.

HRESIMS m/z: [(M + H)⁺, 706.36]

¹H NMR (400 MHz, Chloroform-d) δ 9.31 (d, *J =* 1.2 Hz, 1H), 9.16 (d, *J =* 1.2 Hz, 1H), 8.75 (dd, *J* = 5.0, 1.8 Hz, 1H), 8.70 (dd, *J* = 5.0, 1.8 Hz, 1H), 8.36 (dt, *J* = 7.8, 2.0 Hz, 1H), 8.22 (dt, *J* = 7.8*,* 2.0 Hz, 1H), 7.38 (ddd, *J =* 8.0, 4.8, 0.8 Hz, 1H), 7.31 (ddd, *J =* 8.0, 4.8, 0.8 Hz, 1H), 5.32 (d, *J* = 5.2 Hz, 1H), 4.14 *(t, J =* 7.4 Hz, 1H), 4.01 (d, *J =* 6.4 Hz, 1H), 3.64 (d, *J* = 8.2 Hz, 1H), 3.49 (d, *J* = 9.8 Hz, 1H), 3.44 - 3.35 (m, 1H), 3.29 (s, 6H), 3.27 (s, 3H), 3.24 (s, 3H), 3.22 - 3.17 (m, 1H), 3.04 (dd, *J* = 16.2, 7.2 Hz, 2H), 2.89 (s, 1H), 2.67 (t, *J* = 5.6 Hz, 1H), 2.56 - 2.50 (m, 2H), 2.47 (d, *J* = 4.8 Hz, 1H), 2.45 (s, 1H), 2.41 (s, 1H), 2.38 (d, *J=* 2.8 Hz, 1H), 2.36 (s, 1H), 2.14 - 2.08 (m, 3H), 2.00 - 1.87 (m, 2H), 1.67 (d, *J* = 3.4 Hz, 1H), 1.62 (q, *J =* 5.6, 4.6 Hz, 1H), 1.09 (t, *J =* 7.2 Hz, 3H), 0.63 (t, *J =* 6.8 Hz, 3H).

¹³C NMR (100 MHz, CDCl₃) δ 165.15, 164.35, 153.23, 153.14, 151.49, 151.18, 137.35, 137.21, 126.66, 126.48, 123.21, 123.15, 85.11, 84.01, 83.07, 80.10, 80.05, 78.06, 77.81, 61.06, 59.03, 58.83, 58.02, 56.08, 56.02, 53.81, 50.74, 49.08, 48.93, 48.22, 45.20, 42.12, 39.06, 37.54, 36.01, 35.12, 26.33, 15.45, 13.54.

### Example 28 Preparation of compound 28

100 mg of compound 2 was added into a 5 mL round-bottom flask and dissolved with 2 mL of dry pyridine. Under an argon atmosphere, the reaction mixture was added with 2 equivalents of nicotinoyl chloride hydrochloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 96.9 mg of a light yellow solid as a target compound 28 (C₄₁H₅₄N₂O₁₀, 83.1% yield), which was structurally shown as follows:

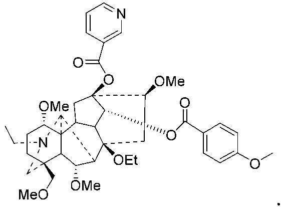

The target compound 28 was characterized as follows.

HRESIMS m/z: [(M + H)⁺, 735.37]

¹H NMR (400 MHz, Chloroform-d) δ 9.17 (d, *J* = 1.6 Hz, 1H), 8.71 (dd, *J* = 4.8, 1.8 Hz, 1H), 8.24 (dt, *J =* 8.0, 2.0 Hz, 1H), 8.07 (d, *J =* 8.8 Hz, 2H), 7.31 (dd, *J =* 8.0, 4.8 Hz, 1H), 6.91 (d, *J* = 9.0 Hz, 2H), 5.26 (d, *J* = 5.2 Hz, 1H), 4.12 (t, *J* = 7.6 Hz, 1H), 4.04 (d, *J* = 6.6 Hz, 1H), 3.85 (s, 3H), 3.64 (d, *J =* 8.2 Hz, 1H), 3.58 - 3.46 (m, 2H), 3.39 (p, *J =* 7.2 Hz, 2H), 3.31 (s, 3H), 3.30 (s, 3H), 3.28 (s, 3H), 3.25 (s, 3H), 3.23 - 3.18 (m, 1H), 3.09 (d, *J =* 8.2 Hz, 1H), 3.04 (dd, *J* = 9.8, 6.2 Hz, 1H), 2.89 (s, 1H), 2.70 - 2.64 (m, 1H), 2.53 (t, *J* = 3.6 Hz, 1H), 2.51 - 2.44 (m, 2H), 2.40 (s, 1H), 2.36 (d, *J* = 8.6 Hz, 2H), 2.16 - 2.06 (m, 3H), 1.91 (dd, *J =* 12.2, 6.0 Hz, 1H), 1.71 (s, 2H), 1.11 (t, *J =* 7.2 Hz, 3H), 0.66 (t, *J =* 6.8 Hz, 3H).

¹³C NMR (100 MHz, CDCl₃) δ 166.39, 164.56, 163.35, 153.19, 151.36, 137.40, 132.10, 132.10, 126.93, 123.27, 123.26, 113.61, 113.61, 85.23, 84.14, 83.30, 80.44, 80.29, 78.14, 77.36, 61.10, 59.18, 58.89, 58.10, 56.24, 56.09, 55.53, 54.05, 50.90, 49.12, 49.03, 48.30, 45.26, 42.29, 39.18, 37.62, 36.22, 35.11, 26.48, 15.50, 13.66.

### Example 29 Preparation of compound 29

100 mg of bulleyaconitine A was added into a 5 mL round-bottom flask and dissolved with 2 mL of dry pyridine. Under an argon atmosphere, the reaction mixture was added with 2 equivalents of nicotinoyl chloride hydrochloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 66.9 mg of a light yellow solid as a target compound 29 (C₄₁H₅₂N₂O₁₁, 57.7% yield), which was structurally shown as follows:

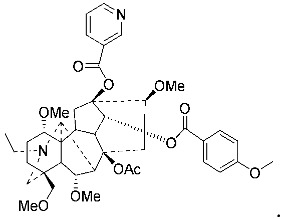

The target compound 29 was characterized as follows.

HRESIMS m/z: [(M + H)⁺, 749.35]

¹H NMR (400 MHz, Chloroform-d) δ 9.15 (d, *J =* 1.2 Hz, 1H), 8.70 (dd, *J =* 4.8, 1.6 Hz, 1H), 8.21 (dt, *J =* 8.0, 1.8 Hz, 1H), 8.04 (d, *J* = 8.8 Hz, 2H), 7.30 (ddd, *J* = 8.0, 4.8, 0.8 Hz, 1H), 6.90 (d, *J* = 8.8 Hz, 2H), 5.29 - 5.26 (m, 1H), 4.11 (dd, *J* = 8.8, 5.8 Hz, 1H), 3.98 (d, *J =* 6.8 Hz, 1H), 3.83 (s, 3H), 3.62 (d, *J =* 8.4 Hz, 1H), 3.59 - 3.53 (m, 1H), 3.30 (s, 3H), 3.26 (s, 3H), 3.24 (s, 3H), 3.15 (s, 3H), 3.12 (s, 1H), 3.03 (t, *J =* 4.6 Hz, 2H), 3.00 (s, 1H), 2.89 (dd, *J* = 7.4, 5.2 Hz, 1H), 2.58 (dd, *J* = 12.2, 7.2 Hz, 1H), 2.52 (d, *J =* 5.8 Hz, 1H), 2.49 (d, *J =* 4.2 Hz, 1H), 2.47 (d, *J =* 2.8 Hz, 1H), 2.44 (t, *J* = 6.0 Hz, 1H), 2.29 (dt, *J* = 12.2, 6.2 Hz, 1H), 2.18 - 2.06 (m, 3H), 1.98 (s, 1H), 1.92 (dt, *J =* 10.4, 6.2 Hz, 1H), 1.67 - 1.56 (m, 2H), 1.36 (s, 3H), 1.09 (t, *J =* 7.2 Hz, 3H).

¹³C NMR (100 MHz, CDCl₃) δ 169.92, 166.20, 164.42, 163.64, 153.23, 151.26, 137.32, 132.02, 132.02, 126.73, 123.23, 122.53, 113.91, 113.91, 85.52, 84.81, 83.48, 83.34, 80.45, 80.16, 76.96, 61.67, 59.19, 58.23, 57.95, 56.07, 55.54, 53.83, 50.40, 49.34, 49.14, 49.07, 43.98, 41.86, 39.71, 39.20, 35.61, 35.04, 26.39, 21.82, 13.58.

### Example 30 Preparation of compound 30

100 mg of compound 3 was added into a 5 mL round-bottom flask and dissolved with 2 mL of dry pyridine. Under an argon atmosphere, the reaction mixture was added with 2 equivalents of nicotinoyl chloride hydrochloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 109.3 mg of a light yellow solid as a target compound 30 (C₃₈H₄₉N₃O₉, 75.3% yield), which was structurally shown as follows:

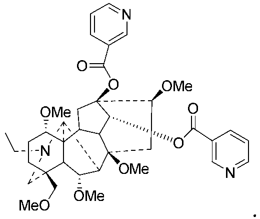

The target compound 30 was characterized as follows.

HRESIMS m/z : [(M + H)⁺, 692.34]

¹H NMR (400 MHz, Chloroform-d) δ 9.29 (d, *J* = 1.4 Hz, 1H), 9.16 (d, *J =* 1.4 Hz, 1H), 8.75 (dd, *J* = 4.8, 1.8 Hz, 1H), 8.71 (dd, *J =* 4.8, 1.6 Hz, 1H), 8.35 (dt, *J =* 7.8, 1.8 Hz, 1H), 8.23 (dt, *J* = 8.0, 2.0 Hz, 1H), 7.42 - 7.36 (m, 1H), 7.32 (dd, *J* = 7.8, 5.6 Hz, 1H), 5.39 (d, *J* = 5.2 Hz, 1H), 4.16 (t, *J =* 7.4 Hz, 1H), 3.99 (d, *J =* 6.6 Hz, 1H), 3.66 (d, *J =* 8.2 Hz, 1H), 3.50 (d, *J* = 9.4 Hz, 1H), 3.30 (s, 3H), 3.29 (s, 6H), 3.25 (s, 3H), 3.11 (d, *J* = 8.2 Hz, 1H), 3.06 (s, 3H), 2.92 (s, 1H), 2.63 - 2.54 (m, 3H), 2.49 (d, *J =* 9.6 Hz, 2H), 2.44 (s, 1H), 2.39 (s, 1H), 2.37 (d, *J =* 3.8 Hz, 1H), 2.16 - 2.08 (m, 3H), 1.93 (s, 2H), 1.84 (s, 1H), 1.69 - 1.59 (m, 2H), 1.11 (t, *J* = 7.2 Hz, 3H).

¹³C NMR (100 MHz, CDCl₃) δ 165.38, 164.48, 153.31, 153.28, 151.59, 151.32, 137.55, 137.36, 126.73, 126.55, 123.37, 123.29, 85.18, 84.19, 83.09, 80.31, 80.02, 78.43, 77.70, 61.28, 59.20, 58.89, 58.16, 56.20, 53.96, 50.84, 49.11, 48.78, 48.58, 48.51, 45.37, 42.32, 39.20, 36.83, 36.11, 35.21, 26.44, 13.69.

### Example 31 Preparation of compound 31

100 mg of compound 3 was added into a 5 mL round-bottom flask and dissolved with 2 mL of dry pyridine. Under an argon atmosphere, the reaction mixture was added with 2 equivalents of 4-methoxybenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 101.5 mg of a light yellow solid as a target compound 31 (C₄₂H₅₅NO₁₁, 64.5% yield), which was structurally shown as follows:

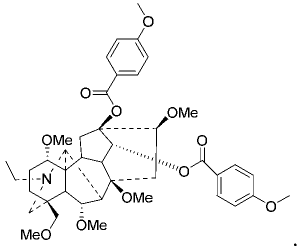

The target compound 31 was characterized as follows.

HRESIMS m/z : [(M + H)⁺, 750.37]

¹H NMR (400 MHz, Chloroform-d) δ 8.07 (d, *J=* 8.8 Hz, 2H), 7.94 (d, *J* = 8.8 Hz, 2H), 6.91 (d, *J* = 8.8 Hz, 2H), 6.84 (d, *J* = 8.8 Hz, 2H), 5.30 (d, *J =* 5.2 Hz, 1H), 4.15 (dd, *J* = 8.8, 6.2 Hz, 1H), 4.01 (d, *J* = 6.2 Hz, 1H), 3.84 (s, 3H), 3.80 (s, 3H), 3.64 (d, *J* = 8.2 Hz, 1H), 3.51 - 3.42 (m, 1H), 3.31 (s, 3H), 3.29 (s, 3H), 3.28 (s, 3H), 3.24 (s, 3H), 3.13 (d, *J* = 8.2 Hz, 1H), 3.04 (s, 3H), 2.92 (s, 1H), 2.62 - 2.52 (m, 3H), 2.48 (dq, *J* = 12.0, 6.2, 5.4 Hz, 2H), 2.40 (d, *J* = 14.4 Hz, 2H), 2.32 (dd, *J* = 14.4, 8.8 Hz, 2H), 2.14 - 2.03 (m, 3H), 1.89 (dd, *J* = 11.8, 6.0 Hz, 1H), 1.81 (s, 1H), 1.64 (dd, *J* = 9.4*,* 4.2 Hz, 2H), 1.11 (t, *J* = 7.2 Hz, 3H).

¹³C NMR (100 MHz, CDCl₃) δ 166.51, 165.60, 163.24, 163.21, 132.14, 132.14, 131.95, 131.95, 123.52, 123.35, 113.57, 113.57, 113.52, 113.52, 85.10, 83.21, 83.16, 80.57, 80.40, 78.45, 77.42, 60.96, 59.19, 58.75, 58.22, 56.21, 55.48, 55.48, 54.21, 50.86, 48.97, 48.63, 48.44, 48.38, 45.27, 42.44, 39.16, 36.85, 36.28, 34.97, 26.42, 13.65.

### Example 32 Preparation of compound 32

100 mg of compound 3 was added into a 5 mL round-bottom flask and dissolved with 2 mL of dry pyridine. Under an argon atmosphere, the reaction mixture was added with 2 equivalents of 2-furoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 92.6 mg of a light yellow solid as a target compound 32 (C₃₆H₄₇NO₁₁, 65.9% yield), which was structurally shown as follows:

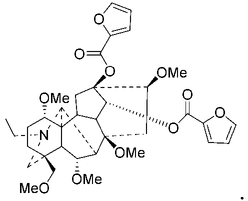

The target compound 32 was characterized as follows.

HRESIMS m/z : [(M + H)⁺, 670.31]

¹H NMR (400 MHz, Chloroform-d) δ 7.58 (dd, *J =* 1.8, 0.8 Hz, 1H), 7.52 (dd, *J =* 1.8, 0.8 Hz, 1H), 7.17 (dd, *J =* 3.4, 0.8 Hz, 1H), 7.11 (dd, *J =* 3.4, 0.8 Hz, 1H), 6.48 (dd, *J =* 3.4, 1.6 Hz, 1H), 6.44 (dd, *J* = 3.4, 1.6 Hz, 1H), 5.27 (d, *J* = 5.2 Hz, 1H), 4.11 (dd, *J* = 8.4, 6.8 Hz, 1H), 3.98 (d, *J =* 5.8 Hz, 1H), 3.64 (d, *J =* 8.2 Hz, 1H), 3.50 (q, *J =* 12.2 Hz, 1H), 3.34 (s, 3H), 3.29 (s, 3H), 3.28 (s, 3H), 3.23 (s, 3H), 3.12 (d, *J =* 8.2 Hz, 1H), 3.06 (s, 3H), 3.01 (dd, *J* = 9.8, 6.2 Hz, 1H), 2.88 (s, 1H), 2.60 - 2.54 (m, 2H), 2.53 - 2.47 (m, 2H), 2.41 (s, 1H), 2.38 (d, *J =* 6.4 Hz, 1H), 2.35 - 2.26 (m, 2H), 2.07 (d, *J =* 6.0 Hz, 3H), 1.91 (dd, *J =* 12.0, 6.2 Hz, 1H), 1.63 (dd, *J* = 9.6, 4.2 Hz, 2H), 1.17 (t, *J* = 7.2 Hz, 1H), 1.11 (t, *J* = 7.2 Hz, 3H).

¹³C NMR (100 MHz, CDCl₃) δ 158.73, 157.92, 146.41, 146.21, 145.21, 145.00, 118.26, 118.20, 111.83, 111.79, 85.07, 83.67, 83.22, 80.56, 80.36, 78.44, 77.36, 61.34, 59.21, 58.80, 58.24, 56.25, 53.93, 50.82, 49.14, 48.61, 48.53, 48.36, 45.12, 42.18, 39.17, 36.69, 36.14, 35.08, 26.49, 13.67.

### Example 33 Preparation of compound 33

100 mg of compound 1 was added into a 5 mL round-bottom flask and dissolved with 2 mL of dry pyridine. Under an argon atmosphere, the reaction mixture was added with 2 equivalents of furoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 73.4 mg of a light yellow solid as a target compound 33 (C₃₉H₅₁NO₁₁, 63.7% yield), which was structurally shown as follows:

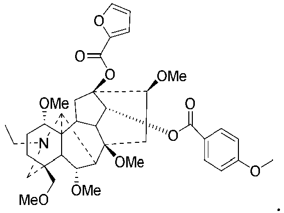

The target compound 33 was characterized as follows.

HRESIMS m/z : [(M + H)⁺, 710.34]

¹H NMR (400 MHz, Chloroform-d) δ 8.05 (d, *J =* 8.8 Hz, 2H), 7.51 (dd, *J* = 1.6, 0.8 Hz, 1H), 7.08 (dd, *J* = 3.4, 0.8 Hz, 1H), 6.91 (d, *J* = 8.8 Hz, 2H), 6.42 (dd, *J* = 3.4, 1.6 Hz, 1H), 5.25 (d, *J =* 5.2 Hz, 1H), 4.13 (dd, *J* = 8.8, 6.2 Hz, 1H), 4.00 (d, *J =* 6.2 Hz, 1H), 3.85 (s, 3H), 3.64 (d, *J* = 8.4 Hz, 1H), 3.52 - 3.40 (m, 1H), 3.36 (s, 3H), 3.29 (s, 3H), 3.27 (s, 3H), 3.24 (s, 3H), 3.13 (d, *J =* 8.2 Hz, 1H), 3.02 (s, 3H), 2.90 (s, 1H), 2.58 (dt, *J =* 14.0, 6.6 Hz, 2H), 2.53 - 2.45 (m, 2H), 2.45 - 2.37 (m, 2H), 2.32 (dd, *J =* 14.0, 8.8 Hz, 2H), 2.08 (d, *J = 5.8* Hz, 3H), 1.92 (s, 1H), 1.80 - 1.57 (m, 3H), 1.26 (d, *J=* 11.6 Hz, 1H), 1.11 (t, *J* = 7.0 Hz, 3H).

¹³C NMR (100 MHz, CDCl₃) δ 166.48, 163.26, 158.00, 146.17, 145.09, 132.14, 132.14, 123.27, 118.05, 113.59, 113.59, 111.79, 85.16, 83.83, 83.16, 80.60, 80.38, 78.37, 77.36, 77.28, 61.42, 59.21, 58.79, 58.28, 56.28, 55.51, 53.96, 50.83, 49.16, 48.64, 48.42, 45.18, 42.31, 39.18, 36.81, 36.13, 35.09, 26.48, 13.69.

### Example 34 Preparation of compound 34

100 mg of compound 3 was added into a 5 mL round-bottom flask and dissolved with 2 mL of dry pyridine. Under an argon atmosphere, the reaction mixture was added with 2 equivalents of cyclohexanecarbonyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 63.9 mg of a light yellow solid as a target compound 34 (C₄₀H₆₃NO₉, 43.4% yield), which was structurally shown as follows:

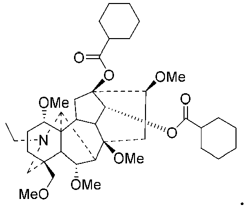

The target compound 34 was characterized as follows.

HRESIMS m/z : [(M + H)⁺, 702.45]

¹H NMR (400 MHz, Chloroform-d) δ 4.86 (d, *J* = 5.2 Hz, 1H), 3.94 (dd, *J* = 6.6, 1.6 Hz, 1H), 3.86 (t, *J* = 7.8 Hz, 1H), 3.61 (d, *J* = 8.2 Hz, 1H), 3.32 (d, *J* = 5.4 Hz, 1H), 3.28 (s, 3H), 3.28 (s, 3H), 3.26 (s, 3H), 3.19 (s, 3H), 3.14 (s, 3H), 3.09 (d, *J* = 8.2 Hz, 1H), 2.97 (dd, *J =* 9.2, 6.2 Hz, 1H), 2.78 (s, 1H), 2.56 - 2.47 (m, 2H), 2.46 (d, *J* = 4.4 Hz, 1H), 2.44 - 2.38 (m, 2H), 2.37 (s, 2H), 2.32 (q, *J* = 3.6 Hz, 1H), 2.29 (t, *J* = 3.6 Hz, 1H), 2.27 - 2.24 (m, 1H), 2.24 - 2.20 (m, 1H), 2.18 (dd, *J* = 7.8, 3.8 Hz, 2H), 2.02 (d, *J* = 6.6 Hz, 1H), 1.97 - 1.88 (m, 3H), 1.86 - 1.81 (m, 3H), 1.80 (d, *J =* 3.2 Hz, 1H), 1.74 - 1.69 (m, 3H), 1.61 (s, 2H), 1.59 (d, *J* = 4.4 Hz, 2H), 1.49 - 1.41 (m, 2H), 1.41 - 1.34 (m, 2H), 1.28 (d, *J* = 3.6 Hz, 2H), 1.24 - 1.18 (m, 3H), 1.06 (t, *J* = 7.2 Hz, 3H).

¹³C NMR (100 MHz, CDCl₃) δ 175.97, 175.28, 84.81, 83.31, 81.89, 80.35, 80.18, 78.45, 76.88, 60.48, 59.16, 58.77, 57.90, 56.04, 54.35, 50.81, 48.78, 48.56, 48.01, 47.99, 44.84, 43.24, 43.16, 42.29, 39.02, 36.79, 36.21, 34.72, 29.23, 29.06, 29.00, 28.76, 26.36, 26.06, 26.00, 25.65, 25.56, 25.50, 25.45, 13.54.

### Example 35 Preparation of compound 35

100 mg of compound 4 was added into a 5 mL round-bottom flask and dissolved with 2 mL of dry pyridine. Under an argon atmosphere, the reaction mixture was added with 2 equivalents of 4-ethoxybenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 84.2 mg of a light yellow solid as a target compound 35 (C₄₅H₆₁NO₁₁, 53.2% yield), which was structurally shown as follows:

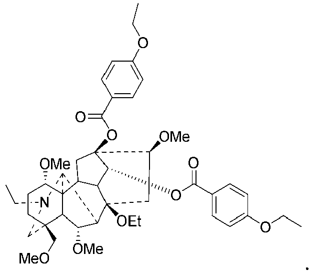

The target compound 35 was characterized as follows.

HRESIMS *m*/*z:* [(M + H)⁺, 792.42]

¹H NMR (400 MHz, Chloroform-d) δ 8.07 (d, *J =* 8.8 Hz, 2H), 7.93 (d, *J* = 8.8 Hz, 2H), 6.89 (d, *J* = 8.8 Hz, 2H), 6.83 (d, *J =* 8.8 Hz, 2H), 5.24 (d, *J =* 5.2 Hz, 1H), 4.22 - 4.12 (m, 1H), 4.06 (dq, *J =* 14.2, 7.0 Hz, 5H), 3.63 (d, *J = 8.2* Hz, 1H), 3.46 (d, *J =* 9.6 Hz, 1H), 3.40 - 3.34 (m, 1H), 3.32 (s, 3H), 3.30 (s, 3H), 3.27 (s, 3H), 3.24 (s, 3H), 3.22 - 3.17 (m, 1H), 3.09 (d, *J =* 8.2 Hz, 1H), 3.02 (dd, *J =* 9.6*,* 6.2 Hz, 1H), 2.90 (s, 1H), 2.65 (t, *J =* 5.2 Hz, 1H), 2.57 - 2.50 (m, 2H), 2.49 - 2.42 (m, 2H), 2.40 - 2.33 (m, 3H), 2.12 - 2.04 (m, 3H), 1.72 (s, 2H), 1.67 - 1.59 (m, 2H), 1.46 - 1.41 (m, 3H), 1.41 - 1.37 (m, 3H), 1.10 *(t, J =* 7.0 Hz, 3H), 0.63 (t, *J =* 7.0 Hz, 3H).

¹³C NMR (100 MHz, CDCl₃) δ 166.57, 165.65, 162.68, 162.62, 132.12, 132.12, 131.95, 131.95, 123.36, 123.25, 114.01, 114.01, 113.99, 112.99, 85.19, 83.34, 83.11, 80.80, 80.33, 78.18, 77.66, 77.36, 63.72, 60.90, 59.18, 58.85, 58.24, 56.26, 56.02, 54.20, 50.89, 49.06, 48.94, 48.20, 45.27, 42.35, 39.16, 37.68, 36.37, 35.01, 26.44, 15.47, 14.83, 14.80, 13.64.

### Example 36 Preparation of compound 36

100 mg of compound 2 was added into a 5 mL round-bottom flask and dissolved with 2 mL of dry pyridine. Under an argon atmosphere, the reaction mixture was added with 2 equivalents of 4-ethoxybenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 104.3 mg of a light yellow solid as a target compound 36 (C₄₄H₅₉NO₁₁, 84.5% yield), which was structurally shown as follows:

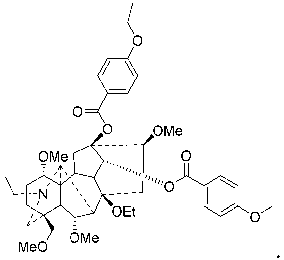

The target compound 36 was characterized as follows.

HRESIMS *m*/*z:* [(M + H)⁺, 778.40]

¹H NMR (400 MHz, Chloroform-d) δ 8.08 (d, *J =* 8.8 Hz, 2H), 7.93 (d, *J =* 8.8 Hz, 2H), 6.91 (d, *J* = 8.8 Hz, 2H), 6.83 (d, *J =* 8.8 Hz, 2H), 5.24 (d, *J* = 5.2 Hz, 1H), 4.18 - 4.10 (m, 1H), 4.04 (q, *J* = 7.0 Hz, 3H), 3.85 (s, 3H), 3.63 (d, *J* = 8.2 Hz, 1H), 3.49 - 3.42 (m, 1H), 3.40 - 3.34 (m, 1H), 3.32 (s, 3H), 3.30 (s, 3H), 3.27 (s, 3H), 3.24 (s, 3H), 3.22 - 3.17 (m, 1H), 3.09 (d, *J =* 8.2 Hz, 1H), 3.02 (dd, *J =* 9.6, 6.2 Hz, 1H), 2.90 (s, 1H), 2.66 (t, *J* = 5.2 Hz, 1H), 2.52 (d, *J =* 6.6 Hz, 1H), 2.48 (d, *J =* 7.2 Hz, 1H), 2.44 (t, *J =* 6.0 Hz, 1H), 2.41 - 2.33 (m, 3H), 2.14 - 2.02 (m, 3H), 1.89 (dd, *J =* 11.6, 5.8 Hz, 1H), 1.76 (s, 2H), 1.63 (t, *J* = 5.8 Hz, 2H), 1.40 (t, *J =* 7.0 Hz, 3H), 1.10 (t, *J* = 7.2 Hz, 3H), 0.63 (t, *J =* 6.8 Hz, 3H).

¹³C NMR (100 MHz, CDCl₃) δ 166.53, 165.65, 163.26, 162.62, 132.13, 132.13, 131.94, 123.44, 123.34, 113.99, 113.99, 113.55, 113.55, 85.20, 83.32, 83.10, 80.78, 80.32, 78.18, 77.69, 77.36, 63.71, 60.91, 59.17, 58.85, 58.25, 56.26, 56.02, 55.50, 54.18, 50.88, 49.06, 48.94, 48.20, 45.25, 42.34, 39.15, 37.69, 36.35, 35.01, 26.44, 15.47, 14.79, 13.64.

### Example 37 Preparation of compound 37

100 mg of bulleyaconitine A was added into a 5 mL round-bottom flask and dissolved with 2 mL of dry pyridine. Under an argon atmosphere, the reaction mixture was added with 2 equivalents of 4-ethoxybenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 97.9 mg of a light yellow solid as a target compound 37 (C₄₄H₅₇NO₁₂, 79.8% yield), which was structurally shown as follows:

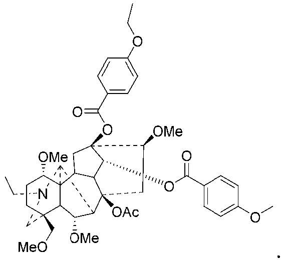

The target compound 37 was characterized as follows.

HRESIMS *m*/*z :* [(M + H)⁺, 792.38]

¹H NMR (400 MHz, Chloroform-d) δ 8.07 (d, *J =* 8.8 Hz, 2H), 7.93 (d, *J =* 8.8 Hz, 2H), 6.92 (d, *J* = 8.8 Hz, 2H), 6.83 (d, *J =* 8.8 Hz, 2H), 5.27 (d, *J* = 5.2 Hz, 1H), 4.15 (dd, *J* = 8.8, 5.6 Hz, 1H), 4.04 (q, *J =* 7.0 Hz, 2H), 3.99 (d, *J =* 6.6 Hz, 1H), 3.85 (s, 3H), 3.63 (d, *J* = 8.4 Hz, 1H), 3.52 (d, *J* = 10.8 Hz, 1H), 3.33 (s, 3H), 3.28 (s, 3H), 3.25 (s, 3H), 3.16 (s, 3H), 3.06 (d, *J* = 8.8 Hz, 1H), 3.03 (s, 3H), 3.01 (d, *J =* 5.8 Hz, 1H), 2.89 (s, 1H), 2.62 - 2.57 (m, 1H), 2.54 (t, *J =* 6.6 Hz, 1H), 2.51 (d, *J =* 5.2 Hz, 1H), 2.48 (d, *J =* 6.4 Hz, 1H), 2.44 (t, *J =* 3.4 Hz, 1H), 2.11 (d, *J =* 6.4 Hz, 2H), 1.92 (d, *J =* 5.8 Hz, 1H), 1.64 (s, 4H), 1.40 (t, *J =* 7.0 Hz, 3H), 1.35 (s, 3H), 1.11 (t, *J =* 7.2 Hz, 3H).

¹³C NMR (100 MHz, CDCl₃) δ 170.01, 166.42, 165.56, 163.61, 162.72, 132.13, 132.13, 131.96, 131.96, 123.18, 122.78, 114.02, 114.02, 113.91, 113.91, 85.73, 84.85, 83.42, 82.54, 80.56, 77.36, 77.33, 63.75, 61.56, 59.26, 58.44, 57.97, 56.17, 55.59, 54.01, 50.45, 49.34, 49.12, 44.08, 41.99, 39.86, 39.25, 35.83, 35.05, 29.84, 26.41, 21.89, 14.80, 13.62.

### Example 38 Preparation of compound 38

100 mg of compound 4 was added into a 5 mL round-bottom flask and dissolved with 2 mL of dry pyridine. Under an argon atmosphere, the reaction mixture was added with 2 equivalents of 4-nitrobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 109.0 mg of a light yellow solid as a target compound 38 (C₄₁H₅₁N₃O₁₃, 68.7% yield), which was structurally shown as follows:

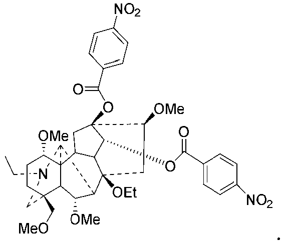

The target compound 38 was characterized as follows.

HRESIMS m/z : [(M + H)⁺, 794.34]

¹H NMR (400 MHz, Chloroform-d) δ 8.30 (td, J = 9.8, 1.0 Hz, 4H), 8.23 (d, *J* = 8.8 Hz, 2H), 8.15 (d, *J* = 9.0 Hz, 2H), 5.33 (d, *J =* 5.2 Hz, 1H), 4.17 (dd, *J* = 8.8, 5.8 Hz, 1H), 4.02 (d, *J* = 6.6 Hz, 1H), 3.67 (d, *J* = 8.2 Hz, 1H), 3.56 - 3.47 (m, 1H), 3.45 - 3.39 (m, 1H), 3.30 (s, 3H), 3.30 (s, 3H), 3.28 (s, 3H), 3.25 (s, 3H), 3.22 - 3.17 (m, 1H), 3.05 (dd, *J =* 10.4, 7.0 Hz, 2H), 2.91 (s, 1H), 2.71 (t, *J* = 5.4 Hz, 1H), 2.56 - 2.47 (m, 3H), 2.45 - 2.40 (m, 2H), 2.40 - 2.30 (m, 2H), 2.20 - 2.08 (m, 3H), 1.95 (dd, *J =* 11.2, 6.2 Hz, 1H), 1.70 - 1.59 (m, 3H), 1.11 (t, *J =* 7.2 Hz, 3H), 0.64 (t, *J =* 6.8 Hz, 3H).

¹³C NMR (100 MHz, CDCl₃) δ 164.65, 163.90, 150.62, 150.56, 136.32, 136.32, 136.15, 136.15, 131.17, 131.02, 123.63, 123.63, 123.56, 123.56, 85.29, 84.56, 83.15, 80.20, 80.13, 78.35, 78.20, 77.36, 61.36, 59.18, 59.01, 58.19, 56.21, 53.83, 50.89, 49.29, 49.14, 48.40, 45.33, 42.21, 39.22, 37.68, 36.07, 35.34, 26.47, 15.65, 13.69.

### Example 39 Preparation of compound 39

100 mg of compound 2 was added into a 5 mL round-bottom flask and dissolved with 2 mL of dry pyridine. Under an argon atmosphere, the reaction mixture was added with 2 equivalents of 4-nitrobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 73.9 mg of a light yellow solid as a target compound 39 (C₄₂H₅₄N₂O₁₂, 59.8% yield), which was structurally shown as follows:

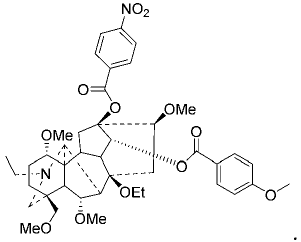

The target compound 39 was characterized as follows.

HRESIMS *m*/*z :* [(M + H)⁺, 779.36]

¹H NMR (400 MHz, Chloroform-d) δ 8.19 (dd, J = 9.0, 9.0 Hz, 4H), 8.07 (d, *J* = 8.8 Hz, 2H), 6.91 (d, *J =* 8.8 Hz, 2H), 5.29 (d, *J* = 5.2 Hz, 1H), 4.17 - 4.08 (m, 1H), 4.04 (d, *J =* 7.8 Hz, 1H), 3.85 (s, 3H), 3.65 (d, *J* = 8.2 Hz, 1H), 3.53 (q, *J* = 12.0 Hz, 1H), 3.44 - 3.37 (m, 1H), 3.30 (s, 3H), 3.29 (s, 3H), 3.28 (s, 3H), 3.25 (s, 3H), 3.22 (d, *J* = 8.4 Hz, 1H), 3.09 (d, *J =* 8.2 Hz, 1H), 3.04 (dd, *J* = 9.8, 6.2 Hz, 1H), 2.89 (s, 1H), 2.67 (t, *J =* 5.2 Hz, 1H), 2.55 (t, *J =* 6.0 Hz, 1H), 2.52 (s, 1H), 2.49 (d, *J* = 3.2 Hz, 1H), 2.46 (d, *J =* 4.8 Hz, 1H), 2.41 (s, 1H), 2.40 - 2.35 (m, 2H), 2.35 - 2.29 (m, 1H), 2.16 - 2.06 (m, 3H), 1.92 (dd, *J* = 11.0, 5.6 Hz, 1H), 1.72 (s, 1H), 1.64 (d, *J = 5.8* Hz, 1H), 1.11 (t, *J =* 7.2 Hz, 3H), 0.68 (t, *J =* 6.8 Hz, 3H).

¹³C NMR (100 MHz, CDCl₃) δ 166.37, 163.98, 163.40, 150.46, 136.53, 132.07, 132.07, 131.06, 131.06, 123.49, 123.49, 123.20, 113.63, 113.63, 85.26, 84.52, 83.26, 80.27, 78.12, 77.36, 77.26, 61.20, 59.17, 58.89, 58.04, 56.22, 56.12, 55.52, 53.98, 50.89, 49.14, 49.06, 48.32, 45.32, 42.24, 39.17, 37.60, 36.14, 35.15, 26.49, 15.51, 13.66.

### Example 40 Preparation of compound 40

100 mg of bulleyaconitine A was added into a 5 mL round-bottom flask and dissolved with 2 mL of dry pyridine. Under an argon atmosphere, the reaction mixture was added with 2 equivalents of 4-nitrobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 85.1 mg of a light yellow solid as a target compound 40 (C₄₂H₅₂N₂O₁₃, 69.3% yield), which was structurally shown as follows:

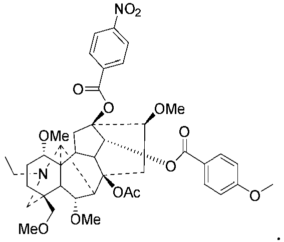

The target compound 40 was characterized as follows.

HRESIMS *m*/*z* : [(M + H)⁺, 793.34]

¹H NMR (400 MHz, Chloroform-*d*) δ 8.18 (dd, J = 28.0, 8.8 Hz, 4H), 8.05 (d, *J* = 8.8 Hz, 2H), 6.92 (d, *J* = 8.8 Hz, 2H), 5.31 (d, *J* = 5.2 Hz, 1H), 4.12 (dd, *J* = 8.8, 5.8 Hz, 1H), 4.00 (d, *J* = 6.6 Hz, 1H), 3.85 (s, 3H), 3.64 (d, *J* = 8.4 Hz, 1H), 3.62 - 3.56 (m, 1H), 3.30 (s, 3H), 3.28 (s, 3H), 3.26 (s, 3H), 3.17 (s, 3H), 3.15 (d, *J* = 8.4 Hz, 1H), 3.10 - 3.06 (m, 1H), 3.05 (s, 1H), 3.01 (s, 1H), 2.93 - 2.88 (m, 1H), 2.59 (dd, *J=* 12.0, 7.2 Hz, 1H), 2.54 (d, *J* = 5.8 Hz, 1H), 2.51 (s, 1H), 2.49 (s, 1H), 2.46 (t, *J* = 6.0 Hz, 1H), 2.37 - 2.27 (m, 1H), 2.14 (dd, *J* = 10.4, 6.2 Hz, 3H), 2.00 - 1.90 (m, 1H), 1.69 (s, 1H), 1.66 - 1.60 (m, 2H), 1.39 (s, 3H), 1.11 (t, *J* = 7.2 Hz, 3H).

¹³C NMR (100 MHz, CDCl₃) δ 169.96, 166.24, 163.90, 163.74, 150.52, 136.35, 132.05, 132.05, 131.05, 131.05, 123.52, 123.52, 122.51, 113.98, 113.98, 85.50, 84.88, 83.90, 83.37, 80.47, 80.06, 76.93, 61.81, 59.24, 58.23, 58.01, 56.11, 55.60, 53.82, 50.45, 49.39, 49.22, 49.13, 44.07, 41.86, 39.73, 39.24, 35.58, 35.12, 26.44, 21.86, 13.63.

### Example 41 Preparation of compound 41

100 mg of compound 4 was added into a 5 mL round-bottom flask and dissolved with 2 mL of dry pyridine. Under an argon atmosphere, the reaction mixture was added with 2 equivalents of acetyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 89.6 mg of a light yellow solid as a target compound 41 (C₃₁H₄₉NO₉, 77.3% yield), which was structurally shown as follows:

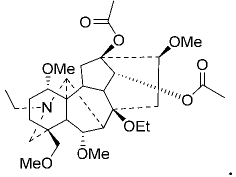

The target compound 41 was characterized as follows.

HRESIMS *m*/*z* : [(M + H)⁺, 580.34]

¹H NMR (400 MHz, Chloroform-*d*) δ 4.79 (d, *J* = 5.2 Hz, 1H), 3.97 (d, *J* = 5.2 Hz, 1H), 3.93 - 3.87 (m, 1H), 3.62 (d, *J* = 8.2 Hz, 1H), 3.50 - 3.45 (m, 1H), 3.37 (d, *J=* 6.0 Hz, 1H), 3.34 (dd, *J* = 6.8, 1.6 Hz, 1H), 3.31 (s, 3H), 3.28 (s, 6H), 3.21 (s, 3H), 3.06 (d, *J* = 8.2 Hz, 1H), 2.98 (dd, *J* = 9.6, 6.2 Hz, 1H), 2.76 (s, 1H), 2.53 (d, *J* = 5.4 Hz, 1H), 2.50 (d, *J* = 4.6 Hz, 1H), 2.48 - 2.45 (m, 2H), 2.44 (s, 1H), 2.37 (s, 1H), 2.27 - 2.21 (m, 1H), 2.18 (dd, *J* = 13.8, 7.2 Hz, 1H), 2.07 (s, 3H), 2.04 (s, 3H), 1.95 (ddd, *J* = 12.2, 7.2, 5.0 Hz, 1H), 1.89 (d, *J* = 6.0 Hz, 1H), 1.86 (d, *J=* 2.6 Hz, 1H), 1.83 (s, 2H), 1.61 (q, *J* = 6.2, 3.8 Hz, 2H), 1.11 (t, J = 7.0 Hz, 3H), 1.07 (t, J = 7.2 Hz, 3H).

¹³C NMR (100 MHz, CDCl₃) δ 171.46, 170.43, 85.07, 83.34, 82.27, 80.40, 80.22, 78.29, 77.69, 61.10, 59.14, 58.88, 58.10, 56.18, 56.14, 53.93, 50.80, 49.03, 48.89, 48.05, 44.56, 41.88, 39.08, 37.47, 36.36, 34.95, 26.43, 21.59, 21.50, 16.29, 13.56.

### Example 42 Preparation of compound 42

100 mg of compound 2 was added into a 5 mL round-bottom flask and dissolved with 2 mL of dry pyridine. Under an argon atmosphere, the reaction mixture was added with 2 equivalents of acetyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 54.6 mg of a light yellow solid as a target compound 42 (C₃₇H₅₃NO₁₀, 51.2% yield), which was structurally shown as follows:

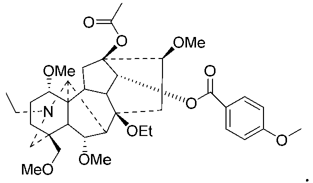

The target compound 42 was characterized as follows.

HRESIMS *m*/*z :* [(M + H)⁺, 672.36]

¹H NMR (400 MHz, Chloroform-d) δ 8.04 (d, *J* = 8.8 Hz, 2H), 6.90 (d, *J* = 8.8 Hz, 2H), 5.03 (d, *J* = 5.2 Hz, 1H), 4.03 - 3.96 (m, 2H), 3.85 (s, 3H), 3.61 (d, *J* = 8.2 Hz, 1H), 3.37 (s, 3H), 3.32 (s, 1H), 3.28 (s, 3H), 3.24 (d, *J* = 5.0 Hz, 6H), 3.17 - 3.11 (m, 1H), 3.08 (d, *J* = 8.4 Hz, 1H), 3.00 (dd, *J* = 9.8, 6.4 Hz, 1H), 2.82 (s, 1H), 2.61 (t, *J* = 6.2 Hz, 1H), 2.56 - 2.40 (m, 4H), 2.33 (d, *J* = 12.0 Hz, 2H), 2.31 - 2.24 (m, 2H), 2.05 (d, *J* = 6.4 Hz, 1H), 2.02 (s, 3H), 2.01 - 1.83 (m, 3H), 1.77 (s, 1H), 1.67 - 1.58 (m, 2H), 1.08 (t, *J* = 7.2 Hz, 3H), 0.55 (t, *J= 6.8* Hz, 3H).

¹³C NMR (100 MHz, CDCl₃) δ 170.59, 166.38, 163.23, 132.09, 132.09, 123.44, 113.51, 113.51, 85.28, 83.24, 82.82, 80.48, 80.30, 78.07, 77.53, 61.33, 59.16, 58.83, 58.12, 56.24, 55.94, 55.50, 53.92, 50.84, 49.09, 49.09, 48.32, 45.04, 42.17, 39.16, 37.48, 36.27, 35.11, 26.48, 21.61, 15.35, 13.65.

### Example 43 Preparation of compound 43

100 mg of bulleyaconitine A was added into a 5 mL round-bottom flask and dissolved with 2 mL of dry pyridine. Under an argon atmosphere, the reaction mixture was added with 2 equivalents of acetyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 91.7 mg of a light yellow solid as a target compound 43 (C₃₇H₅₁NO₁₁, 86.3% yield), which was structurally shown as follows:

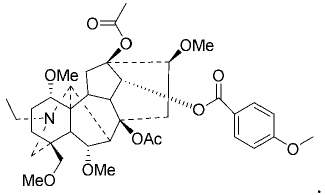

The target compound 43 was characterized as follows.

HRESIMS *m*/*z :* [(M + H)⁺, 686.34]

¹H NMR (400 MHz, Chloroform-d) δ 8.03 (d, *J* = 8.9 Hz, 2H), 6.91 (d, *J* = 8.9 Hz, 2H), 5.07 (d, *J* = 5.2 Hz, 1H), 3.99 (dd, *J* = 8.9, 5.8 Hz, 1H), 3.94 (d, *J* = 6.7 Hz, 1H), 3.85 (s, 3H), 3.61 (d, *J* = 8.5 Hz, 1H), 3.37 (s, 3H), 3.26 (s, 3H), 3.23 (s, 3H), 3.13 (s, 3H), 3.02 - 2.97 (m, 3H), 2.95 (d, *J* = 3.8 Hz, 1H), 2.86 - 2.80 (m, 1H), 2.59 - 2.53 (m, 1H), 2.46 (t, *J* = 2.7 Hz, 3H), 2.42 (d, *J* = 5.9 Hz, 1H), 2.28 (ddt, *J* = 12.3, 5.9, 2.8 Hz, 1H), 2.07 (dd, *J* = 6.4, 4.8 Hz, 2H), 2.03 (s, 3H), 2.00 - 1.91 (m, 2H), 1.82 (s, 2H), 1.63 - 1.57 (m, 2H), 1.36 (s, 1H), 1.24 (s, 1H), 1.08 (t, *J* = 7.1 Hz, 3H).

¹³C NMR (100 MHz, CDCl₃) δ 170.52, 169.99, 166.24, 163.57, 132.06, 132.06, 122.64, 113.86, 113.86, 85.59, 84.89, 83.29, 82.22, 80.46, 80.14, 77.09, 61.92, 59.21, 58.28, 57.94, 56.15, 55.56, 53.72, 50.36, 49.26, 49.04, 43.83, 41.76, 39.66, 39.19, 35.68, 35.05, 26.38, 22.78, 21.77, 21.55, 13.60.

### Example 44 Preparation of compound 44

100 mg of compound 3 was added into a 5 mL round-bottom flask and dissolved with 2 mL of dry pyridine. Under an argon atmosphere, the reaction mixture was added with 2 equivalents of acetyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 94.9 mg of a light yellow solid as a target compound 44 (C₃₀H₄₇NO₉, 79.9% yield), which was structurally shown as follows:

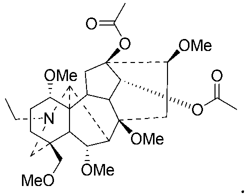

The target compound 44 was characterized as follows.

HRESIMS *m*/*z:* [(M + H)⁺, 566.32]

¹H NMR (400 MHz, Chloroform-d) δ 5.28 (s, 1H), 4.78 (d, *J* = 5.2 Hz, 1H), 3.92 (q, *J* = 7.8, 6.8 Hz, 2H), 3.61 (d, *J* = 8.2 Hz, 1H), 3.38 (dd, *J* = 14.8, 5.0 Hz, 1H), 3.32 (s, 3H), 3.28 (d, *J* = 2.8 Hz, 6H), 3.20 (s, 3H), 3.16 (s, 3H), 3.10 (d, *J* = 8.2 Hz, 1H), 2.96 (dd, *J* = 9.8, 6.4 Hz, 1H), 2.77 (s, 1H), 2.55 - 2.49 (m, 2H), 2.48 - 2.40 (m, 3H), 2.37 (s, 1H), 2.32 - 2.23 (m, 1H), 2.19 (dd, *J* = 8.0, 2.2 Hz, 2H), 2.04 (d, *J* = 5.8 Hz, 6H), 1.93 (dd, *J* = 7.2, 5.2 Hz, 1H), 1.90 - 1.83 (m, 2H), 1.60 (dd, *J* = 9.6, 4.4 Hz, 2H), 1.07 (t, *J* = 7.2 Hz, 3H).

¹³C NMR (100 MHz, CDCl₃) δ 171.54, 170.34, 84.98, 83.20, 82.32, 80.36, 80.31, 78.52, 77.63, 61.20, 59.15, 58.77, 58.09, 56.13, 53.92, 50.71, 49.04, 48.70, 48.42, 48.09, 44.31, 41.96, 39.08, 36.68, 36.25, 34.95, 26.46, 21.49, 21.47, 13.61.

### Example 45 Preparation of compound 45

100 mg of compound 1 was added into a 5 mL round-bottom flask and dissolved with 2 mL of dry pyridine. Under an argon atmosphere, the reaction mixture was added with 2 equivalents of acetyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 84.7 mg of a light yellow solid as a target compound 45 (C₃₆H₅₁NO₁₀, 79.3 % yield), which was structurally shown as follows:

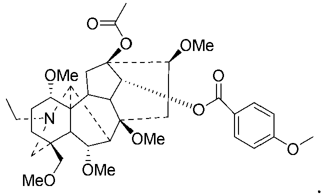

The target compound 45 was characterized as follows.

HRESIMS *m*/*z :* [(M + H)⁺, 658.35]

¹H NMR (400 MHz, Chloroform-d) δ 8.02 (d, *J* = 8.8 Hz, 2H), 6.91 (d, *J* = 8.8 Hz, 2H), 5.29 (s, 1H), 5.08 (d, *J* = 5.2 Hz, 1H), 4.04 - 3.98 (m, 1H), 3.96 (d, *J=* 6.0 Hz, 1H), 3.84 (s, 3H), 3.61 (d, *J* = 8.2 Hz, 1H), 3.37 (s, 3H), 3.33 (d, *J* = 4.8 Hz, 1H), 3.28 (s, 3H), 3.25 (s, 3H), 3.23 (s, 3H), 3.12 (d, *J* = 8.2 Hz, 1H), 3.00 (dd, *J* = 9.8, 6.2 Hz, 1H), 2.96 (s, 3H), 2.84 (s, 1H), 2.58 - 2.51 (m, 2H), 2.49 - 2.42 (m, 2H), 2.36 (s, 1H), 2.35 - 2.18 (m, 3H), 2.04 (d, *J* = 6.6 Hz, 1H), 2.01 (s, 3H), 1.96 (d, *J* = 14.6 Hz, 1H), 1.93 - 1.85 (m, 1H), 1.79 (s, 1H), 1.62 (dd, *J* = 8.8, 3.8 Hz, 2H), 1.09 (t, *J* = 7.2 Hz, 3H).

¹³C NMR (100 MHz, CDCl₃) δ 170.57, 166.46, 163.21, 132.10, 132.10, 123.32, 113.54, 113.54, 85.19, 83.11, 82.79, 80.36, 80.32, 78.33, 77.33, 61.39, 59.18, 58.74, 58.13, 56.20, 55.49, 53.94, 50.80, 49.12, 48.54, 48.54, 48.40, 45.01, 42.23, 39.15, 36.68, 36.19, 35.04, 26.45, 21.59, 13.67.

### Example 46 Preparation of compound 46

100 mg of compound 4 was added into a 5 mL round-bottom flask and dissolved with 2 mL of dry pyridine. Under an argon atmosphere, the reaction mixture was added with 2 equivalents of 4-(trifluoromethyl)benzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 106.4 mg of a light yellow solid as a target compound 46 (C₄₃H₅₁F₆NO₉, 63.4% yield), which was structurally shown as follows:

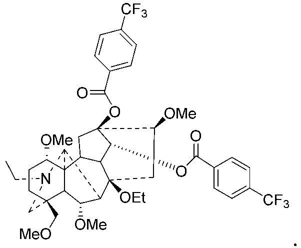

The target compound 46 was characterized as follows.

HRESIMS *m*/*z :* [(M + H)⁺, 840.34]

¹H NMR (400 MHz, Chloroform-d) δ 8.25 (d, *J* = 8.0 Hz, 2H), 8.10 (d, *J* = 8.0 Hz, 2H), 7.70 (d, *J* = 8.2 Hz, 2H), 7.64 (d, *J* = 8.2 Hz, 2H), 5.31 (d, *J* = 5.2 Hz, 1H), 4.16 (d, *J* = 14.8 Hz, 1H), 4.03 (d, *J* = 5.0 Hz, 1H), 3.66 (d, *J* = 8.2 Hz, 1H), 3.51 (q, *J* = 10.4, 8.6 Hz, 1H), 3.43 - 3.37 (m, 1H), 3.30 (s, 3H), 3.30 (s, 3H), 3.28 (s, 3H), 3.25 (s, 3H), 3.21 - 3.16 (m, 1H), 3.09 - 3.06 (m, 1H), 3.06 - 3.02 (m, 1H), 2.91 (s, 1H), 2.70 (t, *J* = 5.0 Hz, 1H), 2.59 - 2.52 (m, 2H), 2.52 - 2.46 (m, 2H), 2.42 (s, 1H), 2.39 - 2.34 (m, 2H), 2.34 - 2.29 (m, 1H), 2.13 (d, *J* = 9.2 Hz, 3H), 1.92 (dd, *J* = 11.6, 5.8 Hz, 1H), 1.65 (t, *J* = 4.4 Hz, 1H), 1.44 - 1.32 (m, 1H), 1.11 (t, *J* = 7.2 Hz, 3H), 0.62 (t, *J* = 6.8 Hz, 3H).

¹³C NMR (100 MHz, CDCl₃) δ 165.37, 164.61, 134.54, 134.43, 134.22, 134.18, 134.11, 134.01, 130.46, 130.29, 125.49, 125.45, 125.41, 125.37, 125.20, 125.18, 122.49, 122.47, 85.23, 84.11, 83.20, 80.26, 80.22, 78.19, 78.13, 61.21, 59.16, 58.95, 58.15, 56.21, 56.14, 53.96, 50.89, 49.21, 49.08, 45.26, 42.26, 39.19, 37.66, 36.11, 26.44, 15.48, 13.64.

### Example 47 Preparation of compound 47

100 mg of compound 2 was added into a 5 mL round-bottom flask and dissolved with 2 mL of dry pyridine. Under an argon atmosphere, the reaction mixture was added with 2 equivalents of 4-(trifluoromethyl)benzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 61.7 mg of a light yellow solid as a target compound 47 (C₄₃H₅₄F₃NO₁₀, 48.5% yield), which was structurally shown as follows:

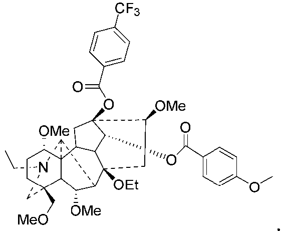

The target compound 47 was characterized as follows.

HRESIMS *m*/*z :* [(M + H)⁺, 802.37]

¹H NMR (400 MHz, Chloroform-d) δ 8.10 (d, *J=* 8.2 Hz, 2H), 8.07 (d, *J* = 8.8 Hz, 2H), 7.62 (d, *J* = 8.2 Hz, 2H), 6.91 (d, *J* = 9.0 Hz, 2H), 5.27 (d, *J* = 5.2 Hz, 1H), 4.17 - 4.09 (m, 1H), 4.04 (d, *J* = 5.2 Hz, 1H), 3.84 (s, 3H), 3.64 (d, *J* = 8.2 Hz, 1H), 3.56 - 3.45 (m, 1H), 3.42 - 3.37 (m, 1H), 3.30 (s, 6H), 3.28 (s, 3H), 3.25 (s, 3H), 3.24 - 3.18 (m, 1H), 3.09 (d, *J* = 8.2 Hz, 1H), 3.04 (dd, *J* = 9.6, 6.2 Hz, 1H), 2.89 (s, 1H), 2.67 (t, *J* = 5.2 Hz, 1H), 2.55 (dd, *J* = 7.0, 5.0 Hz, 1H), 2.51 (d, *J* = 12.8 Hz, 2H), 2.47 (d, *J* = 3.0 Hz, 1H), 2.41 (s, 1H), 2.37 (t, *J* = 7.0 Hz, 2H), 2.35 - 2.28 (m, 1H), 2.11 (d, *J* = 6.4 Hz, 3H), 1.91 (dd, *J* = 12.2, 6.0 Hz, 2H), 1.64 (d, *J* = 3.4 Hz, 1H), 1.11 (t, *J* = 7.2 Hz, 3H), 0.66 (t, *J* = 6.8 Hz, 3H).

¹³C NMR (100 MHz, CDCl₃) δ 166.41, 164.67, 163.35, 134.29, 133.98, 132.08, 132.08, 130.33, 130.33, 125.35, 125.31, 123.26, 122.50, 113.59, 113.59, 85.20, 84.07, 83.26, 80.39, 80.25, 78.14, 77.38, 61.10, 59.16, 58.87, 58.08, 56.22, 56.09, 55.50, 54.06, 50.88, 49.11, 49.02, 48.21, 45.25, 42.26, 39.15, 37.62, 36.16, 35.04, 26.44, 15.48, 13.62.

### Example 48 Preparation of compound 48

100 mg of bulleyaconitine A was added into a 5 mL round-bottom flask and dissolved with 2 mL of dry pyridine. Under an argon atmosphere, the reaction mixture was added with 2 equivalents of 4-(trifluoromethyl)benzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 96.7 mg of a light yellow solid as a target compound 48 (C₄₃H₅₂F₃NO₁₁, 76.5% yield), which was structurally shown as follows:

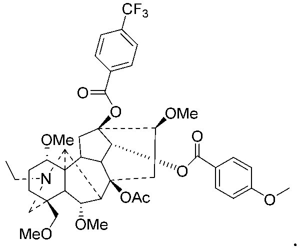

The target compound 48 was characterized as follows.

HRESIMS *m*/*z :* [(M + H)⁺, 816.34]

¹H NMR (400 MHz, Chloroform-d) δ 8.08 (d, *J* = 8.0 Hz, 2H), 8.04 (d, *J* = 9.0 Hz, 2H), 7.62 (d, *J* = 8.0 Hz, 2H), 6.90 (d, *J* = 9.0 Hz, 2H), 5.29 (d, *J* = 5.2 Hz, 1H), 4.13 (dd, *J* = 8.8, 5.8 Hz, 1H), 3.99 (d, *J* = 6.6 Hz, 1H), 3.83 (s, 3H), 3.63 (d, *J* = 8.4 Hz, 1H), 3.57 (dd, *J* = 13.8, 3.8 Hz, 1H), 3.30 (s, 3H), 3.27 (s, 3H), 3.25 (s, 3H), 3.16 (s, 3H), 3.14 (d, *J* = 8.4 Hz, 1H), 3.10 - 3.04 (m, 1H), 3.04 - 2.99 (m, 3H), 2.90 (t, *J* = 5.8 Hz, 1H), 2.59 (dd, *J* = 12.0, 7.2 Hz, 1H), 2.53 (s, 1H), 2.50 (d, *J* = 4.2 Hz, 1H), 2.48 (s, 1H), 2.45 (t, *J* = 6.0 Hz, 1H), 2.37 - 2.25 (m, 1H), 2.14 (d, *J* = 6.0 Hz, 2H), 2.11 (d, *J* = 4.0 Hz, 1H), 1.93 (dd, *J* = 10.6, 6.2 Hz, 1H), 1.89 (s, 1H), 1.63 (d, *J* = 4.2 Hz, 1H), 1.37 (s, 3H), 1.10 (t, *J* = 7.2 Hz, 3H).

¹³C NMR (100 MHz, CDCl₃) δ 169.96, 166.26, 164.56, 163.66, 134.38, 134.08, 134.06, 132.03, 132.03, 130.29, 130.29, 125.35, 125.31, 122.53, 113.91, 113.91, 85.54, 84.85, 83.45, 83.35, 80.45, 80.15, 77.02, 61.70, 59.20, 58.24, 57.96, 56.09, 55.54, 53.85, 50.41, 49.32, 49.17, 49.08, 44.01, 41.86, 39.74, 39.21, 35.59, 35.05, 26.39, 21.82, 13.57.

### Example 49 Preparation of compound 49

100 mg of compound 4 was added into a 5 mL round-bottom flask and dissolved with 2 mL of dry pyridine. Under an argon atmosphere, the reaction mixture was added with 2 equivalents of isonicotinoyl chloride hydrochloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 94.4 mg of a light yellow solid as a target compound 49 (C₃₉H₅₁N₃O₉, 66.9% yield), which was structurally shown as follows:

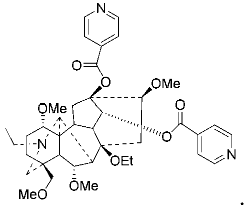

The target compound 49 was characterized as follows.

HRESIMS *m*/*z :* [(M + H)⁺, 706.36]

¹H NMR (400 MHz, Chloroform-*d*) δ 8.77 (dd, J = 4.4, 1.6 Hz, 2H), 8.71 (dd, J = 4.4, 1.6 Hz, 2H), 7.93 (dd, J = 4.4, 1.6 Hz, 2H), 7.78 (dd, J = 4.4, 1.6 Hz, 2H), 5.28 (d, *J* = 5.4 Hz, 1H), 4.14 (dd, *J* = 8.8, 6.2 Hz, 1H), 4.00 (d, *J* = 8.2 Hz, 1H), 3.65 (d, *J* = 8.4 Hz, 1H), 3.50 (q, *J* = 12.2 Hz, 1H), 3.39 (dd, *J* = 8.4, 6.8 Hz, 1H), 3.29 (s, 3H), 3.29 (s, 3H), 3.27 (s, 3H), 3.23 (s, 3H), 3.18 (dd, *J* = 8.4, 7.0 Hz, 1H), 3.03 (dd, *J* = 14.4, 7.4 Hz, 2H), 2.88 (s, 1H), 2.68 (t, *J* = 5.6 Hz, 1H), 2.53 (dd, *J* = 11.8, 7.2 Hz, 2H), 2.48 (d, *J* = 5.6 Hz, 1H), 2.43 (d, *J* = 19.8 Hz, 2H), 2.39 - 2.28 (m, 3H), 2.11 (t, *J* = 5.6 Hz, 3H), 1.92 (d, *J=* 6.4 Hz, 1H), 1.68 - 1.57 (m, 2H), 1.10 (t, *J* = 7.2 Hz, 3H), 0.62 (t, *J* = 6.8 Hz, 3H).

¹³C NMR (100 MHz, CDCl₃) δ 165.06, 164.30, 150.65, 150.65, 150.59, 150.59, 138.06, 137.88, 123.29, 123.29, 123.18, 123.18, 85.23, 84.36, 83.15, 80.19, 80.11, 78.26, 78.16, 61.24, 59.14, 58.95, 58.14, 56.18, 56.16, 53.85, 50.85, 49.21, 49.08, 48.35, 45.19, 42.17, 39.18, 37.62, 36.00, 35.27, 26.45, 15.52, 13.65.

### Example 50 Preparation of compound 50

100 mg of compound 2 was added into a 5 mL round-bottom flask and dissolved with 2 mL of dry pyridine. Under an argon atmosphere, the reaction mixture was added with 2 equivalents of isonicotinoyl chloride hydrochloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 79.1 mg of a light yellow solid as a target compound 50 (C₄₁H₅₄N₂O₁₀, 67.8% yield), which was structurally shown as follows:

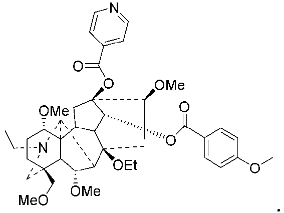

The target compound 50 was characterized as follows.

HRESIMS *m*/*z:* [(M + H)⁺, 735.37]

¹H NMR (400 MHz, Chloroform-*d*) δ 8.70 (dd, J = 4.4, 1.8 Hz, 2H), 8.07 (d, *J* = 8.8 Hz, 2H), 7.80 (dd, J = 4.6, 1.6 Hz, 2H), 6.91 (d, *J* = 8.8 Hz, 2H), 5.26 (d, *J* = 5.2 Hz, 1H), 4.14 - 4.08 (m, 1H), 4.04 (d, *J* = 5.8 Hz, 1H), 3.85 (s, 3H), 3.64 (d, *J* = 8.2 Hz, 1H), 3.56 - 3.47 (m, 1H), 3.44 - 3.36 (m, 1H), 3.30 (s, 3H), 3.29 (s, 3H), 3.28 (s, 3H), 3.24 (s, 3H), 3.23 - 3.17 (m, 1H), 3.09 (d, *J* = 8.2 Hz, 1H), 3.03 (dd, *J* = 9.8, 6.2 Hz, 1H), 2.88 (s, 1H), 2.67 (t, *J* = 5.6 Hz, 1H), 2.52 (t, *J* = 3.4 Hz, 1H), 2.48 (d, *J* = 3.2 Hz, 1H), 2.46 (s, 1H), 2.44 - 2.36 (m, 3H), 2.36 - 2.31 (m, 1H), 2.10 (d, *J* = 6.1 Hz, 3H), 1.92 (dd, *J* = 11.6, 6.0 Hz, 1H), 1.78 (s, 1H), 1.65 (dd, *J=* 12.4, 5.2 Hz, 2H), 1.11 (t, *J* = 7.2 Hz, 3H), 0.67 (t, *J* = 6.8 Hz, 3H).

¹³C NMR (100 MHz, CDCl₃) δ 166.36, 164.40, 163.37, 150.55, 150.55, 138.25, 132.08, 132.08, 123.28, 123.28, 123.25, 113.62, 113.62, 85.24, 84.40, 83.26, 80.28, 80.28, 78.12, 77.27, 61.15, 59.17, 58.88, 58.05, 56.22, 56.10, 55.52, 54.00, 50.88, 49.13, 49.04, 48.31, 45.29, 42.24, 39.17, 37.60, 36.10, 35.13, 26.48, 15.50, 13.66.

### Example 51 Preparation of compound 51

100 mg of bulleyaconitine A was added into a 5 mL round-bottom flask and dissolved with 2 mL of dry pyridine. Under an argon atmosphere, the reaction mixture was added with 2 equivalents of isonicotinoyl chloride hydrochloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 88.7 mg of a light yellow solid as a target compound 51 (C₄₁H₅₂N₂O₁₁, 76.5% yield), which was structurally shown as follows:

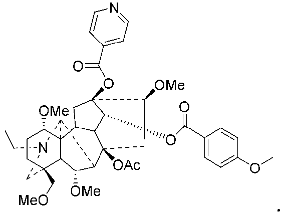

The target compound 51 was characterized as follows.

HRESIMS *m*/*z:* [(M + H)⁺, 749.35]

¹H NMR (400 MHz, Chloroform-*d*) δ 8.70 (dd, J = 4.4, 1.6 Hz, 2H), 8.04 (d, *J* = 8.8 Hz, 2H), 7.78 (dd, J = 4.4, 1.6 Hz, 2H), 6.91 (d, *J* = 8.8 Hz, 2H), 5.28 (d, *J* = 5.2 Hz, 1H), 4.11 (dd, *J* = 8.8, 5.8 Hz, 1H), 3.99 (d, *J* = 6.8 Hz, 1H), 3.84 (s, 3H), 3.63 (d, *J* = 8.4 Hz, 1H), 3.57 (dd, *J* = 14.2, 4.4 Hz, 1H), 3.29 (s, 3H), 3.27 (s, 3H), 3.25 (s, 3H), 3.16 (s, 3H), 3.14 (d, *J* = 8.4 Hz, 1H), 3.03 (t, *J* = 4.8 Hz, 2H), 3.00 (s, 1H), 2.92 - 2.87 (m, 1H), 2.58 (dd, *J* = 12.2, 7.2 Hz, 1H), 2.52 (d, *J* = 5.8 Hz, 1H), 2.51 - 2.46 (m, 2H), 2.46 - 2.42 (m, 1H), 2.31 (dt, *J* = 15.4, 5.6 Hz, 1H), 2.19 - 2.05 (m, 3H), 1.93 (dd, *J* = 19.4, 7.4 Hz, 2H), 1.67 - 1.58 (m, 2H), 1.37 (s, 3H), 1.10 *(t, J=* 7.2 Hz, 3H).

¹³C NMR (100 MHz, CDCl₃) δ 169.93, 166.21, 164.29, 163.69, 150.55, 150.55, 138.06, 132.04, 132.04, 123.21, 123.21, 122.52, 113.94, 113.94, 85.49, 84.84, 83.77, 83.35, 80.46, 80.04, 76.91, 61.74, 59.21, 58.22, 57.97, 56.08, 55.57, 53.82, 50.41, 49.37, 49.18, 49.10, 44.02, 41.84, 39.71, 39.22, 35.51, 35.09, 26.41, 21.83, 13.60.

### Example 52 Preparation of compound 52

100 mg of compound 4 was added into a 5 mL round-bottom flask and dissolved with 2 mL of dry pyridine. Under an argon atmosphere, the reaction mixture was added with 2 equivalents of benzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 77.7 mg of a light yellow solid as a target compound 52 (C₄₁H₅₃NO₉, 55.2% yield), which was structurally shown as follows:

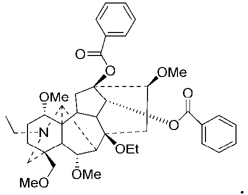

The target compound 52 was characterized as follows.

HRESIMS *m*/*z :* [(M + H)⁺, 704.37]

¹H NMR (400 MHz, Chloroform-*d*) δ 8.14 (dd, J = 8.4, 1.4 Hz, 2H), 8.00 (dd, J = 8.4, 1.4 Hz, 2H), 7.56 - 7.51 (m, 1H), 7.51 - 7.46 (m, 1H), 7.42 (t, *J* = 7.6 Hz, 2H), 7.38 (d, *J=* 8.0 Hz, 2H), 5.29 (d, *J= 5.2* Hz, 1H), 4.21 - 4.14 (m, 1H), 4.04 (d, *J* = 6.4 Hz, 1H), 3.64 (d, *J* = 8.2 Hz, 1H), 3.56 - 3.45 (m, 1H), 3.40 - 3.35 (m, 1H), 3.33 (s, 3H), 3.30 (s, 3H), 3.28 (s, 3H), 3.25 (s, 3H), 3.22 - 3.16 (m, 1H), 3.09 (d, *J* = 8.2 Hz, 1H), 3.04 (dd, *J* = 9.6, 6.2 Hz, 1H), 2.91 (s, 1H), 2.70 (t, *J* = 5.6 Hz, 1H), 2.53 (s, 1H), 2.49 (d, *J* = 6.4 Hz, 1H), 2.45 (dd, *J* = 7.2, 2.8 Hz, 1H), 2.40 (s, 2H), 2.37 (d, *J* = 10.6 Hz, 1H), 2.33 (dd, *J* = 8.8, 5.0 Hz, 1H), 2.10 (d, *J* = 6.2 Hz, 3H), 1.90 (dt, *J=* 11.0, 5.4 Hz, 1H), 1.82 (s, 1H), 1.64 (q, *J* = 3.4 Hz, 1H), 1.33 (d, *J* = 2.8 Hz, 1H), 1.11 (t, *J* = 7.2 Hz, 3H), 0.59 (t, *J* = 6.8 Hz, 3H).

¹³C NMR (100 MHz, CDCl₃) δ 166.72, 165.86, 132.78, 132.69, 131.03, 130.82, 130.11, 130.11, 129.93, 129.93, 128.30, 128.30, 128.30, 128.30, 85.20, 83.43, 83.29, 80.61, 80.29, 78.19, 77.86, 60.97, 59.16, 58.86, 58.19, 56.23, 56.03, 54.11, 50.88, 49.08, 48.96, 48.24, 45.20, 42.31, 39.15, 37.64, 36.27, 35.06, 26.44, 15.33, 13.64.

### Example 53 Preparation of compound 53

100 mg of compound 2 was added into a 5 mL round-bottom flask and dissolved with 2 mL of dry pyridine. Under an argon atmosphere, the reaction mixture was added with 2 equivalents of benzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 92.7 mg of a light yellow solid as a target compound 53 (C₄₂H₅₅NO₁₀, 79.6% yield), which was structurally shown as follows:

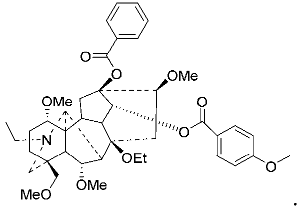

The target compound 53 was characterized as follows.

HRESIMS *m*/*z* : [(M + H)⁺, 734.39]

¹H NMR (400 MHz, Chloroform-d) δ 8.08 (d, *J* = 8.9 Hz, 2H), 8.00 (dd, *J* = 8.3, 1.4 Hz, 2H), 7.52 - 7.46 (m, 1H), 7.37 (dd, *J* = 8.4, 7.1 Hz, 2H), 6.91 (d, *J* = 8.9 Hz, 2H), 5.25 (d, *J* = 5.2 Hz, 1H), 4.16 (dd, *J* = 8.5, 6.6 Hz, 1H), 4.04 (d, *J* = 6.3 Hz, 1H), 3.85 (s, 3H), 3.64 (d, *J* = 8.2 Hz, 1H), 3.49 (q, *J* = 12.2 Hz, 1H), 3.38 (dd, *J* = 8.2, 6.8 Hz, 1H), 3.33 (s, 3H), 3.30 (s, 3H), 3.27 (s, 3H), 3.24 (s, 3H), 3.23 - 3.17 (m, 1H), 3.09 (d, *J* = 8.2 Hz, 1H), 3.03 (dd, *J* = 9.7, 6.2 Hz, 1H), 2.90 (s, 1H), 2.67 (dt, *J* = 5.8, 3.1 Hz, 1H), 2.57 - 2.51 (m, 2H), 2.47 (q, *J=* 5.4, 4.1 Hz, 2H), 2.41 - 2.34 (m, 3H), 2.09 (d, *J= 7.2* Hz, 2H), 1.90 (dd, *J* = 11.9, 5.5 Hz, 1H), 1.77 (s, 2H), 1.66 - 1.62 (m, 1H), 1.43 - 1.32 (m, 1H), 1.11 (t, *J* = 7.1 Hz, 3H), 0.63 (t, *J* = 6.9 Hz, 3H).

¹³C NMR (100 MHz, CDCl₃) δ 166.50, 165.87, 163.26, 132.69, 132.11, 132.11, 131.02, 129.94, 129.94, 128.29, 128.29, 123.36, 113.55, 113.55, 85.20, 83.40, 83.28, 80.64, 80.28, 78.16, 77.59, 60.96, 59.17, 58.86, 58.22, 56.28, 56.03, 55.51, 54.13, 50.86, 49.03, 48.96, 48.15, 45.21, 42.28, 39.13, 37.69, 36.26, 35.02, 26.43, 15.47, 13.64.

### Example 54 Preparation of compound 54

100 mg of bulleyaconitine A was added into a 5 mL round-bottom flask and dissolved with 2 mL of dry pyridine. Under an argon atmosphere, the reaction mixture was added with 2 equivalents of benzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 52.0 mg of a light yellow solid as a target compound 54 (C₄₂H₅₃NO₁₁, 44.9% yield), which was structurally shown as follows:

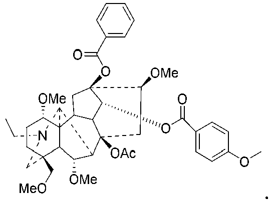

The target compound 54 was characterized as follows.

HRESIMS *m*/*z :* [(M + H)⁺, 748.36]

¹H NMR (400 MHz, Chloroform-d) δ 8.06 (d, *J* = 8.8 Hz, 2H), 7.98 (dd, J = 8.4, 1.2 Hz, 2H), 7.53 - 7.46 (m, 1H), 7.36 (dd, *J* = 8.4, 7.2 Hz, 2H), 6.91 (d, *J* = 9.0 Hz, 2H), 5.28 (d, *J* = 5.2 Hz, 1H), 4.16 (dd, *J* = 9.0, 5.8 Hz, 1H), 3.99 (d, *J* = 6.6 Hz, 1H), 3.84 (s, 3H), 3.63 (d, *J* = 8.4 Hz, 1H), 3.58 - 3.51 (m, 1H), 3.32 (s, 3H), 3.27 (s, 3H), 3.25 (s, 3H), 3.16 (s, 3H), 3.14 (s, 1H), 3.03 (dd, *J* = 4.2, 2.6 Hz, 3H), 2.90 (dt, *J* = 7.6, 3.3 Hz, 1H), 2.58 (dd, *J* = 12.2, 7.2 Hz, 1H), 2.55 - 2.51 (m, 1H), 2.51 - 2.46 (m, 2H), 2.46 - 2.41 (m, 1H), 2.33 - 2.24 (m, 1H), 2.18 - 2.06 (m, 3H), 1.97 - 1.88 (m, 1H), 1.86 (s, 1H), 1.61 (dt, *J=* 13.0, 4.2 Hz, 2H), 1.35 (s, 3H), 1.27 (s, 1H), 1.10 (t, *J* = 7.2 Hz, 3H).

¹³C NMR (100 MHz, CDCl₃) δ 169.98, 166.34, 165.75, 163.58, 132.78, 132.07, 132.07, 130.82, 129.89, 129.89, 128.30, 128.30, 122.63, 113.87, 113.87, 85.63, 84.82, 83.34, 82.80, 80.47, 80.37, 77.19, 61.58, 59.21, 58.37, 57.95, 56.14, 55.55, 53.91, 50.39, 49.25, 49.11, 49.02, 43.99, 41.88, 39.79, 39.18, 35.69, 35.01, 26.37, 21.83, 13.59.

### Example 55 Preparation of compound 55

100 mg of compound 3 was added into a 5 mL round-bottom flask and dissolved with 2 mL of dry pyridine. Under an argon atmosphere, the reaction mixture was added with 2 equivalents of benzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 91.5 mg of a light yellow solid as a target compound 55 (C₄₀H₅₁NO₉, 63.2% yield), which was structurally shown as follows:

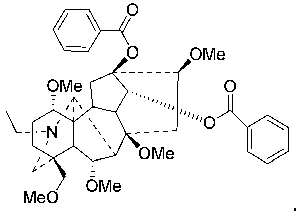

The target compound 55 was characterized as follows.

HRESIMS *m*/*z :* [(M + H)⁺, 690.35]

¹H NMR (400 MHz, Chloroform-*d*) δ 8.13 (dd, J = 8.2, 1.4 Hz, 2H), 8.00 (dd, *J* = 8.4, 1.4 Hz, 2H), 7.56 - 7.51 (m, 1H), 7.51 - 7.46 (m, 1H), 7.43 (t, *J* = 7.6 Hz, 2H), 7.36 (t, *J* = 7.6 Hz, 2H), 5.35 (d, *J* = 5.2 Hz, 1H), 4.18 (dd, *J* = 9.2, 5.8 Hz, 1H), 4.01 (dd, 1H), 3.65 (d, *J* = 8.2 Hz, 1H), 3.56 - 3.46 (m, 1H), 3.33 (s, 3H), 3.30 (s, 3H), 3.29 (s, 3H), 3.25 (s, 3H), 3.14 (d, *J* = 8.2 Hz, 1H), 3.03 (s, 4H), 2.94 (s, 1H), 2.64 - 2.59 (m, 1H), 2.59 - 2.51 (m, 2H), 2.52 - 2.46 (m, 2H), 2.46 - 2.40 (m, 2H), 2.34 (m, 2H), 2.13 - 2.06 (m, 3H), 1.91 (m, 1H), 1.70 (t, *J* = 3.6 Hz, 1H), 1.65 (dd, *J* = 9.2, 4.4 Hz, 2H), 1.12 (t, *J* = 7.2 Hz, 3H).

¹³C NMR (100 MHz, CDCl₃) δ 166.79, 165.87, 132.74, 132.70, 131.01, 130.81, 130.14, 130.14, 129.95, 129.95, 128.33, 128.33, 128.30, 128.30, 85.15, 83.48, 83.21, 80.45, 80.40, 78.47, 77.65, 61.05, 59.21, 58.80, 58.21, 56.23, 54.16, 50.87, 49.02, 48.64, 48.51, 48.43, 45.26, 42.41, 39.19, 36.84, 36.25, 35.04, 26.45, 13.68.

### Example 56 Preparation of compound 56

100 mg of compound 4 was added into a 5 mL round-bottom flask and dissolved with 2 mL of dry pyridine. Under an argon atmosphere, the reaction mixture was added with 2 equivalents of 4-methylbenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 104.3 mg of a light yellow solid as a target compound 56 (C₄₃H₅₇NO₉, 71.3% yield), which was structurally shown as follows:

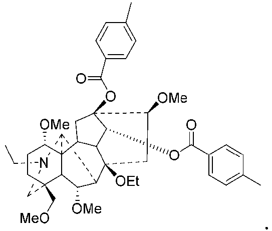

The target compound 56 was characterized as follows.

HRESIMS *m*/*z* : [(M + H)⁺, 732.40]

¹H NMR (400 MHz, Chloroform-d) δ 7.99 (d, *J* = 2.4 Hz, 2H), 7.87 (d, *J* = 8.2 Hz, 2H), 7.22 (d, *J* = 8.0 Hz, 2H), 7.17 (d, J = 7.8 Hz, 2H), 5.90 (s, 2H), 5.26 (d, *J* = 5.2 Hz, 1H), 4.13 - 4.05 (m, 2H), 3.59 (d, *J= 8.2* Hz, 1H), 3.39 - 3.33 (m, 1H), 3.31 (s, 3H), 3.30 (s, 3H), 3.27 (s, 3H), 3.27 (s, 3H), 3.23 - 3.18 (m, 1H), 3.18 - 3.13 (m, 1H), 3.04 (s, 1H), 3.02 (d, *J* = 8.0 Hz, 1H), 2.93 (d, *J* = 11.2 Hz, 1H), 2.77 (d, *J* = 11.2 Hz, 1H), 2.75 - 2.70 (m, 1H), 2.68 (t, *J* = 6.2 Hz, 1H), 2.42 (d, *J* = 1.8 Hz, 1H), 2.39 (s, 3H), 2.37 (s, 3H), 2.35 (s, 3H), 2.24 (d, *J* = 6.4 Hz, 1H), 2.18 (d, *J* = 4.6 Hz, 2H), 1.91 - 1.82 (m, 1H), 1.76 - 1.63 (m, 1H), 1.42 - 1.32 (m, 1H), 1.23 (t, J = 5.8 Hz, 3H), 0.64 (t, *J* = 6.8 Hz, 3H).

¹³C NMR (100 MHz, CDCl₃) δ 166.62, 166.05, 143.52, 143.45, 142.45, 130.09, 130.06, 129.94, 129.09, 129.04, 128.82, 127.96, 127.83, 83.38, 82.85, 82.62, 80.59, 79.33, 78.21, 78.21, 60.76, 59.13, 58.87, 58.31, 56.30, 56.11, 55.11, 50.96, 49.08, 49.04, 45.40, 44.48, 41.87, 38.74, 37.74, 36.09, 31.98, 24.85, 21.79, 21.72, 21.68, 15.33, 12.41.

### Example 57 Preparation of compound 57

100 mg of compound 2 was added into a 5 mL round-bottom flask and dissolved with 2 mL of dry pyridine. Under an argon atmosphere, the reaction mixture was added with 2 equivalents of 4-methylbenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 85.9 mg of a light yellow solid as a target compound 57 (C₄₃H₅₇NO₁₀, 72.4% yield), which was structurally shown as follows:

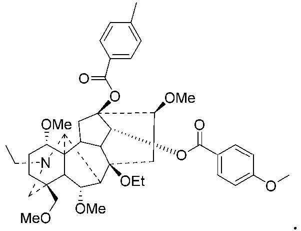

The target compound 57 was characterized as follows.

HRESIMS *m*/*z* : [(M + H)⁺, 748.39]

¹H NMR (400 MHz, Chloroform-*d*) δ 8.08 (d, *J* = 8.8 Hz, 2H), 7.88 (d, *J* = 8.2 Hz, 2H), 7.16 (d, *J* = 8.2 Hz, 2H), 6.91 (d, *J* = 8.8 Hz, 2H), 5.24 (d, *J* = 5.2 Hz, 1H), 4.15 (dd, *J* = 8.4, 6.4 Hz, 1H), 4.04 (dd, *J* = 6.4, 1.6 Hz, 1H), 3.84 (s, 3H), 3.64 (d, *J =* 8.2 Hz, 1H), 3.49 - 3.45 (m, 1H), 3.37 (dd, *J* = 8.2, 6.8 Hz, 1H), 3.32 (s, 3H), 3.30 (s, 3H), 3.27 (s, 3H), 3.24 (s, 3H), 3.22 - 3.17 (m, 1H), 3.09 (d, *J* = 8.2 Hz, 1H), 3.03 (dd, *J* = 9.6, 6.2 Hz, 1H), 2.90 (s, 1H), 2.67 (td, *J* = 5.8, 1.4 Hz, 1H), 2.57 - 2.50 (m, 2H), 2.49 - 2.42 (m, 2H), 2.41 - 2.37 (m, 2H), 2.36 (d, *J* = 3.5 Hz, 3H), 2.11 - 2.07 (m, 2H), 1.89 (q, *J* = 6.2 Hz, 1H), 1.68 - 1.60 (m, 2H), 1.37 (s, 1H), 1.28 (s, 2H), 1.10 (t, *J* = 7.2 Hz, 3H), 0.62 (t, *J* = 6.8 Hz, 3H).

¹³C NMR (100 MHz, CDCl₃) δ 166.50, 165.91, 163.22, 143.22, 132.09, 132.09, 129.95, 129.95, 128.97, 128.97, 128.30, 123.38, 113.52, 113.52, 85.16, 83.27, 83.21, 80.67, 80.28, 78.14, 77.63, 60.90, 59.15, 58.83, 58.20, 56.25, 55.99, 55.48, 54.14, 50.84, 48.92, 48.12, 45.19, 42.30, 39.11, 37.67, 36.26, 35.00, 29.81, 26.41, 21.73, 15.45, 13.63.

### Example 58 Preparation of compound 58

100 mg of bulleyaconitine A was added into a 5 mL round-bottom flask and dissolved with 2 mL of dry pyridine. Under an argon atmosphere, the reaction mixture was added with 2 equivalents of 4-methylbenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 86.7 mg of a light yellow solid as a target compound 58 (C₄₃H₅₅NO₁₁, 73.5% yield), which was structurally shown as follows:

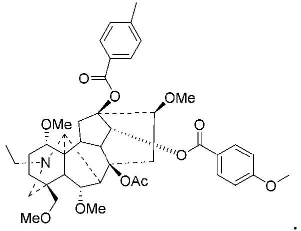

The target compound 58 was characterized as follows.

HRESIMS *m*/*z* : [(M + H)⁺, 762.37]

¹H NMR (400 MHz, Chloroform-*d*) δ 8.07 (d, *J* = 8.8 Hz, 2H), 7.87 (d, *J* = 8.2 Hz, 2H), 7.16 (d, *J* = 8.0 Hz, 2H), 6.91 (d, *J* = 8.8 Hz, 2H), 5.27 (d, *J* = 5.2 Hz, 1H), 4.14 (dt, *J* = 10.2, 5.0 Hz, 1H), 3.99 (d, *J* = 6.6 Hz, 1H), 3.84 (s, 3H), 3.63 (d, *J* = 8.4 Hz, 1H), 3.55 - 3.50 (m, 1H), 3.32 (s, 3H), 3.28 (s, 3H), 3.24 (s, 3H), 3.16 (s, 3H), 3.02 (d, *J* = 6.4 Hz, 3H), 2.91 - 2.86 (m, 1H), 2.62 - 2.55 (m, 1H), 2.51 (dd, *J* = 10.4, 5.2 Hz, 2H), 2.46 (dd, *J* = 11.2, 4.4 Hz, 2H), 2.36 (s, 3H), 2.33 - 2.24 (m, 1H), 2.17 - 2.05 (m, 3H), 1.92 (d, *J* = 3.8 Hz, 1H), 1.62 (dt, *J* = 11.0, 3.8 Hz, 2H), 1.42 - 1.36 (m, 1H), 1.34 (s, 3H), 1.28 (s, 1H), 1.10 (t, *J* = 7.2 Hz, 3H).

¹³C NMR (100 MHz, CDCl₃) δ 169.98, 166.38, 165.82, 163.57, 143.37, 132.09, 132.09, 129.94, 129.94, 129.00, 129.00, 128.13, 122.69, 113.87, 113.87, 85.67, 84.81, 83.37, 82.64, 80.50, 80.44, 77.25, 61.56, 59.22, 58.39, 57.95, 56.15, 55.56, 53.95, 50.40, 49.26, 49.11, 44.00, 41.93, 39.81, 39.20, 35.72, 35.01, 29.82, 26.38, 21.85, 21.75, 13.60.

### Example 59 Preparation of compound 59

100 mg of compound 4 was added into a 5 mL round-bottom flask and dissolved with 2 mL of dry pyridine. Under an argon atmosphere, the reaction mixture was added with 2 equivalents of 4-fluorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 110.3 mg of a light yellow solid as a target compound 59 (C₄₁H₅₁F₂NO₉, 74.6% yield), which was structurally shown as follows:

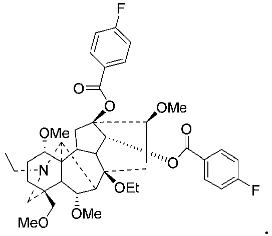

The target compound 59 was characterized as follows.

HRESIMS *m*/*z* : [(M + H)⁺, 740.35]

¹H NMR (400 MHz, Chloroform-d) δ 8.14 (dd, *J* = 8.8, 5.4 Hz, 2H), 8.00 (dd, *J* = 8.8, 5.4 Hz, 2H), 7.06 (dt, *J =* 24.0, 8.6 Hz, 4H), 5.25 (d, *J* = 5.4 Hz, 1H), 4.15 (t, *J* = 7.4 Hz, 1H), 4.02 (d, *J* = 6.0 Hz, 1H), 3.64 (d, *J* = 8.2 Hz, 1H), 3.47 (d, *J* = 9.0 Hz, 1H), 3.41 - 3.36 (m, 1H), 3.31 (s, 3H), 3.29 (s, 3H), 3.27 (s, 3H), 3.24 (s, 3H), 3.19 (dd, *J* = 8.4, 7.0 Hz, 1H), 3.08 (d, *J* = 8.4 Hz, 1H), 3.03 (dd, *J* = 9.8, 6.4 Hz, 1H), 2.90 (s, 1H), 2.54 (dt, *J* = 11.8, 4.2 Hz, 2H), 2.50 - 2.43 (m, 2H), 2.40 (s, 1H), 2.35 (d, *J* = 6.8 Hz, 2H), 2.10 (t, *J* = 6.2 Hz, 3H), 1.91 (dq, *J* = 10.8, 4.6 Hz, 1H), 1.82 (s, 1H), 1.64 (dt, *J* = 10.2, 5.2 Hz, 2H), 1.10 (t, *J* = 7.2 Hz, 3H), 0.63 (t, *J* = 6.8 Hz, 3H).

¹³C NMR (100 MHz, CDCl₃) δ 167.00, 165.71, 164.86, 164.54, 132.65, 132.56, 132.50, 132.40, 127.19, 127.10, 115.57, 115.51, 115.36, 115.29, 85.24, 83.59, 83.24, 80.52, 80.26, 78.16, 77.89, 61.08, 59.15, 58.89, 58.20, 56.22, 56.07, 54.02, 50.86, 49.13, 49.01, 48.27, 45.25, 42.26, 39.16, 37.67, 36.24, 35.14, 26.44, 15.44, 13.64.

### Example 60 Preparation of compound 60

100 mg of compound 2 was added into a 5 mL round-bottom flask and dissolved with 2 mL of dry pyridine. Under an argon atmosphere, the reaction mixture was added with 2 equivalents of 4-fluorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 79.7 mg of a light yellow solid as a target compound 60 (C₄₂H₅₄FNO₁₀, 66.8% yield), which was structurally shown as follows:

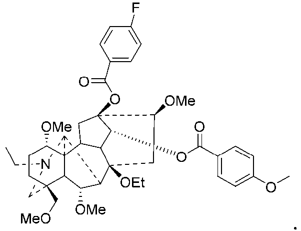

The target compound 60 was characterized as follows.

HRESIMS *m*/*z* : [(M + H)⁺, 752.37]

¹H NMR (400 MHz, Chloroform-d) δ 8.07 (d, *J* = 8.8 Hz, 2H), 8.00 (dd, *J* = 8.8, 5.4 Hz, 2H), 7.02 (t, *J* = 8.8 Hz, 2H), 6.90 (d, *J* = 8.8 Hz, 2H), 5.25 (d, *J* = 5.2 Hz, 1H), 4.13 (dd, *J* = 8.6, 6.6 Hz, 1H), 4.03 (dd, *J* = 6.4, 1.6 Hz, 1H), 3.84 (s, 3H), 3.64 (d, *J* = 8.2 Hz, 1H), 3.53 - 3.42 (m, 1H), 3.38 (dd, *J* = 8.0, 6.6 Hz, 1H), 3.31 (s, 3H), 3.29 (s, 3H), 3.27 (s, 3H), 3.24 (s, 3H), 3.08 (d, *J* = 8.2 Hz, 1H), 3.03 (dd, *J* = 9.6, 6.2 Hz, 1H), 2.89 (s, 1H), 2.68 - 2.63 (m, 1H), 2.56 - 2.50 (m, 2H), 2.50 - 2.43 (m, 2H), 2.40 - 2.32 (m, 3H), 2.11 - 2.06 (m, 3H), 1.97 - 1.84 (m, 2H), 1.68 - 1.60 (m, 2H), 1.36 (s, 1H), 1.10 (t, *J* = 7.2 Hz, 3H), 0.64 (t, *J* = 6.8 Hz, 3H).

¹³C NMR (100 MHz, CDCl₃) δ 166.43, 164.87, 164.42, 163.27, 132.49, 132.40, 132.07, 132.07, 127.23, 123.26, 115.45, 115.23, 113.54, 113.54, 85.18, 83.53, 83.23, 80.53, 80.24, 78.11, 77.48, 60.99, 59.13, 58.83, 58.13, 56.23, 56.03, 55.47, 54.05, 50.83, 49.01, 48.96, 48.13, 45.22, 42.23, 39.10, 37.64, 36.24, 35.04, 26.41, 15.45, 13.62.

### Example 61 Preparation of compound 61

100 mg of bulleyaconitine A was added into a 5 mL round-bottom flask and dissolved with 2 mL of dry pyridine. Under an argon atmosphere, the reaction mixture was added with 2 equivalents of 4-fluorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 80.8 mg of a light yellow solid as a target compound 61 (C₄₂H₅₂FNO₁₁, 68.1% yield), which was structurally shown as follows:

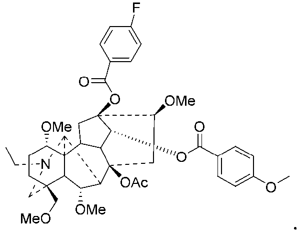

The target compound 61 was characterized as follows.

HRESIMS *m*/*z* : [(M + H)⁺, 766.35]

¹H NMR (400 MHz, Chloroform-d) δ 8.05 (d, *J* = 8.8 Hz, 2H), 7.98 (dd, *J* = 8.8, 5.4 Hz, 2H), 7.02 (t, *J* = 8.8 Hz, 2H), 6.90 (d, *J* = 8.8 Hz, 2H), 5.26 (d, *J* = 5.2 Hz, 1H), 4.13 (dd, *J* = 8.8, 5.8 Hz, 1H), 3.98 (d, *J* = 6.6 Hz, 1H), 3.83 (s, 3H), 3.62 (d, *J* = 8.4 Hz, 1H), 3.53 (dd, *J* = 13.4, 3.6 Hz, 1H), 3.30 (s, 3H), 3.26 (s, 3H), 3.24 (s, 3H), 3.15 (s, 3H), 3.13 (d, *J* = 8.4 Hz, 1H), 3.02 (q, *J* = 6.8, 5.2 Hz, 3H), 2.88 (dd, *J* = 7.2, 5.2 Hz, 1H), 2.61 - 2.54 (m, 1H), 2.54 - 2.48 (m, 2H), 2.45 (dt, *J* = 12.0, 3.8 Hz, 2H), 2.33 - 2.25 (m, 1H), 2.11 (d, *J* = 1.8 Hz, 1H), 2.10 - 2.05 (m, 1H), 2.02 - 1.96 (m, 1H), 1.96 - 1.89 (m, 1H), 1.65 - 1.57 (m, 2H), 1.35 (s, 3H), 1.26 (s, 1H), 1.09 (t, *J* = 7.2 Hz, 3H).

¹³C NMR (100 MHz, CDCl₃) δ 169.94, 166.28, 164.75, 163.60, 132.47, 132.38, 132.03, 132.03, 127.02, 122.55, 115.48, 115.26, 113.87, 113.87, 85.57, 84.80, 83.32, 82.93, 80.43, 80.29, 77.11, 61.61, 59.17, 58.29, 57.93, 56.10, 55.52, 53.85, 50.37, 49.23, 49.11, 49.01, 43.98, 41.84, 39.75, 39.17, 35.67, 35.01, 26.35, 22.75, 21.80, 13.56.

### Example 62 Preparation of compound 62

100 mg of compound 4 was added into a 5 mL round-bottom flask and dissolved with 2 mL of dry pyridine. Under an argon atmosphere, the reaction mixture was added with 2 equivalents of 3-methylbenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 97.0 mg of a light yellow solid as a target compound 62 (C₄₃H₅₇NO₉, 66.3% yield), which was structurally shown as follows:

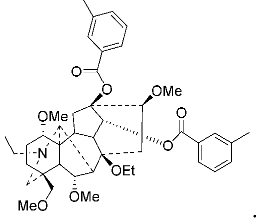

The target compound 62 was characterized as follows.

HRESIMS *m*/*z* : [(M + H)⁺, 732.40]

¹H NMR (400 MHz, Chloroform-*d*) δ 7.97 - 7.91 (m, 2H), 7.80 (d, *J* = 8.2 Hz, 2H), 7.36 - 7.22 (m, 4H), 5.29 (d, *J* = 5.2 Hz, 1H), 4.18 (dd, *J* = 8.8, 6.2 Hz, 1H), 4.04 (d, *J= 8.2* Hz, 1H), 3.64 (d, *J* = 8.4 Hz, 1H), 3.52 - 3.42 (m, 1H), 3.40 - 3.35 (m, 1H), 3.33 (s, 3H), 3.30 (s, 3H), 3.27 (s, 3H), 3.24 (s, 3H), 3.23 - 3.17 (m, 1H), 3.09 (d, *J* = 8.2 Hz, 1H), 3.04 (dd, *J* = 9.8, 6.2 Hz, 1H), 2.92 (s, 1H), 2.67 (t, *J* = 6.4 Hz, 1H), 2.59 - 2.52 (m, 2H), 2.52 - 2.44 (m, 2H), 2.44 - 2.39 (m, 2H), 2.38 (s, 3H), 2.34 (s, 3H), 2.11 (q, *J* = 6.4, 5.8 Hz, 3H), 1.90 (dd, *J* = 12.8, 6.8 Hz, 3H), 1.64 (s, 2H), 1.11 (t, *J* = 7.2 Hz, 3H), 0.62 (t, *J* = 6.8 Hz, 3H).

¹³C NMR (100 MHz, CDCl₃) δ 166.99, 166.06, 138.01, 137.85, 133.52, 133.47, 130.92, 130.75, 130.73, 130.46, 128.21, 127.31, 127.11, 85.21, 83.39, 83.30, 80.55, 80.29, 78.21, 77.77, 61.00, 59.17, 58.85, 58.06, 56.26, 56.06, 54.13, 50.90, 49.06, 48.98, 48.17, 45.24, 42.32, 39.16, 37.54, 36.27, 35.00, 29.82, 26.40, 21.45, 21.36, 15.33, 13.60.

### Example 63 Preparation of compound 63

100 mg of compound 4 was added into a 5 mL round-bottom flask and dissolved with 2 mL of dry pyridine. Under an argon atmosphere, the reaction mixture was added with 2 equivalents of 4-ethylbenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 118.9 mg of a light yellow solid as a target compound 63 (C₄₅H₆₁NO₉, 78.3% yield), which was structurally shown as follows:

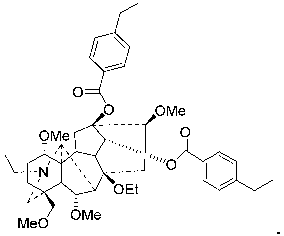

The target compound 63 was characterized as follows.

HRESIMS *m*/*z* : [(M + H)⁺, 760.43]

¹H NMR (400 MHz, Chloroform-d) δ 8.05 (d, *J* = 8.2 Hz, 2H), 7.91 (d, *J* = 8.2 Hz, 2H), 7.24 (d, *J* = 8.2 Hz, 2H), 7.19 (d, *J* = 8.2 Hz, 2H), 5.26 (d, *J* = 5.2 Hz, 1H), 4.20 - 4.09 (m, 1H), 4.04 (d, *J* = 8.2 Hz, 1H), 3.64 (d, *J* = 8.2 Hz, 1H), 3.47 (q, *J* = 11.6 Hz, 1H), 3.40 - 3.34 (m, 1H), 3.32 (s, 3H), 3.30 (s, 3H), 3.27 (s, 3H), 3.24 (s, 3H), 3.22 - 3.17 (m, 1H), 3.09 (d, *J* = 8.2 Hz, 1H), 3.03 (dd, *J* = 9.6, 6.2 Hz, 1H), 2.90 (s, 1H), 2.67 (dq, *J* = 15.2, 7.6 Hz, 5H), 2.55 (t, *J* = 3.6 Hz, 1H), 2.52 (t, *J* = 3.6 Hz, 1H), 2.48 (s, 1H), 2.45 (d, *J* = 4.8 Hz, 1H), 2.39 (s, 1H), 2.37 (d, *J* = 4.6 Hz, 1H), 2.34 (d, *J* = 5.6 Hz, 1H), 2.09 (d, *J* = 8.6 Hz, 3H), 1.89 (dd, *J* = 11.6, 5.8 Hz, 1H), 1.73 (s, 2H), 1.64 (s, 1H), 1.25 - 1.22 (m, 3H), 1.22 - 1.18 (m, 3H), 1.11 (t, *J* = 7.2 Hz, 3H), 0.61 (t, *J* = 6.8 Hz, 3H).

¹³C NMR (100 MHz, CDCl₃) δ 166.86, 165.95, 149.55, 149.48, 130.25, 130.25, 130.08, 130.08, 128.51, 128.33, 127.83, 127.83, 127.83, 127.83, 85.18, 83.30, 83.23, 80.64, 80.31, 78.18, 77.72, 60.89, 59.18, 58.85, 58.19, 56.28, 56.02, 54.18, 50.87, 49.00, 48.94, 48.13, 45.20, 42.33, 39.13, 37.65, 36.28, 35.00, 29.12, 29.07, 26.43, 15.48, 15.47, 15.36, 13.64.

### Example 64 Preparation of compound 64

100 mg of compound 4 was added into a 5 mL round-bottom flask and dissolved with 2 mL of dry pyridine. Under an argon atmosphere, the reaction mixture was added with 2 equivalents of 4-bromobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 160.2 mg of a light yellow solid as a target compound 64 (C₄₁H₅₁Br₂NO₉, 93.2% yield), which was structurally shown as follows:

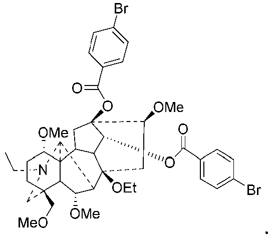

The target compound 64 was characterized as follows.

HRESIMS *m*/*z :* [(M + H)⁺, 860.19]

¹H NMR (400 MHz, Chloroform-d) δ 7.98 (d, *J* = 8.4 Hz, 2H), 7.84 (d, *J* = 8.4 Hz, 2H), 7.57 (d, *J* = 8.4 Hz, 2H), 7.51 (d, *J* = 8.6 Hz, 2H), 5.25 (d, *J* = 5.4 Hz, 1H), 4.13 (t, *J* = 7.4 Hz, 1H), 4.02 (d, *J* = 6.6 Hz, 1H), 3.65 (d, *J* = 8.2 Hz, 1H), 3.51 - 3.42 (m, 1H), 3.42 - 3.36 (m, 1H), 3.30 (s, 3H), 3.29 (s, 3H), 3.27 (s, 3H), 3.24 (s, 3H), 3.21 - 3.15 (m, 1H), 3.07 (d, *J* = 8.4 Hz, 1H), 3.03 (dd, *J* = 9.8, 6.2 Hz, 1H), 2.88 (s, 1H), 2.66 (t, *J* = 5.2 Hz, 1H), 2.57 - 2.53 (m, 1H), 2.51 (s, 1H), 2.49 (s, 1H), 2.46 (s, 1H), 2.40 (s, 1H), 2.36 - 2.31 (m, 2H), 2.10 (t, *J* = 6.6 Hz, 3H), 1.91 (dd, *J* = 11.8, 5.8 Hz, 1H), 1.65 (d, *J* = 18.6 Hz, 3H), 1.10 (t, *J* = 7.2 Hz, 3H), 0.64 (t, *J* = 6.8 Hz, 3H).

¹³C NMR (100 MHz, CDCl₃) δ 165.94, 165.11, 131.73, 131.73, 131.66, 131.66, 131.63, 131.63, 131.50, 131.50, 129.83, 129.71, 127.98, 127.86, 85.24, 83.75, 83.20, 80.35, 80.24, 78.16, 77.95, 61.14, 59.18, 58.93, 58.17, 56.27, 56.12, 53.98, 50.85, 49.11, 49.05, 48.23, 45.21, 42.24, 39.16, 37.66, 36.14, 35.16, 26.44, 15.56, 13.66.

### Example 65 Preparation of compound 65

100 mg of compound 4 was added into a 5 mL round-bottom flask and dissolved with 2 mL of dry pyridine. Under an argon atmosphere, the reaction mixture was added with 2 equivalents of 4-chlorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 129.9 mg of a light yellow solid as a target compound 65 (C₄₁H₅₁Cl₂NO₉, 84.2% yield), which was structurally shown as follows:

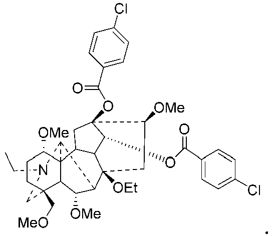

The target compound 65 was characterized as follows.

HRESIMS *m*/*z :* [(M + H)⁺, 772.29]

¹H NMR (400 MHz, Chloroform-d) δ 8.06 (d, J = 8.6 Hz, 2H), 7.92 (d, J = 8.6 Hz, 2H), 7.40 (d, J = 8.6 Hz, 2H), 7.34 (d, J = 8.6 Hz, 2H), 5.25 (d, J = 5.2 Hz, 1H), 4.17 - 4.11 (m, 1H), 4.02 (dd, J = 6.6, 1.6 Hz, 1H), 3.65 (d, J = 8.2 Hz, 1H), 3.52 - 3.42 (m, 1H), 3.42 - 3.36 (m, 1H), 3.30 (s, 3H), 3.29 (s, 3H), 3.27 (s, 3H), 3.24 (s, 3H), 3.18 (dd, J = 8.2, 6.8 Hz, 1H), 3.07 (d, J = 8.2 Hz, 1H), 3.03 (dd, J = 9.8, 6.2 Hz, 1H), 2.89 (s, 1H), 2.69 - 2.64 (m, 1H), 2.57 - 2.50 (m, 2H), 2.48 (d, J = 3.0 Hz, 1H), 2.45 (d, J = 4.8 Hz, 1H), 2.40 (d, J = 1.6 Hz, 1H), 2.34 (tt, J = 7.8, 3.8 Hz, 3H), 2.10 (t, J = 6.4 Hz, 3H), 1.91 (dt, J = 11.6, 5.2 Hz, 1H), 1.79 (s, 1H), 1.63 (d, J = 4.8 Hz, 1H), 1.10 (t, J = 7.2 Hz, 3H), 0.64 (t, J = 6.8 Hz, 3H).

¹³C NMR (100 MHz, CDCl₃) δ 165.79, 164.94, 139.25, 139.15, 131.48, 131.48, 131.34, 131.34, 129.39, 129.26, 128.71, 128.71, 128.64, 128.64, 85.21, 83.71, 83.20, 80.39, 80.23, 78.14, 77.93, 61.11, 59.16, 58.92, 58.17, 56.25, 56.09, 53.98, 50.84, 49.10, 49.02, 48.21, 45.21, 42.23, 39.15, 37.66, 36.16, 35.15, 26.43, 15.53, 13.66.

### Example 66 Preparation of compound 66

100 mg of compound 4 was added into a 5 mL round-bottom flask and dissolved with 2 mL of dry pyridine. Under an argon atmosphere, the reaction mixture was added with 2 equivalents of 2-methoxybenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 127.5 mg of a light yellow solid as a target compound 66 (C₄₃H₅₇NO₁₁, 83.5% yield), which was structurally shown as follows:

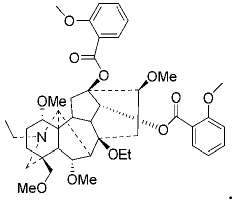

The target compound 66 was characterized as follows.

HRESIMS *m*/*z :* [(M + H)⁺, 764.39]

¹H NMR (400 MHz, Chloroform-*d*) δ 8.08 (dd, *J* = 8.0, 1.8 Hz, 1H), 7.81 (dd, *J* = 7.8, 1.8 Hz, 1H), 7.42 (dddd, *J* = 21.6, 8.4, 7.4, 1.8 Hz, 2H), 6.98 - 6.88 (m, 4H), 5.22 (d, *J* = 5.2 Hz, 1H), 4.17 - 4.11 (m, 1H), 4.05 (d, *J* = 5.4 Hz, 1H), 3.91 (s, 3H), 3.84 (s, 3H), 3.63 (d, *J* = 8.4 Hz, 1H), 3.51 - 3.45 (m, 1H), 3.42 - 3.37 (m, 1H), 3.36 (s, 3H), 3.30 (s, 3H), 3.27 (s, 3H), 3.25 (s, 3H), 3.22 (d, *J* = 8.0 Hz, 1H), 3.10 (d, *J* = 8.4 Hz, 1H), 3.04 (dd, *J* = 9.4, 6.2 Hz, 1H), 2.90 (s, 1H), 2.69 (dd, *J* = 7.4, 5.4 Hz, 1H), 2.56 (d, *J=* 10.6 Hz, 1H), 2.52 - 2.47 (m, 1H), 2.47 - 2.41 (m, 2H), 2.39 (d, *J* = 1.8 Hz, 1H), 2.32 (dd, *J* = 14.2, 8.6 Hz, 2H), 2.10 (s, 1H), 1.87 (dd, *J* = 12.2, 6.0 Hz, 1H), 1.66 (s, 5H), 1.10 (t, *J* = 7.2 Hz, 3H), 0.71 (t, *J* = 6.8 Hz, 3H).

¹³C NMR (100 MHz, CDCl₃) δ 165.25, 165.15, 160.23, 159.61, 133.74, 133.26, 133.26, 132.38, 120.91, 120.17, 120.08, 119.66, 112.25, 111.94, 84.98, 83.41, 83.16, 80.69, 80.35, 78.31, 78.31, 60.37, 59.19, 58.83, 58.24, 56.22, 56.17, 55.98, 54.49, 50.91, 48.83, 48.73, 47.96, 45.03, 42.40, 39.11, 37.49, 36.26, 34.79, 29.83, 26.39, 15.45, 13.58.

### Example 67 Preparation of compound 67

100 mg of compound 4 was added into a 5 mL round-bottom flask and dissolved with 2 mL of dry pyridine. Under an argon atmosphere, the reaction mixture was added with 2 equivalents of 2-bromobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 122.5 mg of a light yellow solid as a target compound 67 (C₄₁H₅₁Br₂NO₉, 71.3% yield), which was structurally shown as follows:

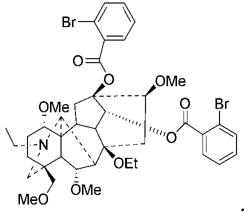

The target compound 67 was characterized as follows.

HRESIMS *m*/*z* : [(M + H)⁺, 860.19]

¹H NMR (400 MHz, Chloroform-d) δ 8.14 (dd, *J* = 7.6, 2.2 Hz, 1H), 7.81 (dd, *J* = 7.4, 2.0 Hz, 1H), 7.65 (dd, *J* = 7.8, 1.4 Hz, 1H), 7.59 (dd, *J* = 7.8, 1.4 Hz, 1H), 7.35 - 7.25 (m, 4H), 5.26 (d, *J* = 5.2 Hz, 1H), 4.19 - 4.13 (m, 1H), 4.04 (d, *J* = 5.6 Hz, 1H), 3.63 (d, *J* = 8.4 Hz, 1H), 3.53 (dd, *J* = 14.6, 4.8 Hz, 1H), 3.47 - 3.39 (m, 1H), 3.36 (s, 3H), 3.30 (s, 3H), 3.29 (s, 3H), 3.25 (s, 3H), 3.22 (d, *J* = 8.4 Hz, 1H), 3.09 (d, *J* = 8.4 Hz, 1H), 3.05 (dd, *J* = 9.8, 6.4 Hz, 1H), 2.89 (s, 1H), 2.70 (t, *J* = 6.0 Hz, 1H), 2.57 - 2.49 (m, 2H), 2.47 (d, *J* = 3.6 Hz, 1H), 2.45 (d, *J* = 4.8 Hz, 1H), 2.41 (d, *J* = 6.8 Hz, 1H), 2.34 (dd, *J* = 7.8, 5.2 Hz, 2H), 2.18 (d, *J* = 12.4 Hz, 1H), 2.13 - 2.07 (m, 2H), 1.90 (td, *J* = 11.4, 10.8, 4.4 Hz, 1H), 1.75 (s, 1H), 1.64 (td, *J* = 10.8, 5.4 Hz, 2H), 1.10 (t, *J* = 7.2 Hz, 3H), 0.76 (t, *J* = 6.8 Hz, 3H).

¹³C NMR (100 MHz, CDCl₃) δ 165.23, 164.85, 134.76, 134.26, 133.05, 132.84, 132.60, 132.39, 131.91, 130.76, 127.18, 127.11, 123.06, 121.90, 85.17, 84.00, 83.25, 80.23, 78.19, 77.89, 60.89, 59.15, 58.91, 58.21, 56.18, 56.12, 54.09, 53.55, 50.87, 49.08, 48.95, 48.20, 45.22, 42.23, 39.15, 37.54, 36.14, 35.07, 26.45, 15.58, 13.64.

### Example 68 Preparation of compound 68

100 mg of compound 4 was added into a 5 mL round-bottom flask and dissolved with 2 mL of dry pyridine. Under an argon atmosphere, the reaction mixture was added with 2 equivalents of 2-chlorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 104.7 mg of a light yellow solid as a target compound 68 (C₄₁H₅₁Cl₂NO₉, 67.9% yield), which was structurally shown as follows:

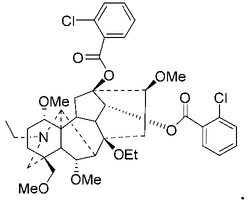

The target compound 68 was characterized as follows.

HRESIMS *m*/*z :* [(M + H)⁺, 772.29]

¹H NMR (400 MHz, Chloroform-d) δ 8.13 (dd, *J* = 7.8, 1.8 Hz, 1H), 7.82 (dd, *J* = 7.8, 1.8 Hz, 1H), 7.45 - 7.31 (m, 4H), 7.29 (ddd, *J* = 7.8, 7.2, 1.6 Hz, 1H), 7.23 (dd, *J= 7.2,* 1.4 Hz, 1H), 5.28 (s, 1H), 5.25 (d, *J* = 5.4 Hz, 1H), 4.18 - 4.12 (m, 1H), 4.04 (d, *J=* 5.4 Hz, 1H), 3.64 (d, *J* = 8.4 Hz, 1H), 3.56 - 3.49 (m, 1H), 3.46 - 3.38 (m, 1H), 3.35 (s, 3H), 3.30 (s, 3H), 3.29 (s, 3H), 3.25 (s, 3H), 3.09 (d, *J* = 8.2 Hz, 1H), 3.05 (dd, *J* = 9.8, 6.4 Hz, 1H), 2.89 (s, 1H), 2.72 - 2.67 (m, 1H), 2.58 - 2.49 (m, 2H), 2.49 - 2.42 (m, 2H), 2.40 (s, 1H), 2.38 - 2.28 (m, 3H), 2.13 - 2.08 (m, 2H), 1.94 - 1.86 (m, 1H), 1.78 (s, 1H), 1.64 (td, *J* = 11.4, 10.6, 5.4 Hz, 2H), 1.09 (t, *J* = 7.2 Hz, 3H), 0.75 (t, *J* = 6.8 Hz, 3H).

¹³C NMR (100 MHz, CDCl₃) δ 164.72, 164.47, 134.97, 133.84, 132.94, 132.76, 132.32, 131.88, 131.33, 130.96, 130.68, 129.12, 126.57, 126.50, 85.11, 83.93, 83.26, 80.24, 78.20, 77.83, 60.80, 59.14, 58.89, 58.16, 56.15, 56.10, 54.12, 53.55, 50.86, 49.04, 48.90, 48.17, 45.17, 42.24, 39.14, 37.53, 36.13, 35.03, 26.44, 15.52, 13.62.

### Example 69 Preparation of compound 69

100 mg of compound 4 was added into a 5 mL round-bottom flask and dissolved with 2 mL of dry pyridine. Under an argon atmosphere, the reaction mixture was added with 2 equivalents of 2-fluorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 101.1 mg of a light yellow solid as a target compound 69 (C₄₁H₅₁F₂NO₉, 68.4% yield), which was structurally shown as follows:

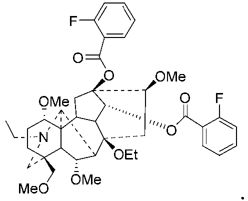

The target compound 69 was characterized as follows.

HRESIMS *m*/*z :* [(M + H)⁺, 740.35]

¹H NMR (400 MHz, Chloroform-*d*) δ 8.08 (td, *J* = 7.6, 1.8 Hz, 1H), 7.89 (td, *J* = 7.6, 1.8 Hz, 1H), 7.53 - 7.41 (m, 2H), 7.20 - 7.03 (m, 4H), 5.27 (d, *J* = 5.2 Hz, 1H), 4.17 - 4.11 (m, 1H), 4.04 (d, *J* = 6.4 Hz, 1H), 3.64 (d, *J* = 8.2 Hz, 1H), 3.57 - 3.47 (m, 1H), 3.45 - 3.36 (m, 1H), 3.34 (s, 3H), 3.30 (s, 3H), 3.28 (s, 3H), 3.25 (s, 3H), 3.08 (d, *J* = 8.4 Hz, 1H), 3.04 (dd, *J* = 9.6, 6.2 Hz, 1H), 2.89 (s, 1H), 2.70 (t, *J* = 5.4 Hz, 1H), 2.57 - 2.50 (m, 2H), 2.48 - 2.43 (m, 2H), 2.43 - 2.38 (m, 2H), 2.36 - 2.31 (m, 1H), 2.17 - 2.05 (m, 3H), 1.94 - 1.85 (m, 1H), 1.73 (s, 2H), 1.65 (dd, *J* = 12.8, 4.4 Hz, 2H), 1.10 (t, *J* = 7.2 Hz, 3H), 0.70 (t, *J* = 6.8 Hz, 3H).

¹³C NMR (100 MHz, CDCl₃) δ 163.62, 163.20, 161.16, 160.82, 134.31, 134.02, 132.77, 132.32, 123.79, 123.79, 119.41, 118.88, 116.92, 116.70, 84.96, 83.59, 83.18, 80.28, 80.15, 78.14, 77.76, 60.60, 59.06, 58.78, 57.98, 56.13, 55.94, 54.09, 50.74, 48.76, 47.94, 44.93, 42.08, 39.00, 37.21, 36.12, 34.86, 26.31, 22.69, 15.32, 13.50.

### Example 70 Preparation of compound 70

100 mg of compound 4 was added into a 5 mL round-bottom flask and dissolved with 2 mL of dry pyridine. Under an argon atmosphere, the reaction mixture was added with 2 equivalents of 3-bromobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 122.9 mg of a light yellow solid as a target compound 70 (C₄₁H₅₁Br₂NO₉, 71.5% yield), which was structurally shown as follows:

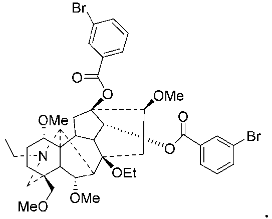

The target compound 70 was characterized as follows.

HRESIMS *m*/*z :* [(M + H)⁺, 860.19]

¹H NMR (400 MHz, Chloroform-*d*) δ 8.29 (t, *J* = 1.8 Hz, 1H), 8.12 (t, *J* = 1.8 Hz, 1H), 8.06 (dt, *J* = 7.8, 1.2 Hz, 1H), 7.92 (dt, *J* = 7.8, 1.2 Hz, 1H), 7.64 (dddd, *J* = 14.2, 8.0, 2.0, 1.2 Hz, 2H), 7.31 (t, *J* = 7.8 Hz, 1H), 7.28 - 7.24 (m, 1H), 5.29 (d, *J* = 5.2 Hz, 1H), 4.17 (t, *J= 7.2 Hz,* 1H), 4.02 (dd, *J* = 6.6, 1.4 Hz, 1H), 3.65 (d, *J* = 8.2 Hz, 1H), 3.50 - 3.43 (m, 1H), 3.42 - 3.36 (m, 1H), 3.32 (s, 3H), 3.30 (s, 3H), 3.28 (s, 3H), 3.24 (s, 3H), 3.07 (d, *J* = 8.2 Hz, 1H), 3.03 (dd, *J* = 9.8, 6.2 Hz, 1H), 2.90 (s, 1H), 2.66 (dt, *J* = 7.2, 3.4 Hz, 1H), 2.57 - 2.50 (m, 2H), 2.50 - 2.43 (m, 2H), 2.42 (s, 1H), 2.37 (d, *J* = 7.4 Hz, 2H), 2.14 - 2.07 (m, 3H), 1.92 (dd, *J* = 11.8, 6.2 Hz, 1H), 1.70 (d, *J* = 2.2 Hz, 3H), 1.65 - 1.60 (m, 1H), 1.11 (t, *J= 7.2* Hz, 3H), 0.66 (t, *J* = 6.8 Hz, 3H).

¹³C NMR (100 MHz, CDCl₃) δ 165.35, 164.48, 135.76, 135.74, 133.37, 132.94, 132.85, 132.74, 129.95, 129.95, 128.63, 128.53, 122.44, 122.39, 85.28, 84.02, 83.19, 80.24, 80.14, 78.16, 77.92, 61.22, 59.17, 58.93, 57.98, 56.27, 56.18, 53.94, 50.86, 49.14, 49.07, 48.27, 45.32, 42.25, 39.18, 37.46, 36.11, 35.22, 26.43, 15.47, 13.67.

### Example 71 Preparation of compound 71

100 mg of compound 4 was added into a 5 mL round-bottom flask and dissolved with 2 mL of dry pyridine. Under an argon atmosphere, the reaction mixture was added with 2 equivalents of 3-chlorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 114.0 mg of a light yellow solid as a target compound 71 (C₄₁H₅₁Cl₂NO₉, 73.9% yield), which was structurally shown as follows:

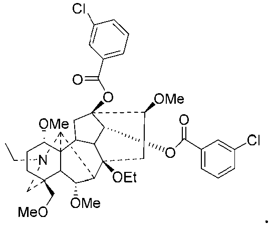

The target compound 71 was characterized as follows.

HRESIMS *m*/*z* : [(M + H)⁺, 772.29]

¹H NMR (400 MHz, Chloroform-d) δ 8.13 *(t, J=* 1.8 Hz, 1H), 8.01 (dt, *J* = 7.8, 1.4 Hz, 1H), 7.97 (t, *J* = 1.8 Hz, 1H), 7.87 (dt, *J* = 7.8, 1.4 Hz, 1H), 7.48 (dddd, *J* = 15.2, 8.0, 2.2, 1.2 Hz, 2H), 7.34 (dt, *J* = 21.8, 7.8 Hz, 2H), 5.29 (s, 1H), 4.16 (t, *J* = 7.4 Hz, 1H), 4.02 (d, *J* = 8.4 Hz, 1H), 3.65 (d, *J* = 8.2 Hz, 1H), 3.51 - 3.44 (m, 1H), 3.39 (dd, *J* = 14.6, 7.6 Hz, 1H), 3.31 (s, 3H), 3.30 (s, 3H), 3.28 (s, 3H), 3.23 (s, 3H), 3.20 (d, *J* = 6.8 Hz, 1H), 3.07 (d, *J* = 8.2 Hz, 1H), 3.03 (dd, *J* = 9.8, 6.2 Hz, 1H), 2.90 (s, 1H), 2.66 (t, *J=* 5.2 Hz, 1H), 2.57 - 2.50 (m, 2H), 2.48 (d, *J* = 4.4 Hz, 1H), 2.47 - 2.40 (m, 2H), 2.37 (d, *J=* 7.4 Hz, 2H), 2.10 (t, *J=* 7.0 Hz, 3H), 1.97 - 1.87 (m, 1H), 1.78 (s, 1H), 1.68 - 1.58 (m, 2H), 1.10 *(t, J=* 7.2 Hz, 3H), 0.66 (t, *J* = 6.8 Hz, 3H).

¹³C NMR (100 MHz, CDCl₃) δ 165.32, 164.48, 134.34, 134.30, 132.72, 132.67, 132.59, 132.48, 130.25, 129.90, 129.55, 129.55, 128.07, 127.94, 85.15, 83.89, 83.10, 80.14, 80.10, 78.05, 77.82, 61.08, 59.04, 58.80, 57.88, 56.09, 56.03, 53.84, 53.44, 50.75, 48.93, 48.23, 45.22, 42.15, 39.08, 37.36, 36.02, 35.10, 26.32, 15.32, 13.54.

### Example 72 Preparation of compound 72

100 mg of compound 4 was added into a 5 mL round-bottom flask and dissolved with 2 mL of dry pyridine. Under an argon atmosphere, the reaction mixture was added with 2 equivalents of 3-fluorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 121.4 mg of a light yellow solid as a target compound 72 (C₄₁H₅₁F₂NO₉, 82.1% yield), which was structurally shown as follows:

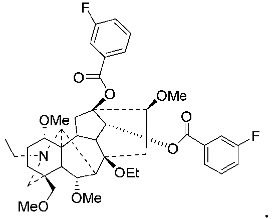

The target compound 72 was characterized as follows.

HRESIMS *m*/*z :* [(M + H)⁺, 740.35]

¹H NMR (400 MHz, Chloroform-*d*) δ 7.92 (dt, *J* = 7.8, 1.2 Hz, 1H), 7.82 (ddd, *J* = 9.6, 2.6, 1.4 Hz, 1H), 7.78 (dt, *J* = 7.8, 1.2 Hz, 1H), 7.67 (ddd, *J* = 9.4, 2.8, 1.4 Hz, 1H), 7.38 (dtd, *J* = 22.8, 8.0, 5.4 Hz, 2H), 7.26 - 7.16 (m, 2H), 5.27 (d, *J* = 5.2 Hz, 1H), 4.16 (t, *J=* 7.4 Hz, 1H), 4.02 (dd, *J* = 6.6, 1.6 Hz, 1H), 3.65 (d, *J* = 8.2 Hz, 1H), 3.52 - 3.43 (m, 1H), 3.43 - 3.35 (m, 1H), 3.31 (s, 3H), 3.30 (s, 3H), 3.28 (s, 3H), 3.24 (s, 3H), 3.21 (dd, *J* = 8.2, 6.8 Hz, 1H), 3.07 (d, *J* = 8.2 Hz, 1H), 3.03 (dd, *J* = 9.8, 6.2 Hz, 1H), 2.90 (s, 1H), 2.67 (td, *J* = 5.8, 1.6 Hz, 1H), 2.57 - 2.53 (m, 1H), 2.52 (s, 1H), 2.48 (d, *J* = 3.6 Hz, 1H), 2.45 (d, *J* = 5.0 Hz, 1H), 2.41 (s, 1H), 2.36 (d, *J* = 7.4 Hz, 3H), 2.14 - 2.05 (m, 3H), 1.96 - 1.87 (m, 1H), 1.69 - 1.57 (m, 2H), 1.10 (t, *J* = 7.2 Hz, 3H), 0.64 (t, *J* = 6.8 Hz, 3H).

¹³C NMR (100 MHz, CDCl₃) δ 165.52, 164.69, 163.81, 161.36, 133.13, 133.00, 130.00, 129.93, 125.83, 125.66, 120.02, 119.81, 117.12, 116.89, 85.24, 83.84, 83.19, 80.29, 80.23, 78.15, 77.97, 61.14, 59.16, 58.91, 58.11, 56.24, 56.12, 53.96, 50.84, 49.11, 49.03, 48.24, 45.23, 42.22, 39.15, 37.59, 36.13, 35.17, 26.43, 15.38, 13.65.

### Example 73 Preparation of compound 73

100 mg of compound 4 was added into a 5 mL round-bottom flask and dissolved with 2 mL of dry pyridine. Under an argon atmosphere, the reaction mixture was added with 2 equivalents of 2,4-dichlorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 144.5 mg of a light yellow solid as a target compound 73 (C₄₁H₄₉Cl₄NO₉, 86.1% yield), which was structurally shown as follows:

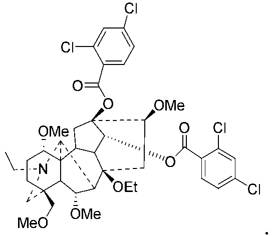

The target compound 73 was characterized as follows.

HRESIMS *m*/*z* : [(M + H)⁺, 840.21]

¹H NMR (400 MHz, Chloroform-d) δ 8.08 (d, *J=* 8.4 Hz, 1H), 7.77 (d, *J* = 8.4 Hz, 1H), 7.46 (d, *J* = 2.0 Hz, 1H), 7.41 (d, *J* = 2.2 Hz, 1H), 7.29 (dd, *J* = 8.4, 2.2 Hz, 1H), 7.23 (dd, *J* = 8.4, 2.0 Hz, 1H), 5.23 (d, *J=* 5.2 Hz, 1H), 4.12 (dd, *J* = 8.8, 6.2 Hz, 1H), 4.05 - 4.00 (m, 1H), 3.64 (d, *J= 8.2* Hz, 1H), 3.50 (d, *J* = 10.4 Hz, 1H), 3.43 (dd, *J=* 8.2, 6.8 Hz, 1H), 3.31 (s, 3H), 3.30 (s, 3H), 3.29 (s, 3H), 3.24 (s, 3H), 3.07 (d, *J* = 8.4 Hz, 1H), 3.05 - 3.00 (m, 1H), 2.87 (s, 1H), 2.67 (t, *J* = 6.2 Hz, 1H), 2.53 (t, *J* = 8.8 Hz, 2H), 2.47 (dd, *J* = 10.2, 6.8 Hz, 2H), 2.40 (s, 1H), 2.35 (dd, *J* = 14.4, 8.8 Hz, 2H), 2.26 (dd, *J* = 14.4, 6.2 Hz, 1H), 2.17 - 2.05 (m, 3H), 1.95 - 1.86 (m, 1H), 1.69 - 1.57 (m, 2H), 1.09 (t, *J* = 7.2 Hz, 3H), 0.78 (t, *J* = 6.8 Hz, 3H).

¹³C NMR (100 MHz, CDCl₃) δ 163.81, 163.58, 138.55, 138.07, 136.05, 135.02, 133.85, 132.93, 131.22, 130.90, 128.92, 127.50, 127.01, 127.01, 85.12, 84.21, 83.19, 80.20, 80.06, 78.17, 77.85, 60.89, 59.15, 58.95, 58.12, 56.14, 54.04, 54.04, 50.84, 49.10, 48.94, 48.20, 45.18, 42.21, 39.14, 37.54, 36.04, 35.12, 26.43, 15.68, 13.63.

### Example 74 Preparation of compound 74

100 mg of compound 4 was added into a 5 mL round-bottom flask and dissolved with 2 mL of dry pyridine. Under an argon atmosphere, the reaction mixture was added with 2 equivalents of 3,5-difluorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 137.2 mg of a light yellow solid as a target compound 74 (C₄₁H₄₉F₄NO₉, 88.5% yield), which was structurally shown as follows:

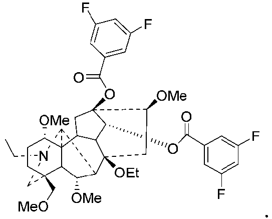

The target compound 74 was characterized as follows.

HRESIMS *m*/*z :* [(M + H)⁺, 776.33]

¹H NMR (400 MHz, Chloroform-*d*) δ 7.65 (dd, *J* = 7.8, 2.4 Hz, 2H), 7.50 (dd, *J* = 7.8, 2.4 Hz, 2H), 6.97 (dtt, *J* = 14.8, 8.4, 2.4 Hz, 2H), 5.26 (d, *J* = 5.2 Hz, 1H), 4.14 (dd, *J* = 8.8, 5.6 Hz, 1H), 4.01 (dd, *J* = 6.4, 1.6 Hz, 1H), 3.66 (d, *J* = 8.2 Hz, 1H), 3.50 - 3.38 (m, 2H), 3.30 (s, 3H), 3.30 (s, 3H), 3.28 (s, 3H), 3.24 (s, 3H), 3.08 - 3.04 (m, 1H), 3.04 - 3.00 (m, 1H), 2.89 (s, 1H), 2.66 (t, *J* = 6.2 Hz, 1H), 2.55 (dd, *J* = 7.0, 4.8 Hz, 1H), 2.53 - 2.51 (m, 1H), 2.49 (d, *J* = 6.2 Hz, 1H), 2.47 - 2.44 (m, 1H), 2.44 - 2.40 (m, 1H), 2.40 - 2.29 (m, 3H), 2.14 - 2.07 (m, 3H), 1.96 - 1.89 (m, 1H), 1.67 (s, 2H), 1.63 (td, *J* = 5.6, 2.4 Hz, 1H), 1.10 (t, *J=* 7.2 Hz, 3H), 0.70 (t, *J* = 6.8 Hz, 3H).

¹³C NMR (100 MHz, CDCl₃) δ 164.37, 164.12, 164.00, 163.59, 161.63, 161.52, 134.15, 134.09, 113.27, 113.09, 113.01, 112.83, 108.32, 108.24, 85.30, 84.34, 83.17, 80.23, 80.06, 78.15, 78.15, 61.31, 59.17, 58.98, 58.04, 56.21, 56.21, 53.88, 50.87, 49.25, 49.11, 48.39, 45.34, 42.22, 39.22, 37.53, 36.03, 35.30, 26.45, 15.46, 13.67.

### Example 75 Preparation of compound 75

100 mg of compound 4 was added into a 5 mL round-bottom flask and dissolved with 2 mL of dry pyridine. Under an argon atmosphere, the reaction mixture was added with 2 equivalents of 2-methylbenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 128.1 mg of a light yellow solid as a target compound 75 (C₄₃H₅₇NO₉, 87.6% yield), which was structurally shown as follows:

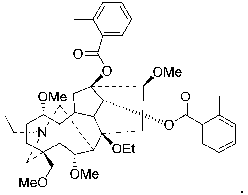

The target compound 75 was characterized as follows.

HRESIMS *m*/*z :* [(M + H)⁺, 732.40]

¹H NMR (400 MHz, Chloroform-d) δ 8.12 (dd, J = 8.2, 1.4 Hz, 1H), 7.87 (dd, *J* = 8.2, 1.4 Hz, 1H), 7.42 - 7.29 (m, 2H), 7.25 - 7.15 (m, 4H), 5.19 (d, *J* = 5.2 Hz, 1H), 4.15 (t, *J* = 7.6 Hz, 1H), 4.06 (dd, *J* = 6.4, 1.4 Hz, 1H), 3.64 (d, *J* = 8.2 Hz, 1H), 3.53 (d, *J* = 9.8 Hz, 1H), 3.43 - 3.34 (m, 1H), 3.33 (s, 3H), 3.31 (s, 3H), 3.29 (s, 3H), 3.26 (s, 3H), 3.21 - 3.15 (m, 1H), 3.10 (d, *J* = 8.2 Hz, 1H), 3.05 (dd, *J* = 9.8, 6.2 Hz, 1H), 2.90 (s, 1H), 2.69 (dd, *J* = 5.8, 3.4 Hz, 1H), 2.66 (s, 3H), 2.55 (s, 3H), 2.53 (d, *J* = 4.6 Hz, 1H), 2.39 (d, *J* = 1.4 Hz, 1H), 2.33 (dd, *J* = 7.8, 2.6 Hz, 3H), 2.10 (dt, *J* = 5.8, 3.8 Hz, 3H), 1.90 (dd, *J* = 11.2, 5.6 Hz, 1H), 1.74 (s, 3H), 1.62 (d, *J* = 7.8 Hz, 2H), 1.10 (t, *J= 7.2* Hz, 3H), 0.68 (t, *J* = 6.8 Hz, 3H).

¹³C NMR (100 MHz, CDCl₃) δ 167.25, 167.23, 141.00, 139.92, 131.93, 131.64, 131.64, 131.57, 131.49, 130.96, 130.80, 129.63, 125.70, 125.68, 85.24, 83.35, 83.30, 80.30, 80.30, 78.21, 78.21, 61.00, 59.18, 58.88, 57.97, 56.24, 56.03, 54.13, 50.90, 49.08, 49.01, 48.18, 45.13, 42.37, 39.14, 37.52, 36.39, 35.03, 26.46, 22.03, 21.67, 15.44, 13.65.

### Example 76 Preparation of compound 76

100 mg of compound 4 was added into a 5 mL round-bottom flask and dissolved with 2 mL of dry pyridine. Under an argon atmosphere, the reaction mixture was added with 2 equivalents of 3-methoxybenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 99.7 mg of a light yellow solid as a target compound 76 (C₄₃H₅₇NO₁₁, 65.3% yield), which was structurally shown as follows:

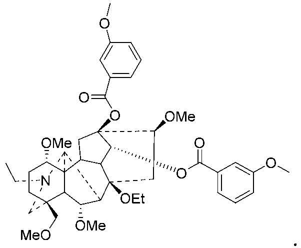

The target compound 76 was characterized as follows.

HRESIMS *m*/*z :* [(M + H)⁺, 764.39]

¹H NMR (400 MHz, Chloroform-d) δ 7.73 (dt, *J* = 7.8, 1.2 Hz, 1H), 7.70 (dd, *J* = 2.8, 1.4 Hz, 1H), 7.59 (dt, *J* = 7.8, 1.2 Hz, 1H), 7.52 (dd, *J=* 2.6, 1.4 Hz, 1H), 7.33 *(t, J=* 7.8 Hz, 1H), 7.27 (t, *J* = 7.8 Hz, 1H), 7.06 (dddd, *J* = 15.8, 8.2, 2.6, 1.0 Hz, 2H), 5.28 (d, *J* = 5.2 Hz, 1H), 4.15 (dd, *J=* 8.6, 6.4 Hz, 1H), 4.04 (dd, *J=* 6.6, 1.4 Hz, 1H), 3.83 (s, 3H), 3.78 (s, 3H), 3.65 (d, *J* = 8.2 Hz, 1H), 3.51 - 3.42 (m, 1H), 3.38 (dt, *J* = 8.0, 6.8 Hz, 1H), 3.31 (s, 3H), 3.30 (s, 3H), 3.28 (s, 3H), 3.25 (s, 3H), 3.21 (dd, *J* = 8.2, 6.8 Hz, 1H), 3.08 (d, *J* = 8.2 Hz, 1H), 3.04 (dd, *J* = 9.6, 6.2 Hz, 1H), 2.91 (s, 1H), 2.70 - 2.65 (m, 1H), 2.58 - 2.51 (m, 2H), 2.50 - 2.43 (m, 2H), 2.42 - 2.35 (m, 3H), 2.11 (dd, *J* = 6.8, 2.4 Hz, 2H), 1.90 (q, *J=* 5.8 Hz, 1H), 1.76 (s, 3H), 1.65 (dd, *J* = 13.0, 4.8 Hz, 1H), 1.11 (t, *J* = 7.2 Hz, 3H), 0.65 (t, *J* = 6.8 Hz, 3H).

¹³C NMR (100 MHz, CDCl₃) δ 166.53, 165.74, 159.49, 159.49, 132.31, 132.09, 129.35, 129.33, 122.61, 122.41, 119.67, 119.51, 114.20, 114.05, 85.21, 83.59, 83.25, 80.51, 80.26, 78.17, 77.81, 60.94, 59.17, 58.88, 58.18, 56.29, 56.11, 55.46, 55.44, 54.13, 50.87, 49.04, 48.95, 48.14, 45.19, 42.33, 39.13, 37.67, 36.11, 35.05, 26.41, 15.42, 13.62.

### Example 77 Preparation of compound 77

100 mg of compound 4 was added into a 5 mL round-bottom flask and dissolved with 2 mL of dry pyridine. Under an argon atmosphere, the reaction mixture was added with 2 equivalents of butyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 99.9 mg of a light yellow solid as a target compound 77 (C₃₅H₅₇NO₉, 78.6% yield), which was structurally shown as follows:

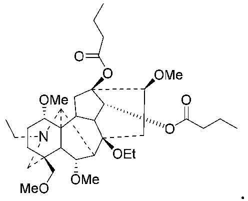

The target compound 77 was characterized as follows.

HRESIMS *m*/*z :* [(M + H)⁺, 636.40]

¹H NMR (400 MHz, Chloroform-d) δ 4.85 (d, *J* = 5.2 Hz, 1H), 3.97 (dd, *J* = 6.6, 1.6 Hz, 1H), 3.89 (t, *J* = 7.8 Hz, 1H), 3.62 (d, *J =* 8.2 Hz, 1H), 3.46 (dt, *J* = 8.8, 7.0 Hz, 1H), 3.37 - 3.30 (m, 2H), 3.28 (d, *J* = 1.8 Hz, 9H), 3.19 (s, 3H), 3.05 (d, *J=* 8.2 Hz, 1H), 2.97 (dd, *J* = 9.4, 6.2 Hz, 1H), 2.76 (s, 1H), 2.48 - 2.45 (m, 2H), 2.43 (t, *J* = 3.6 Hz, 2H), 2.40 (s, 1H), 2.39 - 2.34 (m, 2H), 2.26 (dd, *J* = 7.4, 3.2 Hz, 3H), 2.24 (d, *J* = 3.2 Hz, 1H), 2.20 (d, *J* = 5.2 Hz, 1H), 2.04 (d, *J* = 6.4 Hz, 1H), 1.95 (ddd, *J =* 12.4, 7.2, 5.2 Hz, 1H), 1.88 - 1.80 (m, 3H), 1.62 (dq, *J* = 14.8, 7.4 Hz, 6H), 1.07 (q, *J* = 7.0 Hz, 6H), 0.93 (q, *J* = 7.4 Hz, 6H).

¹³C NMR (100 MHz, CDCl₃) δ 173.83, 172.98, 84.97, 83.39, 82.10, 80.34, 80.22, 78.28, 78.28, 60.64, 59.13, 58.84, 57.96, 56.14, 56.10, 54.16, 50.80, 48.83, 48.67, 47.76, 44.80, 42.05, 39.01, 37.46, 36.40, 36.36, 36.36, 34.85, 26.39, 18.65, 18.05, 16.21, 13.79, 13.69, 13.54.

### Example 78 Preparation of compound 78

100 mg of compound 2 was added into a 5 mL round-bottom flask and dissolved with 2 mL of dry pyridine. Under an argon atmosphere, the reaction mixture was added with 2 equivalents of butyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 64.3 mg of a light yellow solid as a target compound 78 (C₃₉H₅₇NO₁₀, 57.9% yield), which was structurally shown as follows:

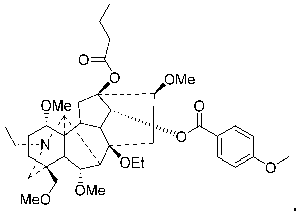

The target compound 78 was characterized as follows.

HRESIMS *m*/*z* : [(M + H)⁺, 700.39]

¹H NMR (400 MHz, Chloroform-*d*) δ 8.03 (d, *J* = 8.8 Hz, 2H), 6.90 (d, *J* = 8.8 Hz, 2H), 5.02 (d, *J* = 5.2 Hz, 1H), 4.03 - 3.97 (m, 2H), 3.84 (s, 3H), 3.61 (d, *J* = 8.2 Hz, 1H), 3.35 (s, 3H), 3.30 (d, *J* = 6.8 Hz, 1H), 3.28 (s, 3H), 3.24 (s, 3H), 3.22 (s, 3H), 3.15 - 3.10 (m, 1H), 3.07 (d, *J* = 8.2 Hz, 1H), 3.00 (dd, *J* = 9.8, 6.2 Hz, 1H), 2.81 (s, 1H), 2.63 - 2.58 (m, 1H), 2.53 - 2.50 (m, 1H), 2.48 (t, *J* = 3.6 Hz, 1H), 2.44 (d, *J* = 2.2 Hz, 1H), 2.43 - 2.39 (m, 1H), 2.33 (s, 1H), 2.31 - 2.28 (m, 2H), 2.28 - 2.25 (m, 2H), 2.25 - 2.22 (m, 1H), 2.05 (d, *J* = 6.4 Hz, 1H), 2.00 (dd, *J* = 7.0, 5.2 Hz, 1H), 1.96 - 1.87 (m, 2H), 1.61 (p, *J* = 7.2 Hz, 4H), 1.38 (d, *J* = 20.4 Hz, 1H), 1.07 (t, *J* = 7.2 Hz, 3H), 0.91 (t, *J* = 7.4 Hz, 3H), 0.55 (t, *J* = 6.8 Hz, 3H).

¹³C NMR (100 MHz, CDCl₃) δ 173.11, 166.36, 163.17, 132.03, 132.03, 123.42, 113.47, 113.47, 85.14, 83.22, 82.56, 80.42, 80.26, 78.07, 77.54, 60.94, 59.13, 58.79, 58.00, 56.16, 55.91, 55.47, 54.06, 50.78, 48.94, 48.91, 48.10, 44.98, 42.19, 39.08, 37.51, 36.50, 36.22, 34.98, 26.41, 18.68, 15.36, 13.67, 13.60.

### Example 79 Preparation of compound 79

100 mg of bulleyaconitine A was added into a 5 mL round-bottom flask and dissolved with 2 mL of dry pyridine. Under an argon atmosphere, the reaction mixture was added with 2 equivalents of butyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 58.7 mg of a light yellow solid as a target compound 79 (C₃₉H₅₅NO₁₁, 53.1% yield), which was structurally shown as follows:

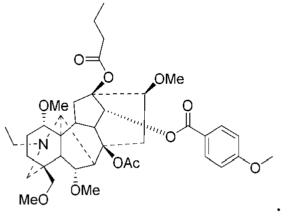

The target compound 79 was characterized as follows.

HRESIMS *m*/*z :* [(M + H)⁺, 714.37]

¹H NMR (400 MHz, Chloroform-d) δ 8.02 (d, *J* = 8.8 Hz, 2H), 6.90 (d, *J* = 8.8 Hz, 2H), 5.06 (d, *J* = 5.2 Hz, 1H), 3.99 (dd, *J* = 8.8, 5.8 Hz, 1H), 3.94 (d, *J* = 6.6 Hz, 1H), 3.84 (s, 3H), 3.60 (d, *J* = 8.4 Hz, 1H), 3.40 (dd, *J* = 15.6, 5.8 Hz, 1H), 3.34 (s, 3H), 3.26 (s, 3H), 3.22 (s, 3H), 3.13 (s, 3H), 3.11 (s, 1H), 2.99 (q, *J* = 6.0 Hz, 3H), 2.94 (s, 1H), 2.82 (dd, *J* = 7.2, 5.4 Hz, 1H), 2.57 - 2.48 (m, 1H), 2.47 - 2.43 (m, 2H), 2.42 - 2.39 (m, 1H), 2.25 (pd, *J* = 8.0, 3.2 Hz, 3H), 2.06 (dd, *J* = 11.2, 6.6 Hz, 2H), 1.97 - 1.87 (m, 3H), 1.62 - 1.57 (m, 3H), 1.28 (s, 3H), 1.23 (s, 1H), 1.07 (t, *J* = 7.2 Hz, 3H), 0.91 (t, *J* = 7.4 Hz, 3H).

¹³C NMR (100 MHz, CDCl₃) δ 173.02, 169.95, 166.23, 163.52, 132.02, 132.02, 122.69, 113.82, 113.82, 85.63, 84.78, 83.29, 82.00, 80.45, 80.12, 77.12, 61.58, 59.19, 58.16, 57.91, 56.05, 55.54, 53.86, 50.32, 49.20, 49.09, 48.96, 43.81, 41.81, 39.66, 39.15, 36.43, 35.66, 34.97, 26.35, 21.77, 18.66, 13.66, 13.55.

### Example 80 Preparation of compound 80

100 mg of compound 4 was added into a 5 mL round-bottom flask and dissolved with 2 mL of dry pyridine. Under an argon atmosphere, the reaction mixture was added with 2 equivalents of isobutyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 66.0 mg of a light yellow solid as a target compound 80 (C₃₅H₅₇NO₉, 51.9% yield), which was structurally shown as follows:

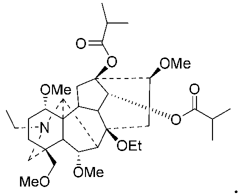

The target compound 80 was characterized as follows.

HRESIMS *m*/*z* : [(M + H)⁺, 636.40]

¹H NMR (400 MHz, Chloroform-d) δ 4.94 (d, *J* = 5.2 Hz, 1H), 3.99 (dd, *J* = 6.8, 1.6 Hz, 1H), 3.84 (t, *J* = 7.8 Hz, 1H), 3.64 (d, *J* = 8.2 Hz, 1H), 3.48 (dq, *J* = 9.0, 7.0 Hz, 1H), 3.35 (dd, *J* = 8.4, 7.0 Hz, 1H), 3.30 (s, 3H), 3.29 (s, 3H), 3.24 (s, 3H), 3.20 (s, 3H), 3.04 (d, *J= 8.2* Hz, 1H), 2.98 (dd, *J* = 9.2, 6.2 Hz, 1H), 2.78 (s, 1H), 2.59 - 2.54 (m, 1H), 2.54 - 2.49 (m, 2H), 2.49 - 2.43 (m, 2H), 2.43 - 2.36 (m, 3H), 2.22 (dd, *J* = 8.0, 2.2 Hz, 2H), 2.06 (d, *J* = 6.4 Hz, 1H), 1.97 (ddd, *J* = 12.2, 7.2, 5.2 Hz, 1H), 1.90 - 1.79 (m, 2H), 1.75 (d, *J* = 3.2 Hz, 2H), 1.62 (tdd, *J* = 13.4, 7.2, 4.8 Hz, 2H), 1.18 (t, *J=* 7.2 Hz, 6H), 1.09 (ddd, *J* = 9.6, 7.0, 4.2 Hz, 12H).

¹³C NMR (100 MHz, CDCl₃) δ 176.84, 176.34, 84.86, 83.52, 82.05, 80.26, 80.10, 78.30, 76.70, 60.37, 59.16, 58.82, 57.82, 56.15, 56.07, 54.38, 50.89, 48.74, 48.49, 47.52, 45.15, 42.33, 38.99, 37.40, 36.06, 34.72, 34.38, 34.21, 26.33, 19.19, 19.13, 19.05, 18.77, 16.16, 13.52.

### Example 81 Preparation of compound 81

100 mg of compound 2 was added into a 5 mL round-bottom flask and dissolved with 2 mL of dry pyridine. Under an argon atmosphere, the reaction mixture was added with 2 equivalents of isobutyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 57.5 mg of a light yellow solid as a target compound 81 (C₃₉H₅₇NO₁₀, 51.8% yield), which was structurally shown as follows:

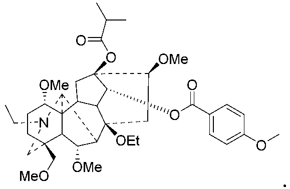

The target compound 81 was characterized as follows.

HRESIMS *m*/*z* : [(M + H)⁺, 700.39]

¹H NMR (400 MHz, Chloroform-d) δ 8.05 (d, *J* = 8.8 Hz, 2H), 6.90 (d, *J* = 8.8 Hz, 2H), 5.03 (d, *J* = 5.2 Hz, 1H), 4.02 - 3.94 (m, 2H), 3.85 (s, 3H), 3.62 (d, *J* = 8.2 Hz, 1H), 3.33 (s, 3H), 3.31 - 3.29 (m, 1H), 3.28 (s, 3H), 3.25 (s, 3H), 3.22 (s, 3H), 3.16 - 3.11 (m, 1H), 3.07 (d, *J* = 8.2 Hz, 1H), 3.01 (dd, *J* = 9.4, 6.2 Hz, 1H), 2.82 (s, 1H), 2.61 (dd, *J* = 7.2, 5.4 Hz, 1H), 2.55 - 2.46 (m, 3H), 2.45 - 2.40 (m, 2H), 2.34 (d, *J* = 1.5 Hz, 1H), 2.28 (dd, *J* = 7.6, 4.4 Hz, 2H), 2.06 (d, *J* = 6.4 Hz, 1H), 2.01 (dd, *J* = 7.0, 5.2 Hz, 1H), 1.94 - 1.82 (m, 3H), 1.61 (q, *J* = 4.2 Hz, 2H), 1.41 (s, 1H), 1.11 (dd, *J* = 6.8, 1.2 Hz, 6H), 1.07 (t, *J* = 7.2 Hz, 3H), 0.57 (t, *J* = 6.8 Hz, 3H).

¹³C NMR (100 MHz, CDCl₃) δ 176.55, 166.36, 163.18, 132.00, 132.00, 123.49, 113.50, 113.50, 85.01, 83.28, 82.34, 80.38, 80.29, 78.13, 77.54, 60.70, 59.15, 58.81, 58.01, 56.12, 55.95, 55.49, 54.23, 50.79, 48.88, 48.82, 48.02, 44.98, 42.29, 39.07, 37.56, 36.09, 34.90, 34.08, 26.39, 19.18, 19.16, 15.43, 13.58.

### Example 82 Preparation of compound 82

100 mg of bulleyaconitine A was added into a 5 mL round-bottom flask and dissolved with 2 mL of dry pyridine. Under an argon atmosphere, the reaction mixture was added with 2 equivalents of isobutyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 59.6 mg of a light yellow solid as a target compound 82 (C₃₉H₅₅NO₁₁, 53.9% yield), which was structurally shown as follows:

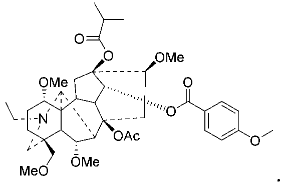

The target compound 82 was characterized as follows.

HRESIMS *m*/*z :* [(M + H)⁺, 712.37]

¹H NMR (400 MHz, Chloroform-d) δ 8.02 (d, *J* = 8.8 Hz, 2H), 6.90 (d, *J* = 8.8 Hz, 2H), 5.05 (d, *J* = 5.2 Hz, 1H), 3.95 (dd, *J* = 8.2, 6.2 Hz, 2H), 3.84 (s, 3H), 3.60 (d, *J* = 8.4 Hz, 1H), 3.38 (dd, *J* = 15.6, 5.8 Hz, 1H), 3.31 (s, 3H), 3.25 (s, 3H), 3.22 (s, 3H), 3.13 (s, 3H), 3.01 - 2.95 (m, 3H), 2.94 (s, 1H), 2.82 (dd, *J=* 7.2, 5.4 Hz, 1H), 2.56 - 2.46 (m, 3H), 2.45 - 2.37 (m, 3H), 2.25 (tdd, *J* = 12.2, 7.2, 3.8 Hz, 1H), 2.06 (dd, *J* = 11.6, 6.2 Hz, 2H), 1.95 - 1.85 (m, 3H), 1.59 (dt, *J* = 12.6, 4.2 Hz, 2H), 1.30 (s, 3H), 1.11 (d, *J* = 1.0 Hz, 3H), 1.09 (d, *J= 1.0* Hz, 3H), 1.06 (t, *J* = 7.2 Hz, 3H).

¹³C NMR (100 MHz, CDCl₃) δ 176.44, 169.94, 166.21, 163.50, 131.96, 131.96, 122.72, 113.82, 113.82, 85.66, 84.64, 83.31, 81.74, 80.45, 80.05, 77.10, 61.38, 59.18, 58.13, 57.89, 55.98, 55.52, 53.95, 50.31, 49.14, 48.98, 48.87, 43.77, 41.86, 39.66, 39.12, 35.52, 34.90, 34.01, 26.32, 21.77, 19.12, 19.12, 13.51.

### Example 83 Preparation of compound 83

100 mg of compound 2 was added into a 5 mL round-bottom flask and dissolved with 2 mL of dry pyridine. Under an argon atmosphere, the reaction mixture was added with 2 equivalents of 4-chlorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 57.8 mg of a light yellow solid as a target compound 83 (C₄₂H₅₄ClNO₁₀, 47.4% yield), which was structurally shown as follows:

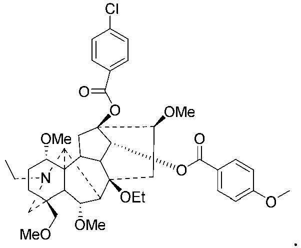

The target compound 83 was characterized as follows.

HRESIMS *m*/*z :* [(M + H)⁺, 768.34]

¹H NMR (400 MHz, Chloroform-d) δ 8.07 (d, J = 8.6 Hz, 2H), 7.92 (d, J = 8.6 Hz, 2H), 7.34 (d, J = 8.6 Hz, 2H), 6.91 (d, J = 8.8 Hz, 2H), 5.25 (d, J = 5.2 Hz, 1H), 4.12 (dd, J = 8.4, 6.4 Hz, 1H), 4.03 (dd, J = 6.4, 1.4 Hz, 1H), 3.85 (s, 3H), 3.64 (d, J = 8.2 Hz, 1H), 3.53 - 3.45 (m, 1H), 3.42 - 3.34 (m, 1H), 3.30 (s, 3H), 3.30 (s, 3H), 3.27 (s, 3H), 3.24 (s, 3H), 3.20 (dt, J = 8.2, 6.8 Hz, 1H), 3.08 (d, J = 8.2 Hz, 1H), 3.03 (dd, J = 9.6, 6.2 Hz, 1H), 2.89 (s, 1H), 2.66 (dt, J = 7.2, 3.4 Hz, 1H), 2.54 (dd, J = 7.2, 4.8 Hz, 1H), 2.51 (d, J = 1.6 Hz, 1H), 2.49 (d, J = 5.4 Hz, 1H), 2.45 (dd, J = 4.8, 2.2 Hz, 1H), 2.40 (s, 1H), 2.36 (dd, J = 7.6, 5.4 Hz, 2H), 2.34 - 2.28 (m, 1H), 2.10 (s, 1H), 2.08 (dd, J = 4.2, 1.6 Hz, 1H), 1.90 (dd, J = 11.4, 5.6 Hz, 1H), 1.68 (dd, J = 13.2, 4.2 Hz, 2H), 1.64 - 1.61 (m, 1H), 1.10 (t, J = 7.0 Hz, 3H), 0.65 (t, J = 7.0 Hz, 3H).

¹³C NMR (100 MHz, CDCl₃) δ 166.46, 165.00, 163.31, 139.06, 132.10, 132.10, 131.38, 131.38, 129.50, 128.61, 128.61, 123.29, 113.58, 113.58, 85.21, 83.71, 83.27, 80.50, 80.28, 78.13, 77.48, 61.05, 59.18, 58.87, 58.14, 56.27, 56.06, 55.52, 54.07, 50.86, 49.05, 49.00, 48.18, 45.24, 42.26, 39.14, 37.65, 36.22, 35.07, 26.45, 15.49, 13.66.

### Example 84 Preparation of compound 84

100 mg of bulleyaconitine A was added into a 5 mL round-bottom flask and dissolved with 2 mL of dry pyridine. Under an argon atmosphere, the reaction mixture was added with 2 equivalents of 4-chlorobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 82.1 mg of a light yellow solid as a target compound 84 (C₄₂H₅₂ClNO₁₁, 67.8% yield), which was structurally shown as follows:

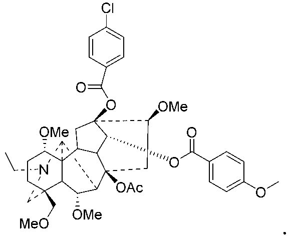

The target compound 84 was characterized as follows.

HRESIMS *m*/*z :* [(M + H)⁺, 782.32]

¹H NMR (400 MHz, Chloroform-d) δ 8.04 (d, *J=* 8.8 Hz, 2H), 7.90 (d, *J* = 8.6 Hz, 2H), 7.32 (d, *J* = 8.6 Hz, 2H), 6.90 (d, *J* = 8.8 Hz, 2H), 5.26 (d, *J* = 5.2 Hz, 1H), 4.11 (dd, *J* = 8.8, 5.8 Hz, 1H), 3.98 (d, *J* = 6.8 Hz, 1H), 3.83 (s, 3H), 3.61 (d, *J* = 8.4 Hz, 1H), 3.57 - 3.50 (m, 1H), 3.29 (s, 3H), 3.26 (s, 3H), 3.23 (s, 3H), 3.15 (s, 3H), 3.12 (s, 1H), 3.02 (dd, *J* = 10.4, 6.8 Hz, 4H), 2.88 (dd, *J* = 7.2, 5.2 Hz, 1H), 2.60 - 2.54 (m, 1H), 2.54 - 2.50 (m, 1H), 2.48 (d, *J* = 3.8 Hz, 1H), 2.46 (q, *J* = 3.2, 2.2 Hz, 1H), 2.45 - 2.40 (m, 1H), 2.33 - 2.23 (m, 1H), 2.15 - 2.10 (m, 2H), 2.08 (d, *J* = 7.0 Hz, 1H), 1.95 - 1.88 (m, 1H), 1.61 (tq, *J* = 8.2, 4.8, 4.2 Hz, 2H), 1.34 (s, 3H), 1.09 (t, *J* = 7.2 Hz, 3H).

¹³C NMR (100 MHz, CDCl₃) δ 169.90, 166.24, 164.82, 163.58, 139.10, 132.01, 132.01, 131.29, 131.29, 129.27, 128.58, 128.58, 122.54, 113.86, 113.86, 85.53, 84.78, 83.31, 83.06, 80.42, 80.22, 77.05, 61.62, 59.17, 58.26, 57.92, 56.08, 55.52, 53.83, 53.54, 50.35, 49.26, 49.11, 43.96, 41.83, 39.72, 39.16, 35.61, 35.01, 26.36, 21.80, 13.57.

### Example 85 Preparation of compound 85s

100 mg of compound 4 was added into a 5 mL round-bottom flask and dissolved with 2 mL of dry pyridine. Under an argon atmosphere, the reaction mixture was added with 2 equivalents of crotonoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 68.8 mg of a light yellow solid as a target compound 85 (,C₃₅H₅₃NO₉ 54.5% yield), which was structurally shown as follows:

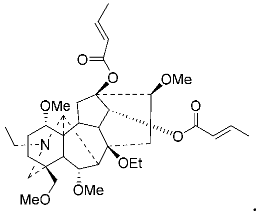

The target compound 85 was characterized as follows.

HRESIMS *m*/*z :* [(M + H)⁺, 632.37]

¹H NMR (400 MHz, Chloroform-d) δ 6.96 - 6.88 (m, 2H), 5.88 (dt, *J* = 15.4, 2.2 Hz, 2H), 5.85 - 5.79 (m, 1H), 5.30 (d, *J* = 12.4 Hz, 1H), 4.92 (t, *J* = 5.2 Hz, 1H), 4.04 - 3.92 (m, 2H), 3.60 (d, *J* = 8.2 Hz, 1H), 3.46 - 3.39 (m, 1H), 3.32 (s, 3H), 3.28 (s, 3H), 3.27 (s, 3H), 3.23 (s, 3H), 3.16 - 3.09 (m, 1H), 3.09 - 3.02 (m, 2H), 2.90 (d, *J* = 5.0 Hz, 1H), 2.75 (d, *J* = 10.4 Hz, 1H), 2.70 - 2.61 (m, 2H), 2.52 (t, *J* = 6.2 Hz, 1H), 2.38 (d, *J* = 1.8 Hz, 1H), 2.25 (d, *J* = 7.8 Hz, 2H), 2.14 (td, *J* = 4.8, 2.2 Hz, 2H), 2.07 - 1.98 (m, 2H), 1.98 - 1.91 (m, 1H), 1.86 (d, *J* = 1.8 Hz, 1H), 1.85 (d, *J* = 1.8 Hz, 2H), 1.84 - 1.83 (m, 2H), 1.82 (s, 1H), 1.69 (s, 2H), 1.15 (t, *J* = 7.2 Hz, 3H), 1.00 (t, *J* = 6.8 Hz, 3H).

¹³C NMR (100 MHz, CDCl₃) δ 166.57, 165.80, 144.77, 144.77, 123.23, 123.17, 83.82, 82.98, 81.89, 80.59, 79.57, 78.19, 78.16, 78.15, 60.85, 59.16, 58.86, 58.28, 56.21, 56.08, 54.76, 50.87, 49.01, 48.87, 46.02, 44.35, 41.74, 38.77, 37.56, 36.19, 25.26, 18.03, 18.03, 15.90, 12.65.

### Example 86 Preparation of compound 86

100 mg of compound 2 was added into a 5 mL round-bottom flask and dissolved with 2 mL of dry pyridine. Under an argon atmosphere, the reaction mixture was added with 2 equivalents of crotonoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 91.5 mg of a light yellow solid as a target compound 86 (C₃₉H₅₅NO₁₀, 82.6% yield), which was structurally shown as follows:

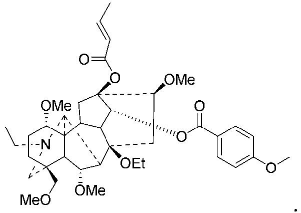

The target compound 86 was characterized as follows.

HRESIMS *m*/*z :* [(M + H)⁺, 698.38]

¹H NMR (400 MHz, Chloroform-d) δ 8.04 (dd, *J* = 9.0, 2.2 Hz, 2H), 6.89 (d, *J* = 8.8 Hz, 3H), 5.90 - 5.76 (m, 1H), 5.08 (dd, *J* = 8.2, 5.2 Hz, 1H), 4.06 - 3.98 (m, 2H), 3.84 (s, 3H), 3.61 (d, *J* = 8.2 Hz, 1H), 3.37 (s, 1H), 3.35 (s, 3H), 3.34 - 3.30 (m, 1H), 3.28 (s, 3H), 3.24 (s, 3H), 3.23 (s, 3H), 3.19 - 3.10 (m, 1H), 3.07 (d, *J* = 8.6 Hz, 1H), 3.05 - 3.00 (m, 1H), 2.87 (s, 1H), 2.61 (t, *J* = 6.2 Hz, 1H), 2.59 - 2.51 (m, 4H), 2.35 (d, *J* = 7.8 Hz, 2H), 2.32 (d, *J* = 2.6 Hz, 1H), 2.29 (s, 1H), 2.28 - 2.22 (m, 1H), 2.09 (d, *J* = 6.4 Hz, 1H), 2.03 (ddd, *J* = 6.2, 4.2, 1.8 Hz, 2H), 1.86 - 1.83 (m, 1H), 1.81 (dd, *J* = 6.8, 1.6 Hz, 3H), 1.64 (dd, *J* = 9.0, 4.8 Hz, 2H), 1.10 (t, *J* = 7.2 Hz, 3H), 0.57 (t, *J* = 6.8 Hz, 3H).

¹³C NMR (100 MHz, CDCl₃) δ 166.42, 165.83, 163.23, 144.43, 132.10, 132.04, 123.42, 123.28, 113.50, 113.50, 84.79, 83.14, 82.49, 80.67, 80.06, 78.08, 77.55, 61.03, 59.15, 58.83, 58.25, 56.20, 56.00, 55.49, 54.24, 50.83, 49.00, 48.96, 47.52, 44.92, 42.09, 39.02, 37.60, 36.23, 34.31, 26.06, 18.02, 15.34, 13.33.

### Example 87 Preparation of compound 87

100 mg of bulleyaconitine A was added into a 5 mL round-bottom flask and dissolved with 2 mL of dry pyridine. Under an argon atmosphere, the reaction mixture was added with 2 equivalents of crotonoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 54.6 mg of a light yellow solid as a target compound 87 (C₃₉H₅₃NO₁₁, 49.5% yield), which was structurally shown as follows:

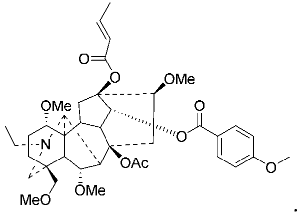

The target compound 87 was characterized as follows.

HRESIMS *m*/*z :* [(M + H)⁺, 712.36]

¹H NMR (400 MHz, Chloroform-d) δ 8.03 (d, *J* = 9.2 Hz, 2H), 6.96 - 6.86 (m, 3H), 5.88 - 5.79 (m, 2H), 5.13 (d, *J* = 5.2 Hz, 1H), 4.03 (dd, *J* = 8.8, 5.8 Hz, 1H), 3.96 (d, *J= 6.8* Hz, 1H), 3.84 (s, 3H), 3.61 (d, *J* = 8.4 Hz, 1H), 3.35 (s, 3H), 3.27 (s, 3H), 3.24 (s, 3H), 3.14 (s, 3H), 3.12 (s, 1H), 3.05 - 3.02 (m, 1H), 3.02 - 2.98 (m, 3H), 2.84 (dd, *J* = 7.2, 5.4 Hz, 1H), 2.54 (s, 3H), 2.46 (dd, *J* = 15.8, 5.8 Hz, 1H), 2.29 - 2.19 (m, 1H), 2.12 (d, *J* = 6.7 Hz, 1H), 2.04 - 1.96 (m, 1H), 1.88 (dq, *J* = 6.8, 1.6 Hz, 3H), 1.81 (dd, *J* = 6.8, 1.6 Hz, 3H), 1.68 - 1.62 (m, 1H), 1.62 - 1.54 (m, 1H), 1.30 (s, 3H), 1.11 (t, *J* = 7.2 Hz, 3H).

¹³C NMR (100 MHz, CDCl₃) δ 170.02, 166.30, 165.75, 163.59, 144.74, 132.10, 132.10, 123.21, 122.57, 113.86, 113.86, 85.50, 84.47, 83.24, 81.96, 80.38, 80.28, 77.16, 61.63, 59.22, 58.42, 57.98, 56.11, 55.56, 54.05, 50.40, 49.21, 48.97, 48.65, 43.77, 41.72, 39.75, 39.11, 35.69, 34.36, 26.03, 21.76, 18.04, 13.30.

### Example 88 Preparation of compound 88

100 mg of compound 4 was added into a 5 mL round-bottom flask and dissolved with 2 mL of dry pyridine. Under an argon atmosphere, the reaction mixture was added with 2 equivalents of 3,3-dimethylbutyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 75.5 mg of a light yellow solid as a target compound 88 (C₃₉H₆₅NO₉, 54.6% yield), which was structurally shown as follows:

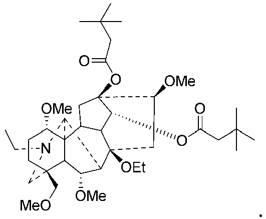

The target compound 88 was characterized as follows.

HRESIMS *m*/*z :* [(M + H)⁺, 692.46]

¹H NMR (400 MHz, Chloroform-d) δ 4.85 (d, *J* = 5.2 Hz, 1H), 3.98 (dd, *J* = 6.4, 1.8 Hz, 1H), 3.89 (t, *J* = 7.8 Hz, 1H), 3.63 (d, *J=* 8.2 Hz, 1H), 3.50 - 3.43 (m, 1H), 3.37 - 3.31 (m, 2H), 3.29 (s, 6H), 3.26 (s, 3H), 3.20 (s, 3H), 3.06 (d, *J= 8.2* Hz, 1H), 2.98 (dd, *J* = 9.4, 6.2 Hz, 1H), 2.77 (s, 1H), 2.52 - 2.48 (m, 1H), 2.47 - 2.43 (m, 2H), 2.40 (d, *J* = 4.6 Hz, 1H), 2.36 (d, *J* = 1.6 Hz, 1H), 2.29 (s, 1H), 2.25 (s, 1H), 2.20 (t, *J* = 8.2 Hz, 2H), 2.17 (s, 1H), 2.15 - 2.13 (m, 2H), 2.04 (d, *J* = 6.4 Hz, 1H), 1.93 (dd, *J* = 7.2, 5.2 Hz, 1H), 1.88 - 1.79 (m, 2H), 1.76 - 1.71 (m, 1H), 1.65 - 1.57 (m, 2H), 1.09 (t, J = 6.8 Hz, 3H), 1.06 (t, J = 7.2 Hz, 3H), 1.03 (s, 9H), 1.01 (s, 9H).

¹³C NMR (100 MHz, CDCl₃) δ 172.52, 171.62, 85.01, 83.43, 82.20, 80.26, 80.13, 78.20, 77.03, 60.63, 59.15, 58.84, 57.55, 56.13, 56.06, 54.20, 50.83, 48.85, 48.74, 48.42, 48.12, 47.80, 44.88, 42.20, 39.03, 37.44, 36.37, 34.87, 30.89, 30.49, 29.77, 29.77, 29.77, 29.74, 29.74, 29.74, 26.43, 16.35, 13.58.

### Example 89 Preparation of compound 89

100 mg of compound 2 was added into a 5 mL round-bottom flask and dissolved with 2 mL of dry pyridine. Under an argon atmosphere, the reaction mixture was added with 2 equivalents of 3,3-dimethylbutyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 83.1 mg of a light yellow solid as a target compound 89 (C₄₁H₆₁NO₁₀, 71.9% yield), which was structurally shown as follows:

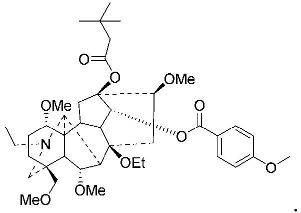

The target compound 89 was characterized as follows.

HRESIMS *m*/*z* : [(M + H)⁺, 728.42]

¹H NMR (400 MHz, Chloroform-*d*) δ 8.03 (d, *J* = 8.8 Hz, 2H), 6.90 (d, *J* = 8.8 Hz, 2H), 5.02 (d, *J* = 5.2 Hz, 1H), 4.06 - 3.97 (m, 2H), 3.85 (s, 3H), 3.62 (d, *J= 8.2* Hz, 1H), 3.36 (s, 3H), 3.32 (dd, *J= 8.2,* 6.4 Hz, 2H), 3.28 (s, 3H), 3.25 (s, 3H), 3.22 (s, 3H), 3.13 (dd, *J* = 8.2, 6.8 Hz, 1H), 3.07 (d, *J* = 8.2 Hz, 1H), 3.00 (dd, *J* = 9.6, 6.2 Hz, 1H), 2.82 (s, 1H), 2.62 (dd, *J* = 7.0, 5.4 Hz, 1H), 2.53 - 2.47 (m, 2H), 2.44 (dd, *J* = 11.4, 4.8 Hz, 2H), 2.34 (d, *J* = 1.6 Hz, 1H), 2.29 (dd, *J* = 7.4, 4.8 Hz, 2H), 2.16 (s, 2H), 2.05 (d, *J* = 6.6 Hz, 1H), 2.03 - 1.99 (m, 1H), 1.97 - 1.84 (m, 2H), 1.81 (d, *J* = 9.6 Hz, 1H), 1.61 (q, *J* = 6.0, 3.8 Hz, 2H), 1.07 (t, *J= 7.2* Hz, 3H), 1.00 (s, 9H), 0.56 (t, *J* = 6.8 Hz, 3H).

¹³C NMR (100 MHz, CDCl₃) δ 171.64, 166.28, 163.07, 131.90, 131.90, 123.32, 113.38, 113.38, 85.15, 83.11, 82.58, 80.30, 80.17, 77.92, 77.57, 60.90, 59.05, 58.72, 57.66, 56.07, 55.83, 55.38, 53.96, 50.70, 48.94, 48.85, 48.28, 48.06, 44.91, 42.15, 39.00, 37.44, 36.14, 34.95, 30.84, 29.55, 29.55, 29.55, 26.34, 15.31, 13.52.

### Example 90 Preparation of compound 90

100 mg of bulleyaconitine A was added into a 5 mL round-bottom flask and dissolved with 2 mL of dry pyridine. Under an argon atmosphere, the reaction mixture was added with 2 equivalents of 3,3-dimethylbutyryl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 71.1 mg of a light yellow solid as a target compound 90 (C₄₁H₅₉NO₁₁, 61.9% yield), which was structurally shown as follows:

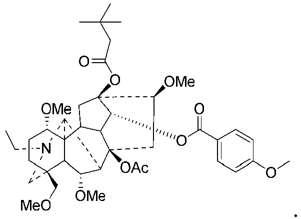

The target compound 90 was characterized as follows.

HRESIMS m/z : [(M + H)⁺, 742.40]

¹H NMR (400 MHz, Chloroform-d) δ 8.02 (d, *J* = 8.8 Hz, 2H), 6.90 (d, *J* = 8.8 Hz, 2H), 5.06 (d, *J* = 5.2 Hz, 1H), 4.02 (dd, *J* = 8.8, 5.6 Hz, 1H), 3.95 (d, *J* = 6.8 Hz, 1H), 3.85 (d, *J* = 0.8 Hz, 3H), 3.61 (d, *J* = 8.4 Hz, 1H), 3.40 (dd, *J* = 15.4, 5.8 Hz, 1H), 3.36 (s, 3H), 3.27 (s, 3H), 3.22 (s, 3H), 3.14 (s, 3H), 3.02 - 2.98 (m, 2H), 2.96 (q, *J* = 5.0, 4.2 Hz, 2H), 2.86 - 2.81 (m, 1H), 2.59 - 2.52 (m, 1H), 2.49 (d, *J* = 11.2 Hz, 1H), 2.45 (q, *J* = 2.4 Hz, 2H), 2.43 - 2.39 (m, 1H), 2.28 (ddt, *J =* 12.2, 6.0, 3.0 Hz, 1H), 2.17 (s, 2H), 2.07 (q, *J* = 8.0, 6.2 Hz, 2H), 1.98 - 1.89 (m, 2H), 1.79 - 1.71 (m, 1H), 1.60 (dt, *J* = 13.2, 3.8 Hz, 2H), 1.31 (s, 3H), 1.08 (t, *J* = 7.0 Hz, 3H), 1.00 (s, 9H).

¹³C NMR (100 MHz, CDCl₃) δ 171.65, 169.99, 166.27, 163.53, 132.01, 132.01, 122.71, 113.84, 113.84, 85.61, 84.90, 83.30, 82.15, 80.49, 80.13, 77.25, 61.62, 59.22, 57.93, 57.93, 56.07, 55.56, 53.89, 50.35, 49.28, 49.13, 49.04, 48.32, 43.87, 41.89, 39.70, 39.19, 35.69, 35.05, 30.96, 29.64, 29.64, 29.64, 26.39, 21.82, 13.59.

### Example 91 Preparation of compound 91

100 mg of compound 2 was added into a 5 mL round-bottom flask and dissolved with 2 mL of dry pyridine. Under an argon atmosphere, the reaction mixture was added with 2 equivalents of 4-bromobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 81.3 mg of a light yellow solid as a target compound 91 (C₄₂H₅₄BrNO₁₀, 63.1% yield), which was structurally shown as follows:

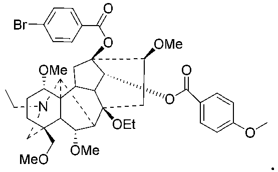

The target compound 91 was characterized as follows.

HRESIMS *m*/*z :* [(M + H)⁺, 812.29]

¹H NMR (400 MHz, Chloroform-*d*) δ 8.07 (d, *J =* 8.8 Hz, 2H), 7.85 (d, *J* = 8.6 Hz, 2H), 7.50 (d, *J* = 8.4 Hz, 2H), 6.91 (d, *J* = 9.0 Hz, 2H), 5.25 (d, *J* = 5.2 Hz, 1H), 4.12 (dd, *J* = 8.6, 6.8 Hz, 1H), 4.06 - 4.01 (m, 1H), 3.85 (s, 3H), 3.64 (d, *J* = 8.2 Hz, 1H), 3.53 - 3.44 (m, 1H), 3.41 - 3.36 (m, 1H), 3.30 (s, 6H), 3.28 (s, 3H), 3.24 (s, 3H), 3.21 (dd, *J* = 8.2, 6.8 Hz, 1H), 3.09 (d, *J* = 8.2 Hz, 1H), 3.03 (dd, *J* = 9.6, 6.2 Hz, 1H), 2.89 (s, 1H), 2.66 (dt, *J* = 6.4, 3.2 Hz, 1H), 2.53 (dq, *J* = 9.8, 6.8, 5.8 Hz, 2H), 2.50 - 2.44 (m, 2H), 2.43 - 2.35 (m, 3H), 2.35 - 2.31 (m, 1H), 2.12 - 2.06 (m, 3H), 1.91 (dq, *J =* 10.8, 4.8 Hz, 1H), 1.63 (s, 3H), 1.11 (t, *J* = 7.2 Hz, 3H), 0.65 (t, *J* = 6.8 Hz, 3H).

¹³C NMR (100 MHz, CDCl₃) δ 166.46, 165.15, 163.34, 132.11, 132.11, 131.62, 131.62, 131.55, 131.55, 130.01, 127.75, 123.35, 113.60, 113.60, 85.23, 83.79, 83.31, 80.51, 80.31, 78.16, 77.36, 61.07, 59.19, 58.88, 58.14, 56.25, 56.07, 55.53, 54.09, 50.90, 49.11, 49.01, 48.28, 45.28, 42.31, 39.18, 37.64, 36.23, 35.09, 26.48, 15.50, 13.66.

### Example 92 Preparation of compound 92

100 mg of bulleyaconitine A was added into a 5 mL round-bottom flask and dissolved with 2 mL of dry pyridine. Under an argon atmosphere, the reaction mixture was added with 2 equivalents of 4-bromobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 89.3 mg of a light yellow solid as a target compound 92 (C₄₂H₅₂BrNO₁₁, 69.8% yield), which was structurally shown as follows:

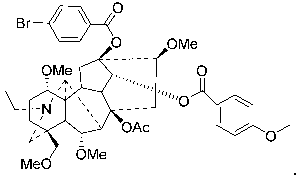

The target compound 92 was characterized as follows.

HRESIMS *m*/*z :* [(M + H)⁺, 826.27]

¹H NMR (400 MHz, Chloroform-*d*) δ 8.06 (d, *J* = 8.8 Hz, 2H), 7.84 (d, *J* = 8.6 Hz, 2H), 7.50 (d, *J* = 8.6 Hz, 2H), 6.92 (d, *J* = 8.8 Hz, 2H), 5.27 (d, *J* = 5.2 Hz, 1H), 4.12 (dd, *J* = 9.0, 5.8 Hz, 1H), 3.99 (d, *J* = 6.8 Hz, 1H), 3.85 (s, 3H), 3.63 (d, *J* = 8.4 Hz, 1H), 3.58 - 3.52 (m, 1H), 3.31 (s, 3H), 3.28 (s, 3H), 3.25 (s, 3H), 3.17 (s, 3H), 3.11 (d, *J* = 23.2 Hz, 1H), 3.05 - 3.01 (m, 3H), 2.90 (td, *J* = 5.8, 5.4, 1.8 Hz, 1H), 2.63 - 2.57 (m, 1H), 2.57 - 2.53 (m, 1H), 2.51 (t, *J* = 5.0 Hz, 1H), 2.49 - 2.47 (m, 1H), 2.45 (t, *J* = 3.4 Hz, 1H), 2.30 (ddd, *J* = 12.6, 6.2, 3.4 Hz, 1H), 2.15 - 2.11 (m, 2H), 2.11 - 2.04 (m, 1H), 1.94 (qd, *J* = 7.4, 6.6, 3.2 Hz, 1H), 1.76 (dd, *J* = 8.8, 4.0 Hz, 1H), 1.62 (qd, *J* = 7.6, 6.2, 2.4 Hz, 2H), 1.37 (s, 3H), 1.11 (t, *J= 7.2* Hz, 3H).

¹³C NMR (100 MHz, CDCl₃) δ 169.95, 166.29, 165.02, 163.65, 132.06, 132.06, 131.63, 131.63, 131.49, 131.49, 129.80, 127.84, 122.61, 113.91, 113.91, 85.59, 84.83, 83.37, 83.15, 80.49, 80.26, 77.10, 61.67, 59.22, 58.30, 57.96, 56.11, 55.56, 53.88, 50.41, 49.34, 49.15, 49.08, 44.02, 41.90, 39.76, 39.22, 35.65, 35.06, 26.40, 21.85, 13.61.

### Example 93 Preparation of compound 93

100 mg of compound 4 was added into a 5 mL round-bottom flask and dissolved with 2 mL of dry pyridine. Under an argon atmosphere, the reaction mixture was added with 2 equivalents of 2-thiopheneacetyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 93.8 mg of a light yellow solid as a target compound 93 (C₃₉H₅₃NO₉S₂, 63.1% yield), which was structurally shown as follows:

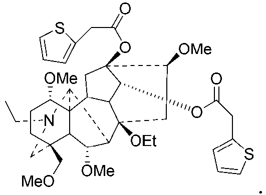

The target compound 93 was characterized as follows.

HRESIMS *m*/*z :* [(M + H)⁺, 744.31]

¹H NMR (400 MHz, Chloroform-*d*) δ 7.22 (dd, *J* = 5.2, 1.2 Hz, 1H), 7.19 (dd, *J=* 3.8, 2.6 Hz, 1H), 7.01 (dt, *J* = 3.2, 1.2 Hz, 1H), 6.97 (dd, *J* = 5.2, 3.4 Hz, 1H), 6.94 (d, *J* = 1.2 Hz, 1H), 6.93 (s, 1H), 4.93 (d, *J* = 5.2 Hz, 1H), 4.02 - 3.96 (m, 2H), 3.92 (t, *J* = 7.8 Hz, 1H), 3.83 (s, 2H), 3.63 (d, *J* = 8.2 Hz, 1H), 3.51 - 3.46 (m, 1H), 3.40 - 3.32 (m, 2H), 3.30 (s, 6H), 3.26 (s, 3H), 3.18 (s, 3H), 3.06 (d, *J* = 8.2 Hz, 1H), 2.97 (dd, *J* = 9.4, 6.2 Hz, 1H), 2.77 (s, 1H), 2.54 - 2.52 (m, 1H), 2.50 (q, *J* = 3.2, 2.6 Hz, 1H), 2.47 (d, *J* = 7.2 Hz, 1H), 2.46 - 2.41 (m, 2H), 2.38 (d, *J* = 1.6 Hz, 1H), 2.24 (dd, *J* = 11.8, 7.8 Hz, 2H), 2.05 (d, *J* = 6.4 Hz, 1H), 1.95 (dd, *J* = 7.2, 4.8 Hz, 1H), 1.87 (dd, *J =* 17.8, 11.8 Hz, 2H), 1.73 - 1.68 (m, 1H), 1.68 - 1.56 (m, 3H), 1.10 (t, J = 6.8 Hz, 3H), 1.06 (t, J = 7.2 Hz, 3H).

¹³C NMR (100 MHz, CDCl₃) δ 170.61, 169.69, 135.68, 135.06, 127.15, 126.82, 126.80, 126.74, 124.95, 124.93, 84.92, 83.35, 83.06, 80.21, 80.21, 78.34, 77.94, 60.61, 59.14, 58.90, 57.99, 56.20, 56.11, 54.15, 50.80, 48.82, 48.78, 47.93, 44.82, 41.96, 39.06, 37.48, 36.04, 35.69, 35.22, 34.92, 26.42, 16.40, 13.55.

### Example 94 Preparation of compound 94

100 mg of compound 4 was added into a 5 mL round-bottom flask and dissolved with 2 mL of dry pyridine. Under an argon atmosphere, the reaction mixture was added with 2 equivalents of 4-(trifluoromethoxy)benzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 119.0 mg of a light yellow solid as a target compound 94 (C₄₃H₅₁F₆NO₁₁, 68.3% yield), which was structurally shown as follows:

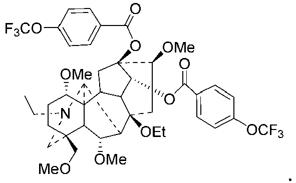

The target compound 94 was characterized as follows.

HRESIMS *m*/*z :* [(M + H)⁺, 872.33]

¹H NMR (400 MHz, Chloroform-*d*) δ 8.17 (d, *J* = 8.8 Hz, 2H), 8.03 (d, *J* = 8.8 Hz, 2H), 7.25 (d, *J* = 8.8 Hz, 2H), 7.19 (d, *J* = 7.4 Hz, 2H), 5.27 (d, *J* = 5.2 Hz, 1H), 4.15 (t, *J* = 7.4 Hz, 1H), 4.04 - 3.99 (m, 1H), 3.65 (d, *J* = 8.2 Hz, 1H), 3.51 - 3.46 (m, 1H), 3.39 (dd, *J* = 8.2, 6.8 Hz, 1H), 3.31 (s, 3H), 3.29 (s, 3H), 3.27 (s, 3H), 3.24 (s, 3H), 3.21 - 3.16 (m, 1H), 3.07 (d, *J* = 8.2 Hz, 1H), 3.05 - 3.00 (m, 1H), 2.90 (s, 1H), 2.70 - 2.65 (m, 1H), 2.55 (dd, *J* = 4.4, 2.8 Hz, 1H), 2.52 (dd, *J* = 4.4, 2.8 Hz, 1H), 2.48 (d, *J* = 3.4 Hz, 1H), 2.45 (d, *J* = 4.8 Hz, 1H), 2.40 (s, 1H), 2.36 (dd, *J* = 7.6, 3.6 Hz, 2H), 2.34 - 2.28 (m, 1H), 2.13 - 2.07 (m, 3H), 1.94 - 1.88 (m, 1H), 1.69 - 1.59 (m, 2H), 1.10 (t, *J =* 7.2 Hz, 3H), 0.61 (t, *J* = 6.8 Hz, 3H).

¹³C NMR (100 MHz, CDCl₃) δ 165.42, 164.61, 152.64, 152.55, 132.05, 132.05, 132.05, 131.89, 131.89, 131.89, 129.40, 129.26, 120.29, 120.29, 120.29, 120.29, 85.25, 83.81, 83.22, 80.42, 80.25, 78.16, 77.99, 61.13, 59.12, 58.90, 58.18, 56.18, 56.09, 53.98, 50.87, 49.18, 49.02, 48.32, 45.27, 42.26, 39.18, 37.67, 36.20, 35.20, 26.45, 15.35, 13.64.

### Example 95 Preparation of compound 95

100 mg of compound 4 was added into a 5 mL round-bottom flask and dissolved with 2 mL of dry pyridine. Under an argon atmosphere, the reaction mixture was added with 2 equivalents of 3-cyanobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 111.0 mg of a light yellow solid as a target compound 95 (C₄₃H₅₁N₃O₉, 73.7% yield), which was structurally shown as follows:

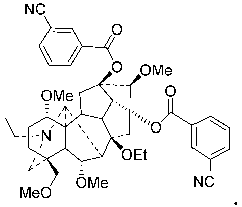

The target compound 95 was characterized as follows.

HRESIMS *m*/*z :* [(M + H)⁺, 754.36]

¹H NMR (400 MHz, Chloroform-*d*) δ 8.43 (t, *J* = 1.6 Hz, 1H), 8.35 (dt, *J* = 8.0, 1.4 Hz, 1H), 8.27 (t, *J* = 1.6 Hz, 1H), 8.21 (dt, *J* = 8.0, 1.4 Hz, 1H), 7.80 (ddt, *J* = 16.2, 7.8, 1.4 Hz, 2H), 7.58 (t, *J =* 7.8 Hz, 1H), 7.52 (t, *J* = 7.8 Hz, 1H), 5.30 (d, *J* = 5.2 Hz, 1H), 4.17 (dd, *J* = 8.8, 5.8 Hz, 1H), 4.00 (dd, *J* = 6.6, 1.4 Hz, 1H), 3.66 (d, *J* = 8.2 Hz, 1H), 3.52 - 3.45 (m, 1H), 3.41 (dd, *J* = 8.4, 6.8 Hz, 1H), 3.30 (s, 3H), 3.29 (s, 3H), 3.27 (s, 3H), 3.23 (s, 3H), 3.22 - 3.16 (m, 1H), 3.07 - 3.00 (m, 2H), 2.90 (s, 1H), 2.68 (dt, *J* = 7.0, 3.4 Hz, 1H), 2.59 - 2.53 (m, 1H), 2.53 - 2.48 (m, 2H), 2.46 (dt, *J* = 7.2, 3.4 Hz, 1H), 2.44 - 2.38 (m, 2H), 2.38 - 2.35 (m, 1H), 2.33 (d, *J* = 5.7 Hz, 1H), 2.15 - 2.08 (m, 3H), 1.97 - 1.88 (m, 1H), 1.72 - 1.56 (m, 2H), 1.10 (t, *J* = 7.2 Hz, 3H), 0.64 (t, *J* = 6.8 Hz, 3H).

¹³C NMR (100 MHz, CDCl₃) δ 164.53, 163.73, 135.96, 135.87, 134.12, 133.97, 133.92, 133.65, 132.09, 132.04, 129.47, 129.40, 118.20, 118.11, 112.87, 112.85, 85.26, 84.37, 83.09, 80.15, 80.03, 78.13, 78.13, 61.35, 59.13, 58.96, 58.08, 56.18, 56.18, 53.78, 50.82, 49.22, 49.11, 48.32, 45.31, 42.15, 39.16, 37.57, 36.06, 35.29, 26.40, 15.53, 13.65.

### Example 96 Preparation of compound 96

100 mg of compound 4 was added into a 5 mL round-bottom flask and dissolved with 2 mL of dry pyridine. Under an argon atmosphere, the reaction mixture was added with 2 equivalents of 3,5-dinitrobenzoyl chloride in an ice water bath, and reacted at room temperature (about 20 °C), where the reaction was monitored through thin-layer chromatography. After reacted for about 12 h, the reaction mixture, including a target product and a by-product, was subjected to rotary evaporation, and purified by column chromatography to obtain 114.1 mg of a light yellow solid as a target compound 96 (C₄₁H₄₉N₅O₁₇, 64.6% yield), which was structurally shown as follows:

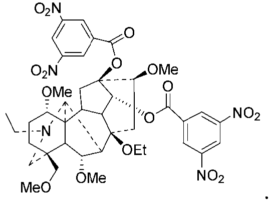

The target compound 96 was characterized as follows.

HRESIMS *m*/*z :* [(M + H)⁺, 884.31]

¹H NMR (400 MHz, Chloroform-*d*) δ 9.32 (d, *J* = 2.2 Hz, 2H), 9.22 (t, *J* = 2.2 Hz, 1H), 9.17 (t, *J* = 2.2 Hz, 1H), 9.09 (d, *J* = 2.2 Hz, 2H), 5.44 (d, *J* = 5.2 Hz, 1H), 4.25 (dd, *J* = 9.0, 4.8 Hz, 1H), 4.02 (d, *J* = 6.6 Hz, 1H), 3.69 (d, *J* = 8.2 Hz, 1H), 3.49 - 3.41 (m, 2H), 3.34 (s, 3H), 3.31 (s, 3H), 3.30 (s, 3H), 3.26 (s, 3H), 3.10 - 3.03 (m, 2H), 2.95 (s, 1H), 2.76 (dt, *J* = 5.8, 3.2 Hz, 1H), 2.62 - 2.56 (m, 1H), 2.54 (dd, *J* = 6.4, 4.6 Hz, 2H), 2.51 - 2.43 (m, 3H), 2.40 (dd, *J* = 15.2, 5.0 Hz, 2H), 2.19 (dd, *J* = 8.0, 5.0 Hz, 3H), 1.71 (dq, *J* = 13.8, 6.8, 4.6 Hz, 2H), 1.61 (ddd, *J* = 15.6, 6.2, 2.8 Hz, 2H), 1.12 (t, *J* = 7.2 Hz, 3H), 0.65 (t, *J=* 6.8 Hz, 3H).

¹³C NMR (100 MHz, CDCl₃) δ 162.28, 161.55, 148.66, 148.66, 148.60, 148.60, 134.37, 134.37, 129.94, 129.94, 129.68, 129.68, 122.30, 122.25, 85.87, 85.29, 82.89, 79.99, 79.34, 78.51, 78.04, 61.64, 59.05, 59.00, 57.78, 56.25, 56.08, 53.48, 50.76, 49.36, 49.16, 48.40, 45.38, 42.15, 39.15, 37.12, 35.58, 35.43, 22.67, 15.68, 13.61.

Beneficial effects of the present disclosure were illustrated through the following experimental examples.

### Experimental example 1 Analgesic activity and toxicity test of compounds in the present disclosure

### 1. Experimental method

### Experimental animals

The experimental animals were Kunming mice (male: female = 1:1), weighed 22±2 g, which were provided by Chengdu dossy experimental animals Co., ltd., China. The mice were kept in the animal room of the School of Life Science and Engineering, Southwest Jiaotong University, where the animal room was set at a temperature of 23±2 °C, a humidity of 55 ± 5% and a photoperiod of 16:8 h (L:D). Before the experiment, the mice were freely fed for 2-3 days with water and feed to adapt to the new environment. Then, the mice were treated by water and food fasting for 12 h. 10 animals were allocated to each group to ensure that there are at least 6 sets of valid data in each group. The animal experiments were carried out in strict accordance with the national regulations on the management of experimental animals.

### Test samples

The compounds prepared in the examples provided herein (test sample set) and bulleyaconitine A (positive control set) were all dissolved with 0.1 mol/L HCl according to a molar ratio of 1:1, and then diluted with physiological saline to a concentration to be determined. Blank control was physiological saline containing 0.1 mol/L HCl. The animals in the control groups were subjected to the same treatment as those in experimental groups except that samples to be test were replaced with physiological saline.

### 2. Experimental method

### (1) Determination of analgesic activity through an acetic acid-induced writhing method

10 mice were randomly selected, male: female=1:1, were placed in independent cages. Each mouse was injected subcutaneously with the sample solution mentioned above at a dose of 0.1 mL/10 g body weight. After 15 min, a 0.7% acetic acid solution was intraperitoneally injected. A number of writhing reactions in each mouse within 15 minutes was recorded. The group that the mice were injected with physiological saline subcutaneously was the blank group; the group that the mice were injected with the sample to be tested was the experimental group; and the group that the mice were injected with bulleyaconitine A was the positive group. The analgesic activity was represented by a percentage of the reduced number of writhing compared to the blank group (writhing inhibition rate), which were specifically as follows: writhing inhibition rate (analgesic activity) = (average number of writhing times in the blank group-average number of writhing times in the experimental group) / average number of writhing times in the blank group × 100%.

### Median effective dose (ED₅₀) determination

The sample to be tested was dissolved with 0.1 mol/L HCl according to the amount of the substance ratio of 1:1, and diluted with physiological saline according to the proportional series to obtain at least 5 groups with different dose concentrations. The writhing inhibition rate of each dose group was tested according to the acetic acid-induced writhing method mentioned above. The obtained dose-writhing inhibition data was entered into the statistical product and service solutions (SPSS) software (version 17.2), and the ED₅₀ of the sample to be tested was calculated through the Probit regression method.

### (2) Median lethal dose (LD₅₀) determination

The sample to be tested was dissolved with 0.1 mol/L HCl according to the amount of the substance ratio of 1:1, and diluted with physiological saline according to the required concentration. 10 mice in each group were randomly selected, half of which were male and half of which were female, and were placed in independent cages. Each mouse was injected subcutaneously with different sample solutions to be tested according to a dose of 0.1 mL/10 g body weight. In the preliminary toxicity experiment, the measured concentration was 20 mg/kg, and then based on the survival rate of the experimental animals in the preliminary experiment, the sample to be tested was set to at least 5 groups with different concentration dose according to the proportional series. The survival rate of mice within 24 h was recorded. The obtained dose-survival rate data was input into the SPSS software (version 17.2), and the LD₅₀ of the sample was calculated according to the Probit regression method.

### (3) Calculation of therapeutic index (TI)

The therapeutic index of each compound was calculated based on themedian effective dose and the median lethal dose, which were specifically as follows: therapeutic index (TI) = median lethal dose (LD₅₀) / median effective dose (ED₅₀).

### (4) Determination of anti-inflammatory activity

The effect of the compound to be tested on viability of RAW 264.7 cells at a concentration of 30 µM was assessed through MTT assay (Methylthiazolyldiphenyl-tetrazolium bromide assay), and the inhibition on NO release in the inflammatory response of LPS-induced RAW 264.7 cells at 30 µM was determined through Griess method. Specific methods were described as follows.

### MTT assay

An MTT solution (5 mg/mL) was prepared with a PBS buffer, filter-sterilized through a 0.22 µm filter membrane, and stored protected from light at low temperature. Each compound was dissolved in DMSO to prepare a 50 mmol/L stock solution, and stored from light at low (frozen) temperature. The stock solution was diluted to 30 µmol/L with DMEM (Dulbecco's modified Eagle's medium) containing 10% FBS (fetal bovine serum). RAW264.7 cells beyond third passage were added to a culture medium and gently pipetted to detach, forming a cell suspension. 10 µL of the cell suspension was applied onto a hemocytometer slide for counting, and cells were seeded into 96-well plates at a density of 6×10³ cells per well with 100 µL of the cell suspension per well. After incubated at 37 °C with 5% CO₂ for 24 h, supernatant was removed. A zero-adjustment group (CO group) received 100 µL of medium per well, the blank group (CK group) received 100 µL of DMSO (1/1000 dilution) per well, and the experimental groups received 100 µL of the test samples per well. After 12 h, supernatant was removed, and 5 µg/mL of MTT solution was added into each well at 120 µL per well. After incubated in CO₂ at a constant temperature for 4 h, supernatant was removed, and DMSO was added into each well by 150 µL per well. The mixture was shaken at 50 r/min for 10 min and subjected to an absorbance test at 492 nm. Relative cell viability (%) was calculated through a formula as follows: relative cell viability (%) = (OD_{Test} - ODco) / (OD_{CK} - ODco) × 100%.

### Griess assay

RAW264.7 cells beyond third passage were added to a culture medium and gently pipetted to detach, forming a cell suspension. 10 µL of the cell suspension was applied onto a hemocytometer slide for counting, and cells were seeded into a 96-well plate at a density of 3×10⁴ cells per well with 100 µL of the cell suspension per well. After incubated at 37 °C with 5% CO₂ for 24 h, supernatant was removed. The blank group received 100 µL of DMSO (1/1000 dilution) per well, a LPS (lipopolysaccharide) group received 100 µL of DMSO (1/1000 dilution) per well, and the experimental groups received 100 µL of the test samples per well. The LPS group received 1µg/ml of LPS by 3 µg per well, and the experimental groups received 1µg/ml of LPS by 3 µg per well. After 22 h, 50 µL of supernatant was transferred into another 96-well plate, added with Griess Reagent I in a NO assay kit by 50 µL per well, then added with Griess Reagent II by 50 µL per well, and subjected to an absorbance test at 562 nm. Nitric oxide (NO) concentration was calculated based on a standard curve, which was specifically as follows: NO inhibition rate (%) = (OD_{LPS} - OD_{Test}) / (OD_{LPS} - OD_{Blank}) × 100%.

### 3. Experimental results

**Table 1 Analgesic activity and acute toxicity of each compound**

| Compound No. | Dosing conce ntration | Analgesia rate (%)±SEM | Acute toxicity ^{c} | Compo und No. | Dosing conce ntration | Analgesia rate (%)±SEM | Acute toxicity ^{c} |
|---|---|---|---|---|---|---|---|
| | (mg/k g) | | | | (mg/k g) | | |
| Bulleyac onitine A | 0.05 | 54.80±1.8** | + | 49 | 0.05 | 13.68±1.0 | - |
| | 0.5 | -^{a} | | | | | |
| 1 | 0.05 | 25.50±2.9 | + | 50 | 0.05 | 33.16±1.4* | - |
| 2 | 0.05 | 41.10±1.8** | + | 51 | 0.05 | 32.63±1.7* | - |
| 3 | 0.05 | 9.78±1.9 | - | 52 | 0.05 | 47.05±1.8** | - |
| | | | | | 0.5 | 64.15±1.3** | |
| 4 | 0.05 | 5.63±1.1 | - | 53 | 0.05 | 0±2.6^{b} | - |
| 5 | 0.05 | 49.70±1.1** | - | 54 | 0.05 | 0±2.3^{b} | - |
| 6 | 0.05 | 59.84±2.3** | - | 55 | 0.05 | 11.68±1.6 | - |
| 7 | 0.05 | 22.81±3.3 | - | 56 | 0.05 | 11.38±2.0 | - |
| 8 | 0.05 | 37.11±1.2** | - | 57 | 0.05 | 5.39±2.0 | - |
| 9 | 0.05 | 33.54±1.9 | - | 58 | 0.05 | 28.76±1.8 | - |
| 10 | 0.05 | 15.19±1.9 | - | 59 | 0.05 | 43.51±2.5 | - |
| | | | | | 0.5 | 57.62±0.9** | |
| 11 | 0.05 | 35.13±1.2 | - | 60 | 0.05 | 50.00±1.4* | - |
| 12 | 0.05 | 45.70±0.6** | - | 61 | 0.05 | 33.99±1.9* | - |
| | 0.5 | 54.23±1.4* | | | | | |
| 13 | 0.05 | 49.37±1.3** | - | 62 | 0.05 | 15.00±2.4 | - |
| 14 | 0.05 | 40.32±1.7* | - | 63 | 0.05 | 0±1.7^{b} | - |
| | 0.5 | 44.39±2.1* | | | | | |
| 15 | 0.05 | 57.59±1.3** | - | 64 | 0.05 | 22.40±1.4 | - |
| 16 | 0.05 | 36.70±1.2* | - | 65 | 0.05 | 32.34±1.7 | - |
| 17 | 0.05 | 44.44±0.4** | - | 66 | 0.05 | 22.95±2.4 | - |
| | 0.5 | 54.73±1.5** | | | | | |
| 18 | 0.05 | 13.38±0.6 | - | 67 | 0.05 | 17.83±1.9 | - |
| 19 | 0.05 | 7.13±2.8 | - | 68 | 0.05 | 24.55±1.7 | - |
| 20 | 0.05 | 0±1.9^{b} | - | 69 | 0.05 | 40.34±0.7 | - |
| | | | | | 0.5 | 60.56±1.9** | |
| 21 | 0.05 | 0±1.9^{b} | - | 70 | 0.05 | 5.83±1.7 | - |
| 22 | 0.05 | 6.27±2.3 | - | 71 | 0.05 | 4.11±2.0 | - |
| 23 | 0.05 | 57.94±0.6** | - | 72 | 0.05 | 24.66±2.0 | - |
| 24 | 0.05 | 46.07±1.7** | - | 73 | 0.05 | 33.53±2.7 | - |
| | 0.5 | 50.89±1.7** | | | | | |
| 25 | 0.05 | 23.03±0.8 | - | 74 | 0.05 | 23.87±2.8 | - |
| 26 | 0.05 | 30.48±1.0 | - | 75 | 0.05 | 10.83±2.2 | - |
| 27 | 0.05 | 61.98±1.6** | - | 76 | 0.05 | 0±1.1^{b} | - |
| 28 | 0.05 | 36.13±2.1* | - | 77 | 0.05 | 34.17±1.8 | - |
| 29 | 0.05 | 47.89±1.8** | - | 78 | 0.05 | 31.67±1.4 | - |
| | 0.5 | 65.58±1.3** | | | | | |
| 30 | 0.05 | 31.05±2.4 | - | 79 | 0.05 | 37.90±2.0* | - |
| 31 | 0.05 | 16.97±2.2 | - | 80 | 0.05 | 19.07±2.1 | - |
| 32 | 0.05 | 37.30±1.4 | - | 81 | 0.05 | 28.76±1.2* | - |
| 33 | 0.05 | 30.17±2.6 | - | 82 | 0.05 | 33.33±1.5* | - |
| 34 | 0.05 | 36.97±1.4 | - | 83 | 0.05 | 46.97±0.9** | - |
| | | | | | 0.5 | 70.75±1.2** | |
| 35 | 0.05 | 0±2.1^{b} | - | 84 | 0.05 | 26.97±1.4 | - |
| 36 | 0.05 | 13.57±2.5 | - | 85 | 0.05 | 22.41±3.3 | - |
| 37 | 0.05 | 21.23±1.7 | - | 86 | 0.05 | 22.75±1.8 | - |
| 38 | 0.05 | 42.40±1.5** | - | 87 | 0.05 | 17.27±3.1 | - |
| | 0.5 | 56.22±1.0** | | | | | |
| 39 | 0.05 | 51.15±1.4** | - | 88 | 0.05 | 47.71±0.6** | - |
| | | | | | 0.5 | 65.07±0.6** | |
| 40 | 0.05 | 31.94±1.8 | - | 89 | 0.05 | 33.73±2.4 | - |
| 41 | 0.05 | 18.46±2.1 | - | 90 | 0.05 | 41.87±1.9 | - |
| | | | | | 0.5 | 58.99±0.8** | |
| 42 | 0.05 | 23.01±1.7 | - | 91 | 0.05 | 31.37±1.5 | - |
| 43 | 0.05 | 4.92±1.5 | - | 92 | 0.05 | 4.12±1.7 | - |
| 44 | 0.05 | 5.82±3.4 | - | 93 | 0.05 | 10.46±2.3 | - |
| 45 | 0.05 | 21.56±1.3 | - | 94 | 0.05 | 0±1.7^{b} | - |
| 46 | 0.05 | 16.67±2.6 | - | 95 | 0.05 | 23.80±1.1 | - |
| 47 | 0.05 | 33.77±2.2 | - | 96 | 0.05 | 30.15±1.3* | - |
| 48 | 0.05 | 17.49±3.0 | - | | | | |

| | | | | | | | |
|---|---|---|---|---|---|---|---|
| a: Unable to test owing to significant toxicity or death in mice; Blank control was physiological saline containing 0.1 mol/L HCl; b: 0% of analgesia rate indicated that under the current experimental conditions, the inhibitory effect of the drug was below detection limit or showed no significant difference compared with other treatment groups. c: In the acute toxicity test, mice were administered at a concentration of 20 mg/kg, and the presence of death within 24 h was observed, if death occurred, (+) represented as positive; and if no death occurred, (-) represented as negative. *P<0.05, **P<0.01. | | | | | | | |

**Table 2 ED₅₀ and LD₅₀ of part of compounds**

| Compound No. | ED₅₀ (mg/kg) | LD₅₀ (mg/kg) ^{a} | TI |
|---|---|---|---|
| Bulleyaconitine A | 0.048 | 0.92 | 19.2 |
| Blank control | - | - | - |
| 5 | 0.083 | >100 | >1204.8 |
| 6 | 0.069 | >600 | >8695.7 |
| 13 | 0.082 | >100 | >1219.5 |
| 15 | 0.073 | 56.43 | 773 |
| 23 | 0.073 | >100 | >1369.9 |
| 27 | 0.033 | >600 | >18181.8 |
| 29 | 1.478 | 41.18 | 27.9 |
| 39 | 0.195 | >100 | >512.8 |
| 52 | 0.479 | >100 | >208.8 |
| 60 | 0.122 | >100 | >819.7 |
| 69 | 0.106 | >100 | >943.4 |
| 83 | 0.146 | 76.64 | 524.9 |
| 88 | 0.161 | >100 | >621.1 |

| | | | |
|---|---|---|---|
| a: LD₅₀ > X in the table 2 indicated that under the current experimental conditions, no death or toxic symptoms were observed when animals were treated with the drug at a concentration of X mg/kg, which suggested that the LD₅₀ may be higher than such concentration, and accurate LD₅₀ still needed to be determined through further experiments. | | | |

**Table 3 Inhibition rate of compounds on LPS-induced NO production in RAW264.7 cells**

| Compound No. | RAW cell viability | Inflammatory NO inhibition rate |
|---|---|---|
| Celecoxib | 93.09% | 74% |
| Bulleyaconitine A | 85.80% | 13% |
| 5 | 28.05% | - |
| 6 | 92.23% | 22% |
| 12 | 119.75% | 17% |
| 13 | 8.29% | - |
| 14 | 13.36% | - |
| 15 | 104.18% | 5% |
| 17 | 87.25% | 47% |
| 23 | 81.35% | - |
| 24 | 94.97% | 8% |
| 27 | 87.20% | 7% |
| 28 | 83.03% | - |
| 29 | 116.43% | 20% |
| 38 | 99.37% | 23% |
| 39 | 6.97% | - |
| 52 | 7.00% | - |
| 59 | 7.00% | - |
| 60 | 96.09% | 2% |
| 69 | 95.11% | 40% |
| 79 | 101.29% | 42% |
| 83 | 85.21% | 50% |
| 88 | 74.05% | - |
| 90 | 101.74% | 12% |

First, analgesic activity of the compounds is evaluated using a reported median effective dose (ED₅₀ = 0.05 mg/kg) of bulleyaconitine A as a preliminary screening concentration. Acute toxicity is preliminary assessed through subcutaneous injection at an initial concentration of 20 mg/kg (Table 1). Data in Table 1 shows that except for Compounds 1 and 2, all compounds prepared in the examples in the present disclosure causes no death in the preliminary acute toxicity test, indicating toxicity of the compounds are significantly lower than of bulleyaconitine A, and most of the compounds shows analgesic activity comparable to bulleyaconitine A (defined as an analgesia rate ≥ 49%). Therefore, compounds with the analgesia rate ≥ 49% are selected for ED₅₀ determination. Besides, owing to low acute toxicity of the compounds prepared herein, compounds with favorable analgesic activity (defined as an initial screening analgesia rate ≥ 40%) are further evaluated at 0.5 mg/kg (Table 1). Compounds with the analgesia rate ≥ 60% at this concentration are subjected to ED₅₀ determination. At the same time, the median lethal dose (LD₅₀) of preferred compounds above are tested. Data in Table 2 shows that, the preferred compounds show activity comparable to bulleyaconitine A but with substantially reduced toxicity, resulting in therapeutic indices significantly higher than that of bulleyaconitine A.

On the other hand, the anti-inflammatory activity of the compounds is evaluated. Compounds with lower toxicity and better analgesic activity than bulleyaconitine A (defined as the initial screening analgesic rate ≥ 40%) are subjected to RAW cytotoxicity assays (shown in Table 3). Then, compounds exhibiting RAW cell viability ≥ 85% are further tested for their inhibitory effects on NO production. Data in Table 3 shows that, some compounds demonstrate significant anti-inflammatory activity (defined as the NO inhibition rate ≥ 40%) (shown in Table 3).

The above experimental results demonstrate that the compounds prepared in the present disclosure have good analgesic activity, good anti-inflammatory activity and less biological toxicity, and are suitable for preparing high-effective and low-toxicity analgesic drugs.

## Claims

1. A compound of formula (I), or a stereoisomer, a deuterated compound, a solvate, a prodrug, a metabolite, a crystalline form, or a pharmaceutically acceptable salt thereof: 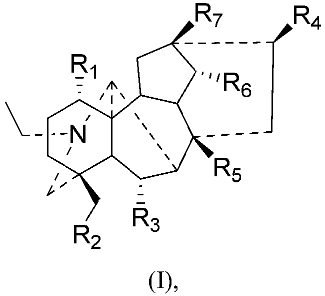 **characterized in that**,
R₁, R₂, R₃, R₄ and R₅ are each independently selected from the group consisting of hydrogen, hydroxyl, halogen, C₁₋₁₈ alkoxy, C₁₋₁₈ alkyl, COORₐ and OCORₐ; wherein Rₐ is selected from the group consisting of hydrogen, C₁₋₁₈ alkyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl;
R₆ and R₇ are each independently selected from the group consisting of hydrogen, hydroxyl, C₁₋₁₈ alkoxy, C₁₋₁₈ alkyl, OCOR_{b}, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl, wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z}; and
each R_{z} is independently selected from the group consisting of hydrogen, substituted and unsubstituted C₁₋₆ alkyl, C₂₋₆ alkenyl, C₂₋₆ alkynyl, substituted and unsubstituted C₁₋₆ alkoxy, 3 to 8-membered saturated cycloalkyl, NR₃R₄, COOR₅, SO₂R₆, halogen, cyano group, nitro, hydroxyl, carboxyl and phenyl, wherein substituted C₁₋₆ alkyl and substituted C₁₋₆ alkoxy independently have a substituent selected from the group consisting of halogen, cyano group, nitro, hydroxyl and carboxyl.

2. The compound of claim 1, or a stereoisomer, a deuterated compound, a solvate, a prodrug, a metabolite, a crystalline form, or a pharmaceutically acceptable salt thereof, **characterized in that** the compound is represented by formula (II): 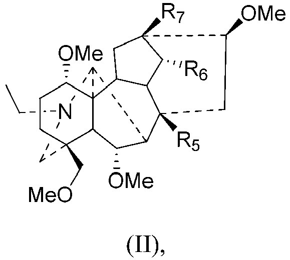
wherein R₅ is selected from the group consisting of hydrogen, hydroxyl, halogen, C₁₋₁₈ alkoxy, C₁₋₁₈ alkyl, COORₐ and OCORₐ, wherein Rₐ is selected from the group consisting of hydrogen, C₁₋₁₈ alkyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl;
R₆ and R₇ are each independently selected from the group consisting of hydrogen, hydroxyl, C₁₋₁₈ alkoxy, C₁₋₁₈ alkyl, OCOR_{b}, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl, wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z};
each R_{z} is independently selected from the group consisting of hydrogen, substituted and unsubstituted C₁₋₆ alkyl, C₂₋₆ alkenyl, C₂₋₆ alkynyl, substituted and unsubstituted C₁₋₆ alkoxy, 3 to 8-membered saturated cycloalkyl, NR₃R₄, COOR₅, SO₂R₆, halogen, cyano group, nitro, hydroxyl, carboxyl and phenyl, wherein substituted C₁₋₆ alkyl and substituted C₁₋₆ alkoxy independently have a substituent selected from the group consisting of halogen, cyano group, nitro, hydroxyl and carboxyl, and R₃ and R₄ are each independently selected from the group consisting of hydrogen and C₁₋₆ alkyl.

3. The compound of claim 2, or a stereoisomer, a deuterated compound, a solvate, a prodrug, a metabolite, a crystalline form, or a pharmaceutically acceptable salt thereof, **characterized in that** the compound is represented by formula (III): 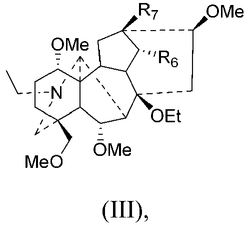
wherein R₆ and R₇ are each independently selected from the group consisting of hydrogen, hydroxyl, C₁₋₆ alkoxy, C₁₋₆ alkyl, OCOR_{b}, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl, wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z};
each R_{z} is independently selected from the group consisting of hydrogen, halogenated and unhalogenated C₁₋₆ alkyl, C₂₋₆ alkenyl, C₂₋₆ alkynyl, halogenated and unhalogenated C₁₋₆ alkoxy, 3 to 8-membered saturated cycloalkyl, NR₃R₄, COOR₅, SO₂R₆, halogen, cyano group, nitro, hydroxyl, carboxyl and phenyl; and
R₃ and R₄ are each independently selected from the group consisting of hydrogen and C₁₋₆ alkyl; and R₅ is C₁₋₆ alkyl.

4. The compound of claim 2, or a stereoisomer, a deuterated compound, a solvate, a prodrug, a metabolite, a crystalline form, or a pharmaceutically acceptable salt thereof, **characterized in that** the compound is represented by formula (IV): 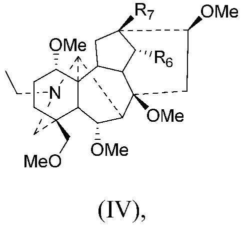
wherein R₆ and R₇ are each independently selected from the group consisting of hydrogen, hydroxyl, C₁₋₆ alkoxy, C₁₋₆ alkyl, OCOR_{b}, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl, wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z};
each R_{z} is independently selected from the group consisting of hydrogen, halogenated and unhalogenated C₁₋₆ alkyl, C₂₋₆ alkenyl, C₂₋₆ alkynyl, halogenated and unhalogenated C₁₋₆ alkoxy, 3 to 8-membered saturated cycloalkyl, NR₃R₄, COOR₅, SO₂R₆, halogen, cyano group, nitro, hydroxyl, carboxyl and phenyl; and
R₃ and R₄ are each independently selected from the group consisting of hydrogen and C₁₋₆ alkyl; and R₅ is C₁₋₆ alkyl.

5. The compound of claim 2, or a stereoisomer, a deuterated compound, a solvate, a prodrug, a metabolite, a crystalline form, or a pharmaceutically acceptable salt thereof, **characterized in that** the compound is represented by formula (V): 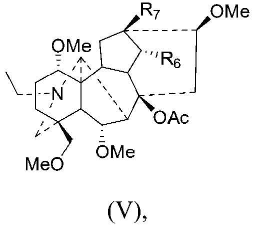
wherein R₆ and R₇ are each independently selected from the group consisting of hydrogen, hydroxyl, C₁₋₆ alkoxy, C₁₋₆ alkyl, OCOR_{b}, OCOCH₂R_{b} and OSO₂R_{b};
R_{b} is selected from the group consisting of hydrogen, C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl, wherein C₁₋₆ alkyl, C₂₋₆ alkenyl, C₂₋₆ alkynyl, phenyl, 3 to 6-membered heteroaryl, 3 to 8-membered saturated cycloalkyl, 3 to 8-membered saturated heterocyclyl, fused cycloalkyl, fused heterocyclyl, bridged cycloalkyl and bridged heterocyclyl are independently substituted with one or more R_{z};
each R_{z} is independently selected from the group consisting of hydrogen, halogenated and unhalogenated C₁₋₆ alkyl, C₂₋₆ alkenyl, C₂₋₆ alkynyl, halogenated and unhalogenated C₁₋₆ alkoxy, 3 to 8-membered saturated cycloalkyl, NR₃R₄, COOR₅, SO₂R₆, halogen, cyano group, nitro, hydroxyl, carboxyl and phenyl; and
R₃ and R₄ are each independently selected from the group consisting of hydrogen and C₁₋₆ alkyl; and R₅ is C₁₋₆ alkyl.

6. The compound of any one of claims 1-5, or a stereoisomer, a deuterated compound, a solvate, a prodrug, a metabolite, a crystalline form, or a pharmaceutically acceptable salt thereof, **characterized in that** the compound is selected from the group consisting of:

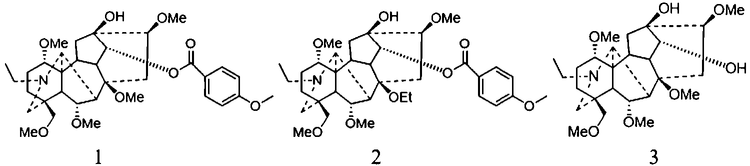

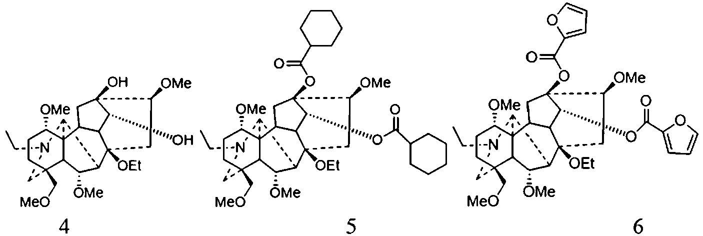

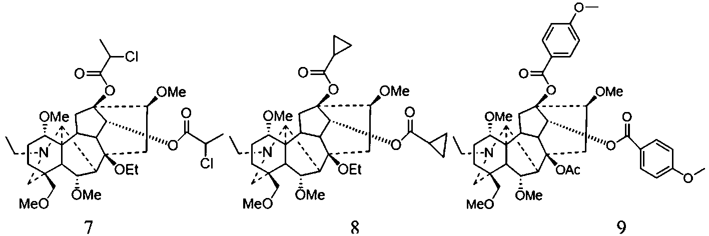

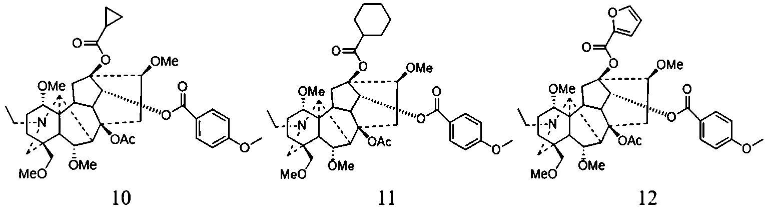

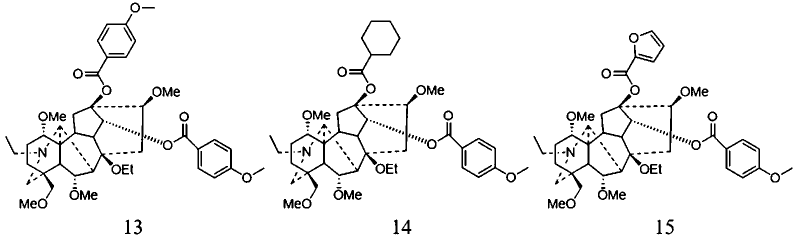

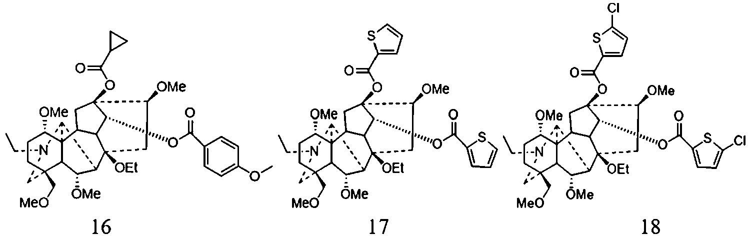

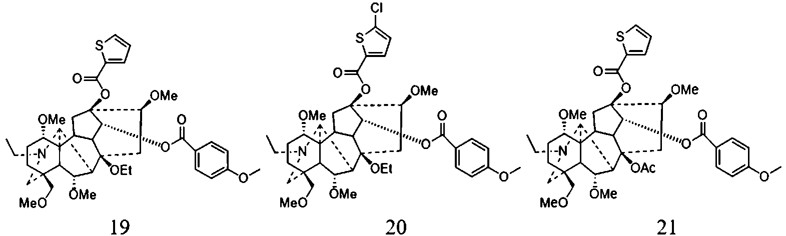

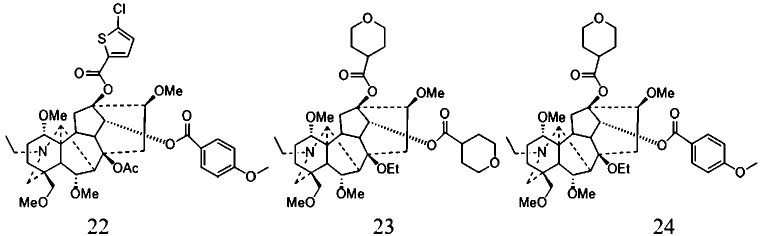

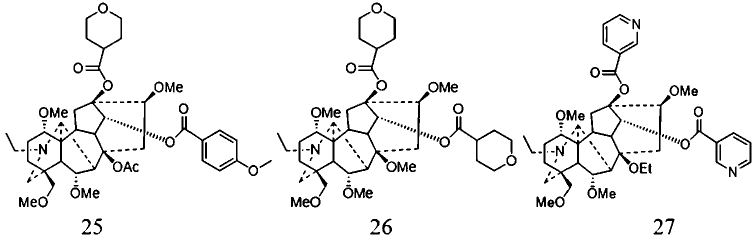

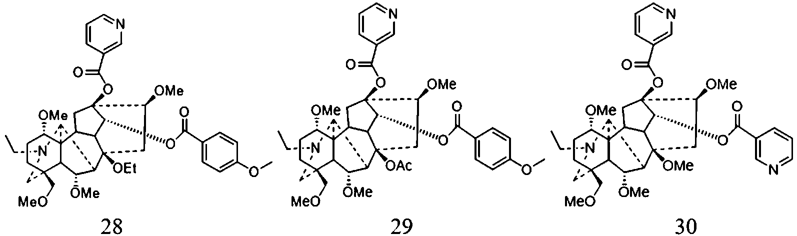

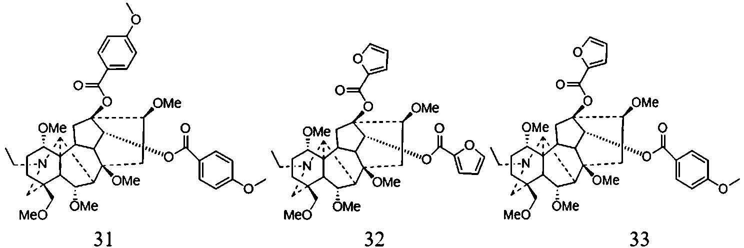

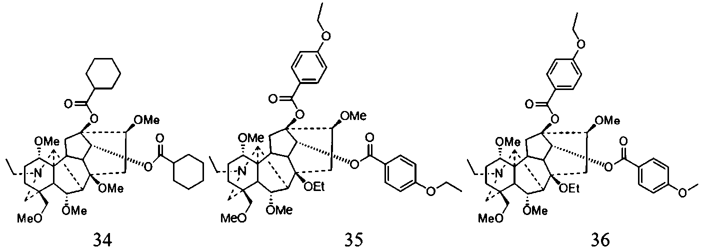

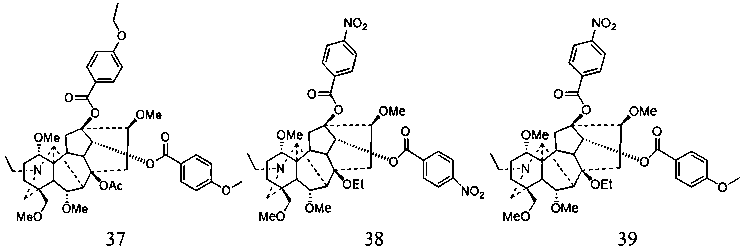

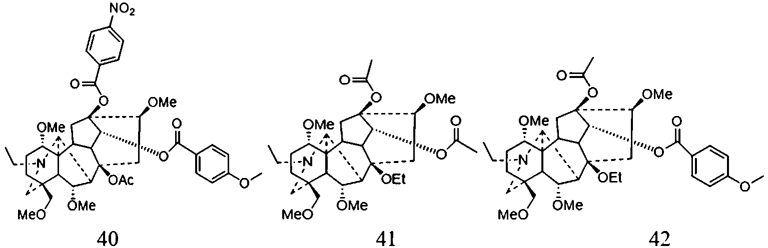

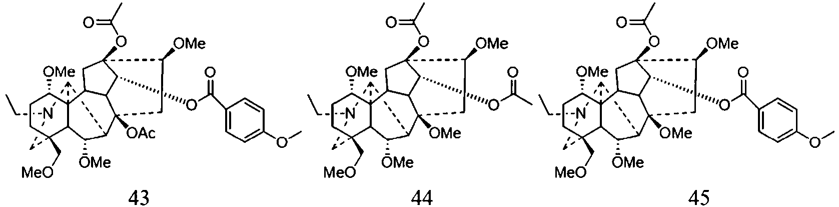

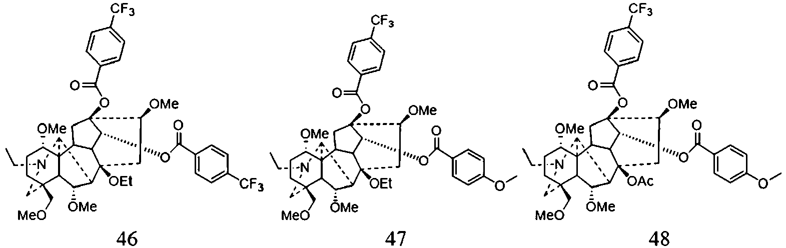

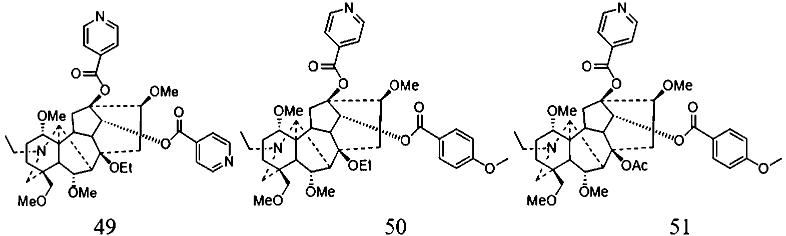

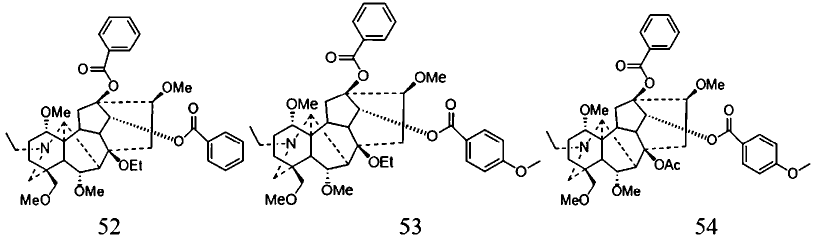

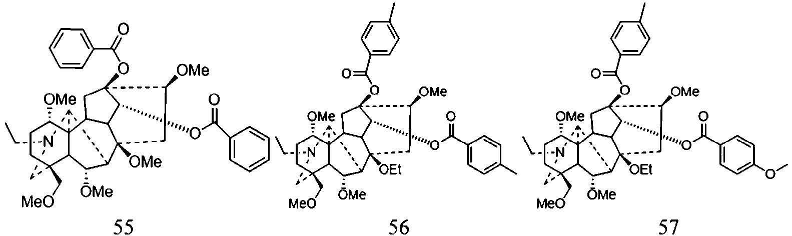

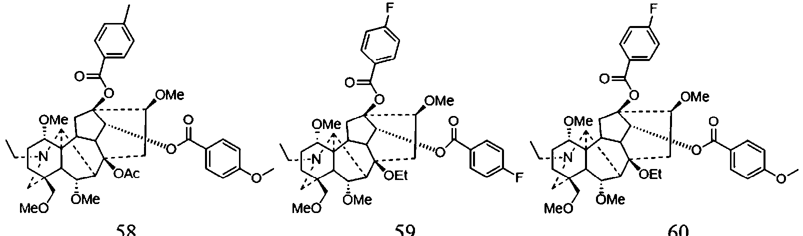

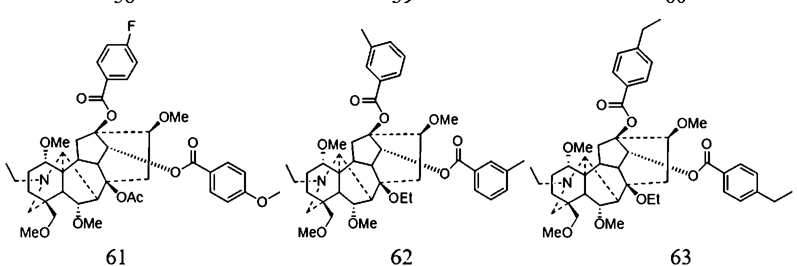

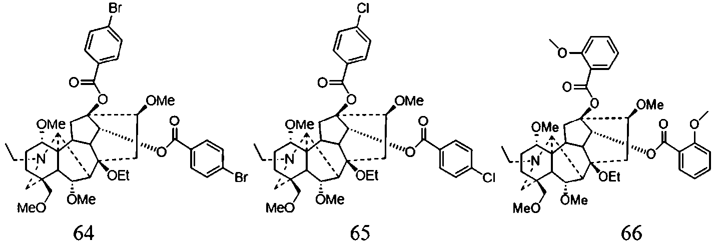

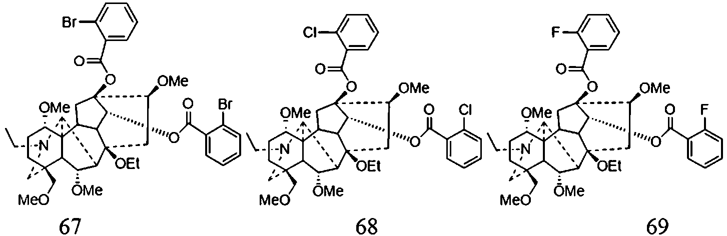

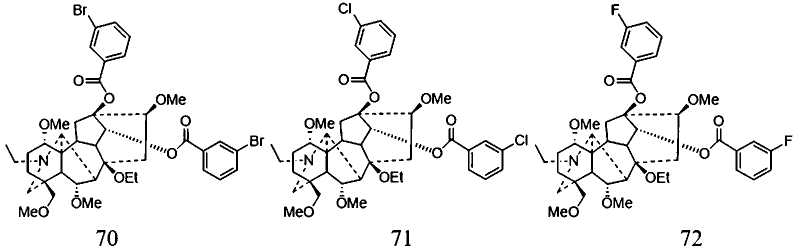

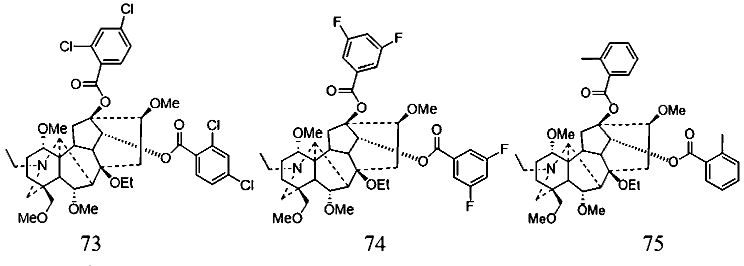

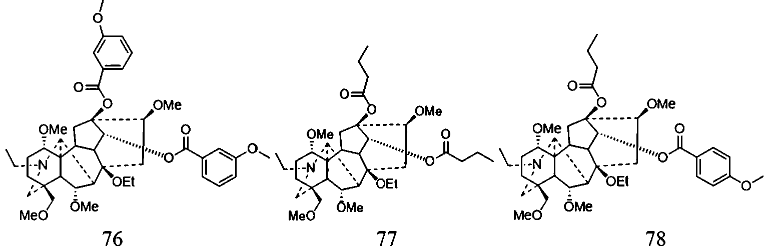

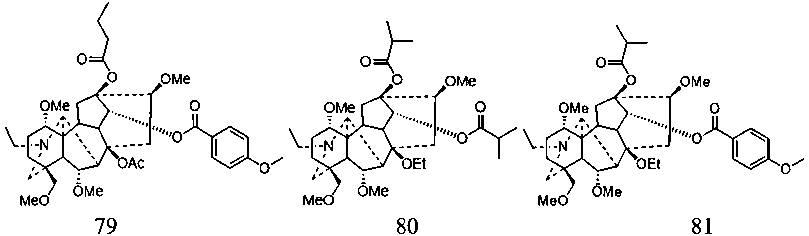

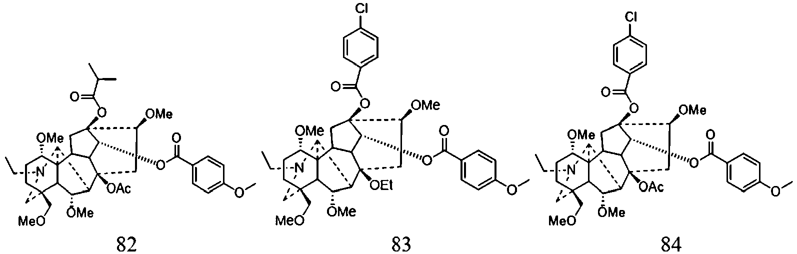

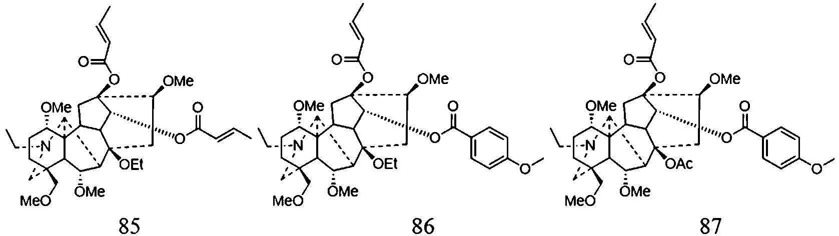

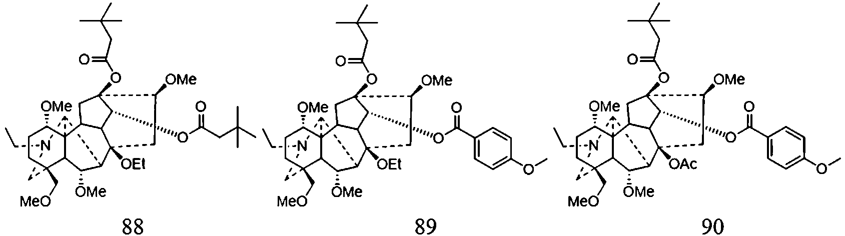

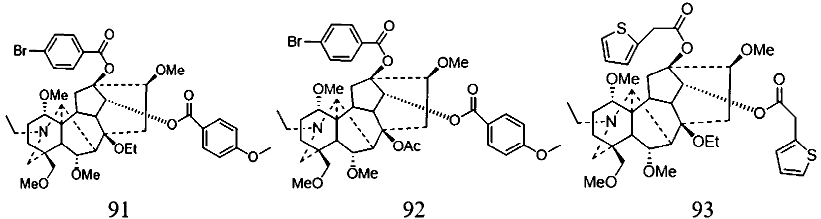

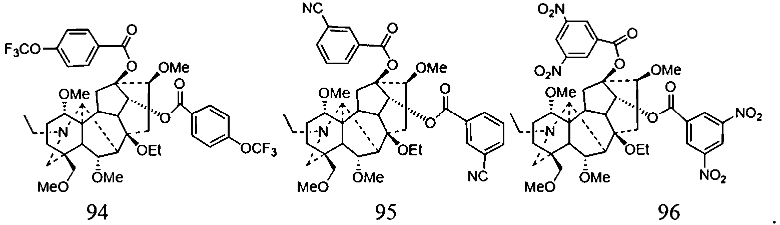

7. A pharmaceutical composition for analgesia and/or anti-inflammation, comprising:
an active ingredient; and
a pharmaceutically acceptable excipient;
wherein the active ingredient is the compound of any one of claims 1-6, or a stereoisomer, a deuterated compound, a solvate, a prodrug, a metabolite, a crystalline form, or a pharmaceutically acceptable salt thereof.

8. The compound of any one of claims 1-6, or a stereoisomer, a deuterated compound, a solvate, a prodrug, a metabolite, a crystalline form, or a pharmaceutically acceptable salt thereof for use in the preparation of a drug for analgesia and/or anti-inflammation.

9. The compound for use of claim 8, **characterized in that** the drug for analgesia and/or anti-inflammation has low toxicity.

10. The compound for use of claim 9, **characterized in that** a median lethal dose of the drug is higher than that of bulleyaconitine A; and/or a therapeutic index of the drug is higher than that of bulleyaconitine A.
